# EUROPEAN PATENT APPLICATION

(11) **EP 4 234 556 A1**
(43) Date of publication of application: **30.08.2023**
(21) Application number: 21882915.8
(22) Date of filing: 21.10.2021
(51) Int. Cl.: C07D 487/04, C09K 11/06, H01L 51/50, H05B 33/10

(54) **MATERIAL FOR ORGANIC ELECTROLUMINESCENT ELEMENT AND ORGANIC ELECTROLUMINESCENT ELEMENT**

(30) Priority: 23.10.2020 JP 2020178249
(71) Applicant: NIPPON STEEL Chemical & Material Co., Ltd., Tokyo 103-0027 (JP)
(72) Inventor: IZUMI Haruka, Tokyo 103-0027 (JP); HAYASHI Kentaro, Tokyo 103-0027 (JP)
(74) Representative: Gerauer, Marc Philippé
(86) International application number: PCT/JP2021/039021
(87) International publication number: WO 2022/085776

(57) **Abstract**

To provide an organic EL device having a low driving voltage and also high efficiency and high driving stability, and a compound suitable therefor. The compound for an organic EL device has a structure in which a nitrogen-containing 6-membered ring group like triazine is directly bonded to one of two N-positions in an indolocarbazole ring and a nitrogen-containing 6-membered ring group is bonded to other thereof via an aromatic hydrocarbon group like phenylene. The organic EL device of the present invention contains the compound for an organic EL device and a biscarbazole compound as hosts in a light-emitting layer.

## Description

### Technical Field

The present invention relates to a compound for an organic electroluminescent element or device (organic EL device) and an organic EL device comprising a specific mixed host material.

### Background Art

Application of a voltage to an organic EL device allows injection of holes and electrons from an anode and a cathode, respectively, into a light-emitting layer. Then, in the light-emitting layer, injected holes and electrons recombine to generate excitons. At this time, according to statistical rules of electron spins, singlet excitons and triplet excitons are generated at a ratio of 1:3. Regarding a fluorescence-emitting organic EL device using light emission from singlet excitons, it is said that the internal quantum efficiency thereof has a limit of 25%. Meanwhile, regarding a phosphorescent organic EL device using light emission from triplet excitons, it is known that intersystem crossing is efficiently performed from singlet excitons, the internal quantum efficiency is enhanced to 100%.

Further, highly efficient organic EL devices utilizing delayed fluorescence have been developed recently. For example, Patent Literature 1 discloses an organic EL device utilizing a TTF (Triplet-Triplet Fusion) mechanism, which is one of delayed fluorescence mechanisms. The TTF mechanism utilizes a phenomenon in which singlet excitons are generated due to collision of two triplet excitons, and it is thought that the internal quantum efficiency can be theoretically raised to 40%. However, since the efficiency is lower compared to phosphorescent organic EL devices, further improvement in efficiency and low voltage characteristics are required.

In addition, patent Literature 2 discloses an organic EL device utilizing a TADF (Thermally Activated Delayed Fluorescence) mechanism. The TADF mechanism utilizes a phenomenon in which reverse intersystem crossing from triplet excitons to singlet excitons is generated in a material having a small energy difference between a singlet level and a triplet level, and it is thought that the internal quantum efficiency can be theoretically raised to 100%.

However, all the mechanisms have room for advancement in terms of both efficiency and lifetime, and are additionally required to be improved also in terms of reduction in driving voltage.

### Citation List

### Patent Literature

Patent Literature 1: WO2010/134350 A
Patent Literature 2: WO2011/070963 A
Patent Literature 3: WO2008/056746 A
Patent Literature 4: WO2011/099374 A
Patent Literature 5: WO2018/198844 A
Patent Literature 6: WO2016/023608 A
Patent Literature 7: US2018/0134718 A

Patent Literatures 3 and 6 disclose use of an indolocarbazole compound as a host material. Patent Literatures 5 discloses use of an indolocarbazole compound and a biscarbazole compound as a mixed host. Patent Literature 4 discloses an indolocarbazole having an aryl linkage group. Patent Literature 7 discloses use of an indolocarbazole compound which is a specific isomer, as a host material.

However, none of these can be said to be sufficient, and further improvements in efficiency and voltage of an organic EL device are desired.

### Summary of Invention

In order to apply organic EL devices to display devices such as flat panel displays, it is necessary to improve the luminous efficiency of devices and at the same time, to sufficiently secure the lifetime characteristics of the devices. In view of the above circumstances, an object of the present invention is to provide an organic EL device having a low driving voltage and also high efficiency and high driving stability, and a compound suitable therefor.

As a result of intensive studies, the present inventors have found that an organic EL device exhibits excellent characteristics by using a specific indolocarbazole compound, and have completed the present invention.

The present invention relates to a compound for an organic EL device, represented by the following general formula (1).

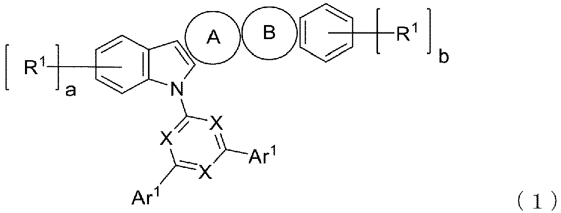

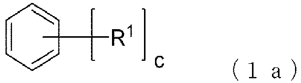

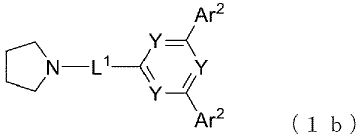

In the formula, a ring A represents an aromatic hydrocarbon ring fused to two adjacent rings at any position and represented by formula (1a),
a ring B represents a heterocyclic ring fused to two adjacent rings at any position and represented by formula (1b),
X and Y each independently represent CR² or N, wherein at least one thereof represents N,
each R¹ independently represents deuterium, an aliphatic hydrocarbon group having 1 to 10 carbon atoms, a substituted or unsubstituted aromatic hydrocarbon group having 6 to 18 carbon atoms, or a substituted or unsubstituted aromatic heterocyclic group having 3 to 17 carbon atoms,
each R² independently represents hydrogen, deuterium, an aliphatic hydrocarbon group having 1 to 10 carbon atoms, a substituted or unsubstituted aromatic hydrocarbon group having 6 to 18 carbon atoms, or a substituted or unsubstituted aromatic heterocyclic group having 3 to 17 carbon atoms,
a and b each independently represent an integer of 0 to 4, and c represents an integer of 0 to 2,
Ar¹ and Ar² each independently represent hydrogen, a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group in which two to five of these aromatic rings are linked to each other, and
L¹ represents a substituted or unsubstituted aromatic hydrocarbon group having 6 to 18 carbon atoms, or a substituted or unsubstituted linked aromatic group in which two to five of these aromatic hydrocarbon groups are linked to each other.

In a preferred aspect, in the general formula (1), all of X represent N or all of Y represent N. In other preferred aspects, L¹ represents a substituted or unsubstituted phenylene group, and Ar¹ or Ar² represents hydrogen, a substituted or unsubstituted aromatic hydrocarbon group having 6 to 18 carbon atoms, or a substituted or unsubstituted linked aromatic group in which two to five of these aromatic hydrocarbon groups are linked to each other.

The present invention is an organic EL device comprising one or more light-emitting layers between an anode and a cathode opposed to each other, wherein at least one of the light-emitting layers contains a first host, a second host, and a light-emitting dopant material, the first host is selected from the compound represented by the general formula (1) for an organic EL device, and the second host is selected from a compound represented by the following general formula (2).

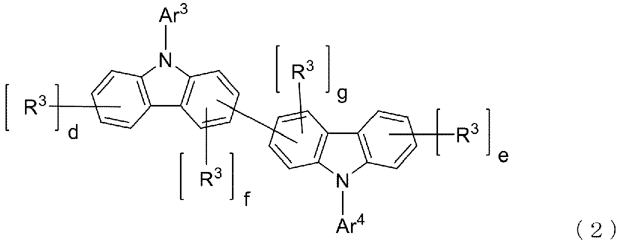

In the formula, Ar³ and Ar⁴ independently represent a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group in which two to five of these aromatic groups are linked to each other,
each R³ independently represents deuterium, an aliphatic hydrocarbon group having 1 to 10 carbon atoms, a substituted or unsubstituted aromatic hydrocarbon group having 6 to 18 carbon atoms, or a substituted or unsubstituted aromatic heterocyclic group having 3 to 17 carbon atoms, and
d and e each independently represent an integer of 0 to 4, and f and g each independently represent an integer of 0 to 3.

In a preferred aspect, in the general formula (2), Ar³ and Ar⁴ independently represent a substituted or unsubstituted aromatic hydrocarbon group having 6 to 18 carbon atoms, or a substituted or unsubstituted linked aromatic group in which two to five of these aromatic hydrocarbon groups are linked to each other, all d, e, f, and g represent 0, or Ar³ and Ar⁴ independently represent any of a substituted or unsubstituted phenyl group, m-biphenyl group, or p-biphenyl group.

The present invention is also a method for producing the organic EL device, comprising providing a premixed composition comprising the first host and the second host, and producing a light-emitting layer by use of the premixed composition. The present invention is also a premixed composition for an organic EL device, comprising the first host and the second host.

In a preferred aspect, a difference in 50% weight reduction temperatures of the first host and the second host is within 20°C.

For improvement of device characteristics, a higher durability of a material used for an organic layer against charges is needed, and particularly, in the light-emitting layer, it is important to reduce leakage of excitons and charges to surrounding layers. For leakage reduction of charges/excitons, it is effective to improve localization of a light emission area in the light-emitting layer. For this purpose, it is necessary to control amounts of both charges (electrons/holes) injected into the light-emitting layer or amounts of both charges transported in the light-emitting layer within a preferred range.

The material for an organic EL device of the present invention is a material obtained by direct substitution with a nitrogen-containing six-membered ring high in electron-accepting properties, on one N in indolocarbazole as a skeleton assumed to have high electron-donating properties and high stability, and substitution with a nitrogen-containing six-membered ring linked by an aromatic hydrocarbon group, on another N in the indolocarbazole. It is considered that charge transport properties are thus expected to be improved and the material is used as a host material in an organic EL device to thereby enable a low voltage and high efficiency to be achieved. Meanwhile, a carbazole compound represented by the general formula (2) especially has high hole injection transport ability, and changing the binding form of a carbazole ring or the kind or number of substituents to a skeleton thereof is assumed to allow a high-level control of hole injection transport properties. Then, it is considered that a combination with the compound represented by the general formula (1) can control amounts of both charges injected into an organic layer within a preferred range, so that localization of a light emission area in the light-emitting layer can be improved.

### Brief Description of Drawing

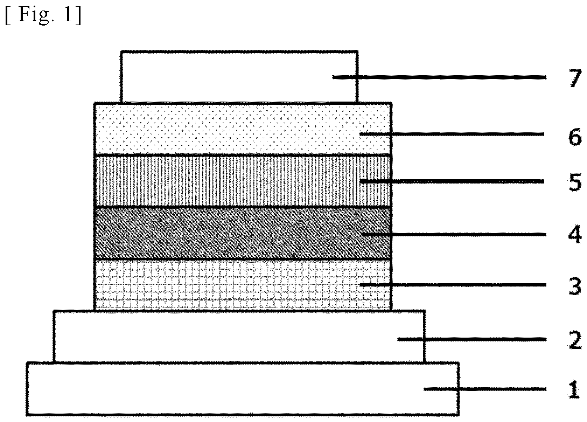

[Figure 1] Figure 1 is a schematic cross-sectional view showing one example of an organic EL device.

### Description of Embodiments

A material for an organic EL device of the present invention is represented by the general formula (1). In the general formula (1), a ring A is a benzene ring represented by formula (1a). A ring B is a heterocyclic ring (pyrrole ring) represented by formula (1b), and is fused to two adjacent rings at any position.

The material for an organic EL device of the present invention can be any compound represented by the following formulas (3a) to (3f). Preferred is any compound represented by the formulas (3a) to (3e). More preferred is any compound represented by the formulas (3a) to (3c). Further preferred is any compound represented by formula (3a).

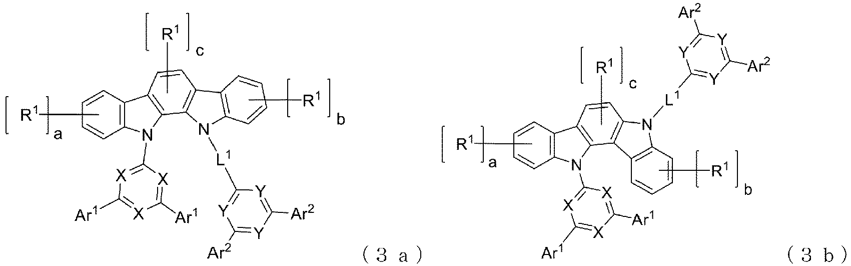

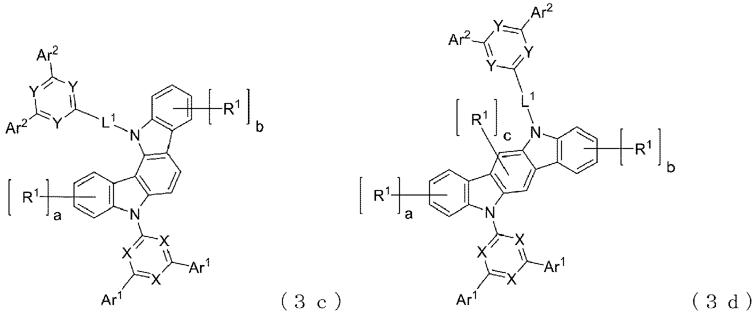

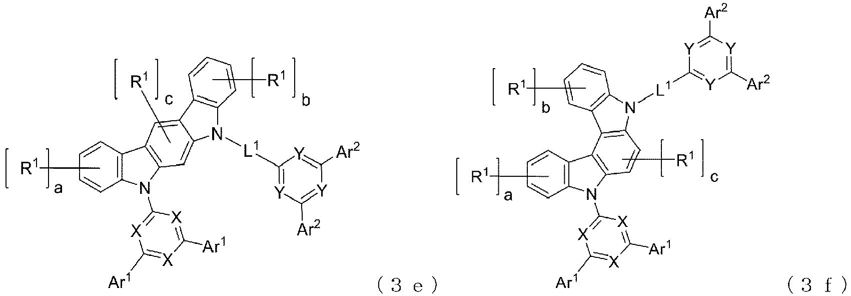

In the general formula (1) and formulas (3a) to (3f), the same symbols have the same meaning.

X and Y each independently represent CR² or N, wherein at least one thereof represents N. Preferably, at least two X represent N, and more preferably, all of X represent N. Preferably, at least two Y represent N, and more preferably, all of Y represent N. Particularly preferably, all X and Y represent N.

Each R¹ independently represents deuterium, an aliphatic hydrocarbon group having 1 to 10 carbon atoms, a substituted or unsubstituted aromatic hydrocarbon group having 6 to 18 carbon atoms, or a substituted or unsubstituted aromatic heterocyclic group having 3 to 17 carbon atoms. Preferred is deuterium, a substituted or unsubstituted aromatic hydrocarbon group having 6 to 18 carbon atoms, or a substituted or unsubstituted aromatic heterocyclic group having 3 to 17 carbon atoms. Further preferred is deuterium, or a substituted or unsubstituted aromatic hydrocarbon group having 6 to 18 carbon atoms.

Each R² independently represents hydrogen, deuterium, an aliphatic hydrocarbon group having 1 to 10 carbon atoms, a substituted or unsubstituted aromatic hydrocarbon group having 6 to 18 carbon atoms, or a substituted or unsubstituted aromatic heterocyclic group having 3 to 17 carbon atoms, preferably represents hydrogen, deuterium, a substituted or unsubstituted aromatic hydrocarbon group having 6 to 18 carbon atoms, or a substituted or unsubstituted aromatic heterocyclic group having 3 to 17 carbon atoms. Further preferred is hydrogen, deuterium, or a substituted or unsubstituted aromatic hydrocarbon group having 6 to 18 carbon atoms.

Specific examples of the aliphatic hydrocarbon group having 1 to 10 carbon atoms include methyl, ethyl, propyl, butyl, pentyl, hexyl, heptyl, octyl, nonyl, and decyl. Preferred examples thereof include an alkyl group having 1 to 4 carbon atoms.

Specific examples of the unsubstituted aromatic hydrocarbon group having 6 to 18 carbon atoms or the unsubstituted aromatic heterocyclic group having 3 to 17 carbon atoms include a group generated by removing one hydrogen from benzene, naphthalene, acenaphthene, acenaphthylene, azulene, anthracene, chrysene, pyrene, phenanthrene, fluorene, triphenylene, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, thiadiazole, phthalazine, tetrazole, indole, benzofuran, benzothiophene, benzoxazole, benzothiazole, indazole, benzimidazole, benzotriazole, benzisothiazole, benzothiadiazole, purine, pyranone, coumarin, isocoumarin, chromone, dibenzofuran, dibenzothiophene, dibenzoselenophene, or carbazole.

Preferred examples thereof include an aromatic group generated from benzene, pyridine, pyrimidine, triazine, thiophene, isothiazole, thiazole, pyridazine, pyrrole, pyrazole, imidazole, triazole, thiadiazole, pyrazine, furan, isoxazole, oxazole, oxadiazole, quinoline, isoquinoline, quinoxaline, quinazoline, oxadiazole, thiadiazole, benzotriazine, phthalazine, tetrazole, indole, benzofuran, benzothiophene, benzoxazole, benzothiazole, indazole, benzimidazole, benzotriazole, benzisothiazole, or benzothiadiazole. More preferred examples thereof include an aromatic group generated from benzene, pyridine, pyrimidine, triazine, thiophene, isothiazole, thiazole, pyridazine, pyrrole, pyrazole, imidazole, triazole, thiadiazole, pyrazine, furan, isoxazole, oxazole, or oxadiazole.

a and b represent an integer of 0 to 4, and c represents an integer of 0 to 2. Preferably, a and b represent 0 to 2. More preferably, all a, b and c represent 0.

Ar¹ and Ar² each independently represent hydrogen, a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group in which two to five of these aromatic rings are linked to each other. Preferred is hydrogen, a substituted or unsubstituted aromatic hydrocarbon group having 6 to 18 carbon atoms, or a substituted or unsubstituted linked aromatic group in which two to five of these aromatic hydrocarbon groups are linked to each other. More preferred is a phenyl group, or a substituted or unsubstituted linked aromatic group in which two to five phenyl groups are linked to each other.

Specific examples of the unsubstituted aromatic hydrocarbon group having 6 to 18 carbon atoms, the unsubstituted aromatic heterocyclic group having 3 to 17 carbon atoms, or the linked aromatic group in which two to five of these aromatic rings are linked to each other include a group generated by removing one hydrogen from benzene, naphthalene, acenaphthene, acenaphthylene, azulene, anthracene, chrysene, pyrene, phenanthrene, fluorene, triphenylene, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, thiadiazole, phthalazine, tetrazole, indole, benzofuran, benzothiophene, benzoxazole, benzothiazole, indazole, benzimidazole, benzotriazole, benzisothiazole, benzothiadiazole, purine, pyranone, coumarin, isocoumarin, chromone, dibenzofuran, dibenzothiophene, dibenzoselenophene, carbazole, or compounds in which two to five of these are linked to each other. Preferred examples thereof include a group generated from benzene, naphthalene, acenaphthene, acenaphthylene, azulene, anthracene, chrysene, pyrene, phenanthrene, fluorene, triphenylene, or compounds in which two to five of these are linked to each other. More preferred is a substituted or unsubstituted phenyl group, biphenyl group, or terphenyl group. The terphenyl group may be linked linearly or branched.

In the present specification, an aromatic hydrocarbon group, an aromatic heterocyclic group, or a linked aromatic group may each have a substituent. In the case of having a substituent, the substituent is preferably deuterium, halogen, a cyano group, a triarylsilyl group, an aliphatic hydrocarbon group having 1 to 10 carbon atoms, an alkenyl group having 2 to 5 carbon atoms, an alkoxy group having 1 to 5 carbon atoms, or a diarylamino group having 12 to 44 carbon atoms. In the case of the aliphatic hydrocarbon group having 1 to 10 carbon atoms, the substituent may be linear, branched, or cyclic.

Note that the number of substituents is 0 to 5 and preferably 0 to 2. When an aromatic hydrocarbon group and an aromatic heterocyclic group have substituents, the calculation of the number of carbon atoms does not include the number of carbon atoms of the substituents. However, it is preferred that the total number of carbon atoms including the number of carbon atoms of substituents satisfy the above range.

Specific examples of the substituents include deuterium, cyano, methyl, ethyl, propyl, i-propyl, butyl, t-butyl, pentyl, cyclopentyl, hexyl, cyclohexyl, heptyl, octyl, nonyl, decyl, vinyl, propenyl, butenyl, pentenyl, methoxy, ethoxy, propoxy, butoxy, pentoxy, diphenylamino, naphthylphenylamino, dinaphthylamino, dianthranylamino, diphenanthrenylamino, and dipyrenylamino. Preferred examples thereof include cyano, methyl, ethyl, propyl, butyl, pentyl, hexyl, heptyl, octyl, diphenylamino, naphthylphenylamino, or dinaphthylamino.

In the present specification, the linked aromatic group refers to an aromatic group in which the carbon atoms of the aromatic rings in the aromatic group are linked to each other by a single bond. It is an aromatic group in which two or more aromatic groups are linked to each other, and they may be linear or branched. The aromatic group may be an aromatic hydrocarbon group or an aromatic heterocyclic group, and the plurality of aromatic groups may be the same or different. The aromatic group corresponding to the linked aromatic group is different from the substituted aromatic group.

L¹ represents a substituted or unsubstituted aromatic hydrocarbon group having 6 to 18 carbon atoms, or a substituted or unsubstituted linked aromatic group in which two to five of these aromatic hydrocarbon groups are linked to each other. Preferred is a substituted or unsubstituted aromatic hydrocarbon group having 6 to 18 carbon atoms, and more preferred is a substituted or unsubstituted phenylene group. The linking scheme may be any of ortho-, meta-, or para-linking. In the case of L¹ being an unsubstituted aromatic hydrocarbon group, specific examples thereof are the same as in the case of Ar¹ and Ar² being each an unsubstituted aromatic hydrocarbon group. Note that L¹ is a divalent group.

Specific examples of the compounds represented by the general formula (1) are shown below, but are not limited to these exemplified compounds.

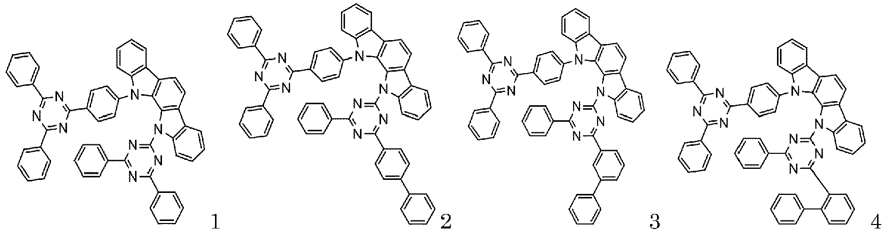

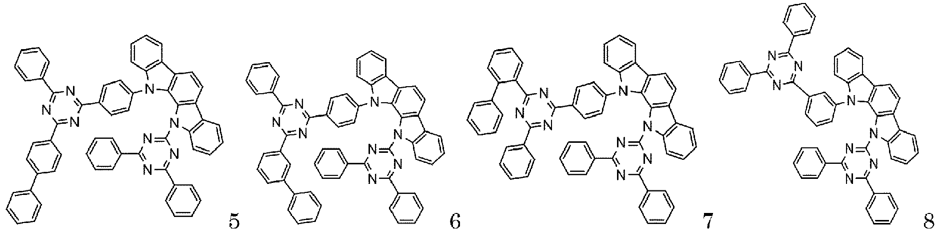

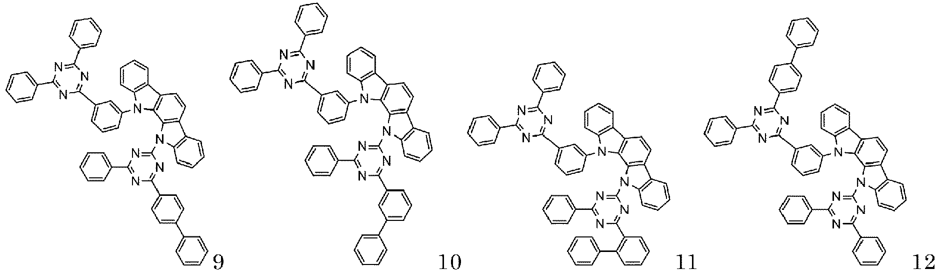

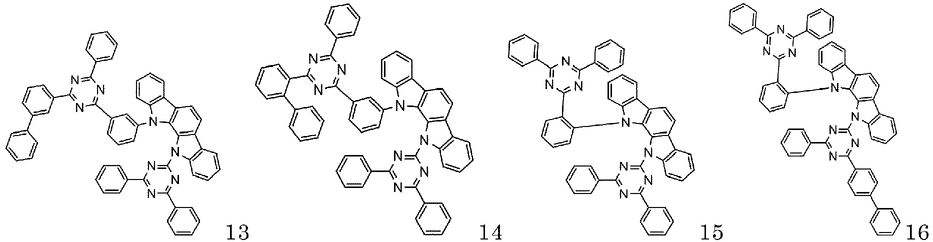

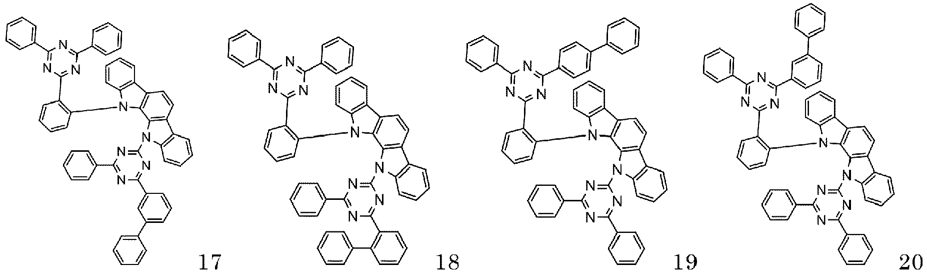

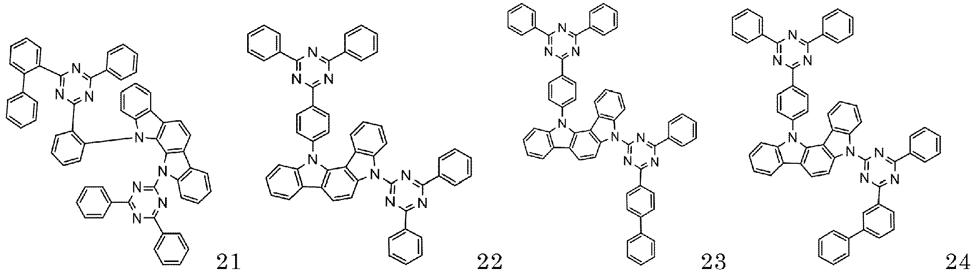

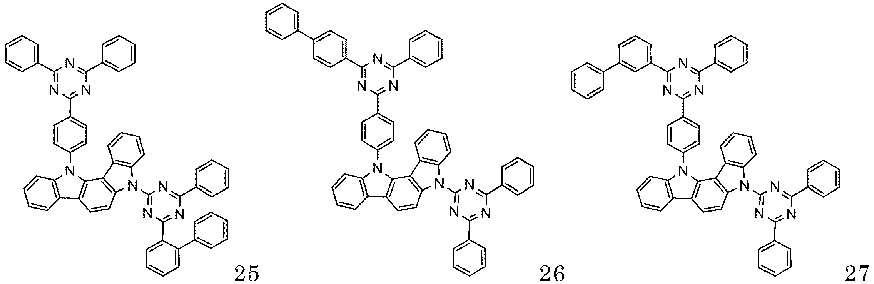

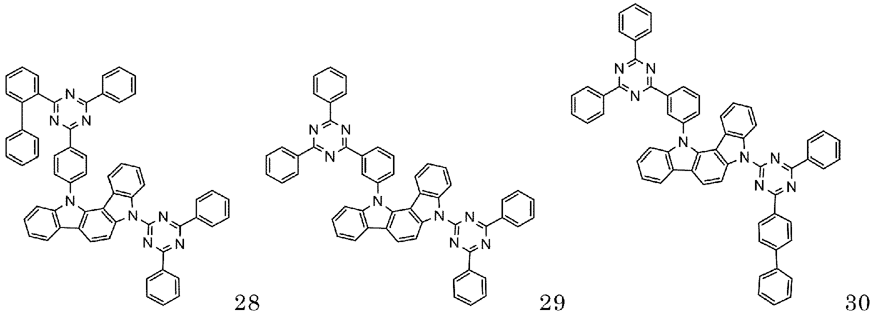

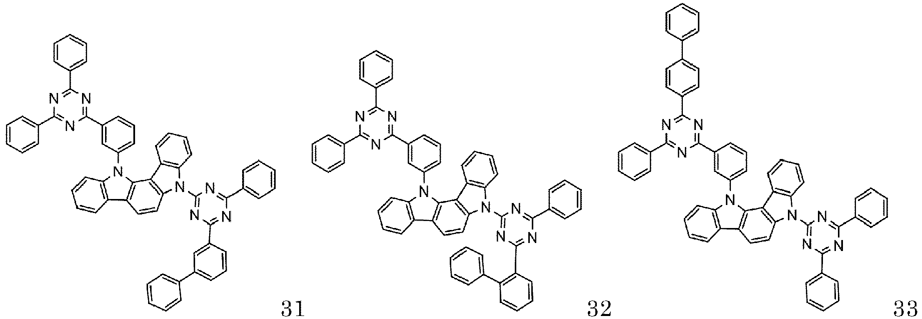

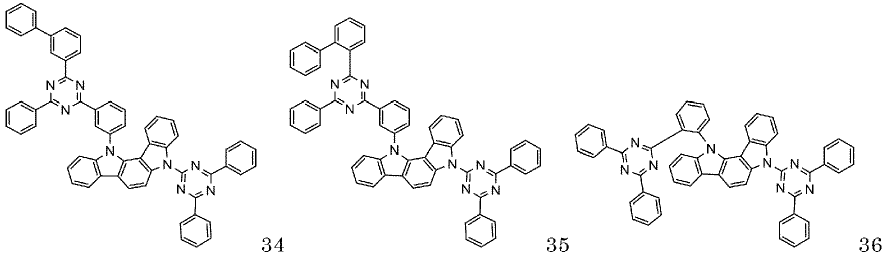

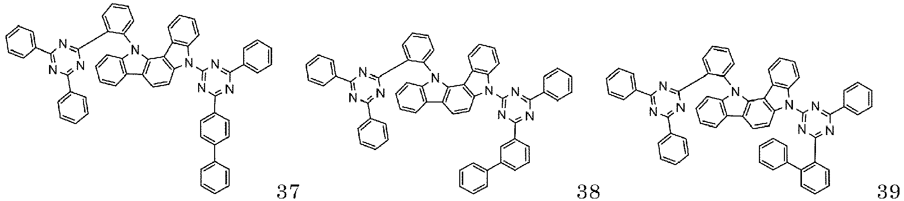

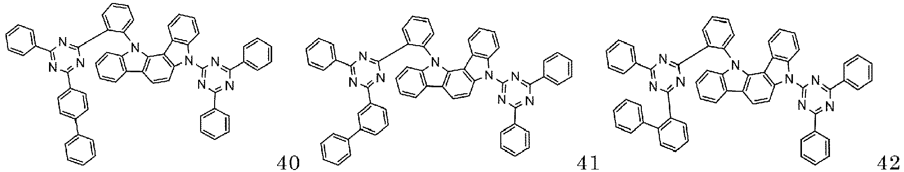

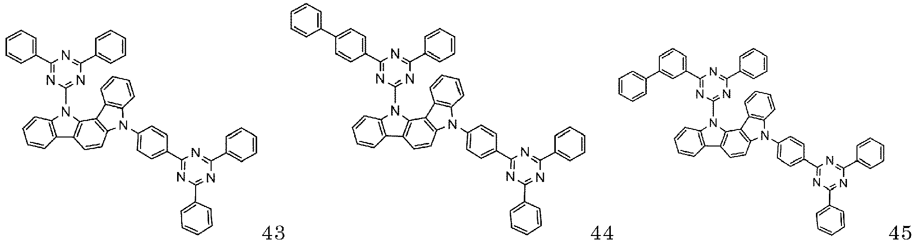

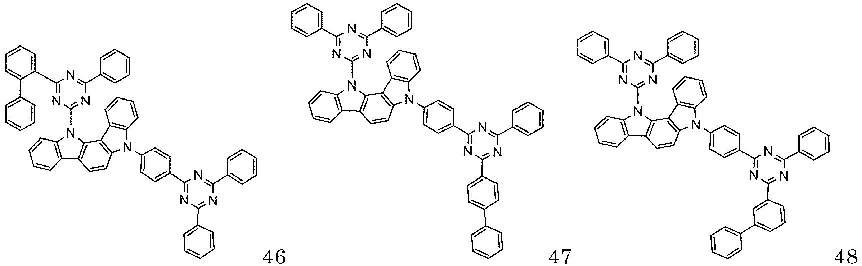

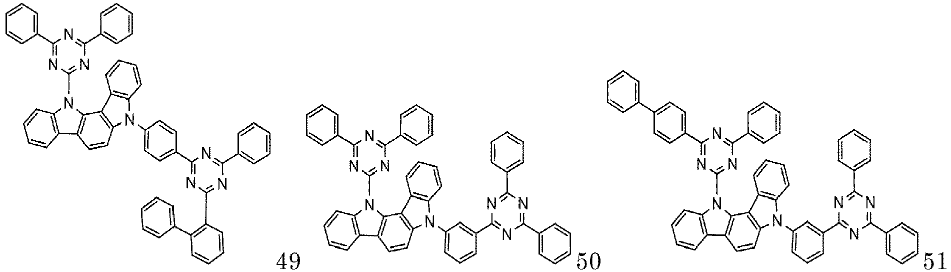

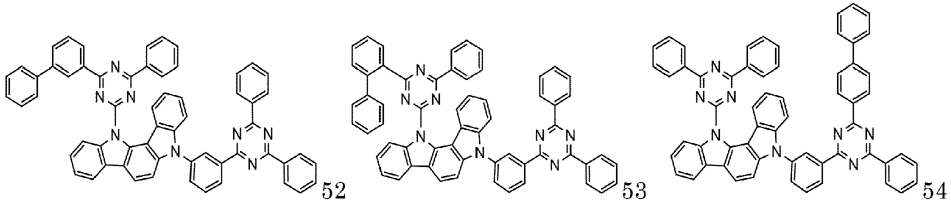

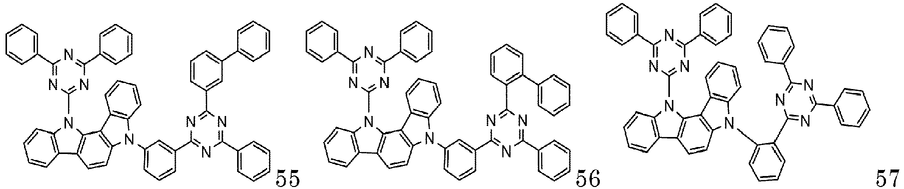

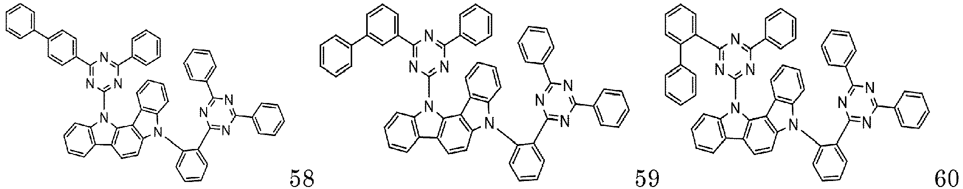

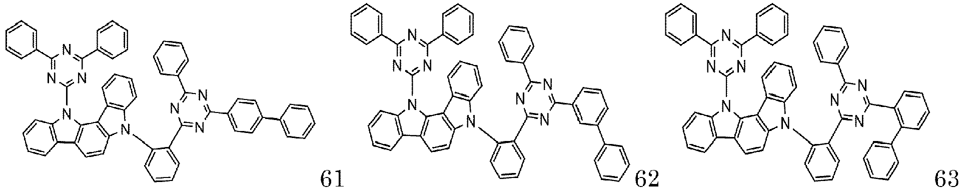

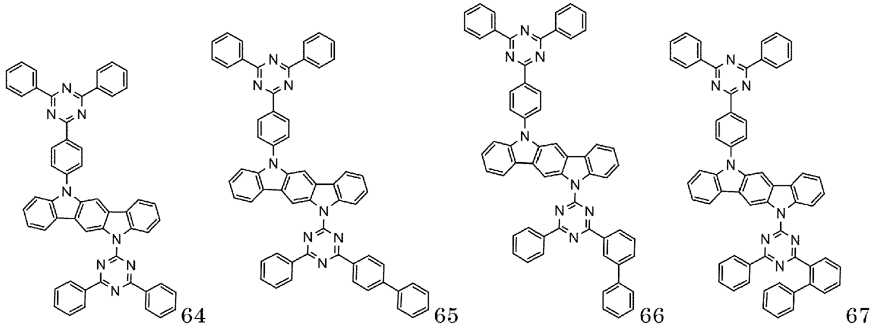

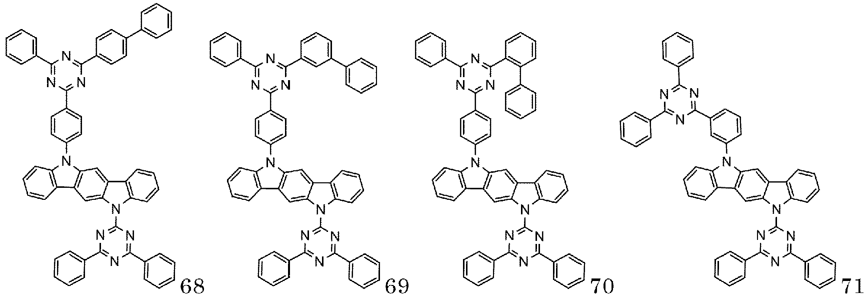

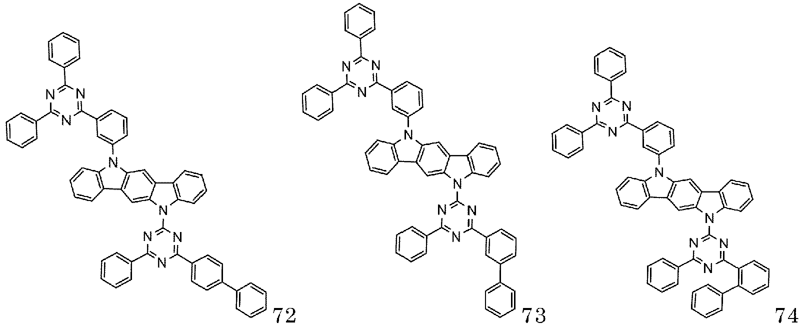

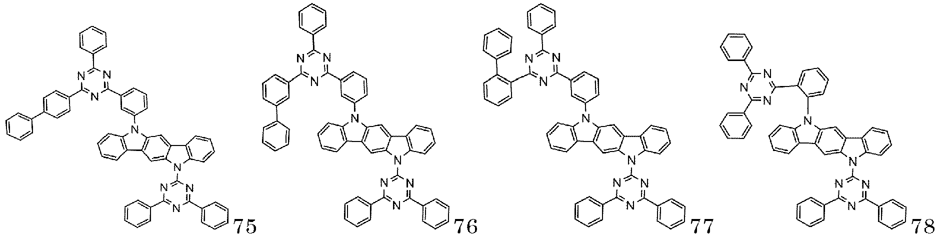

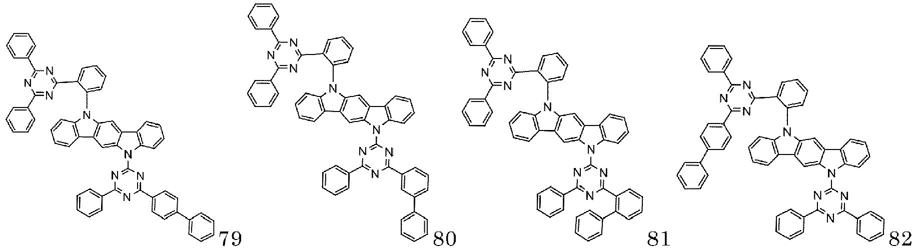

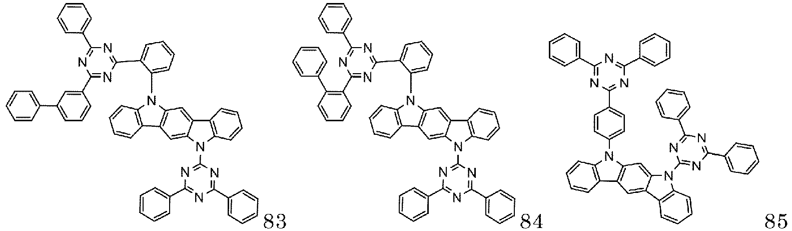

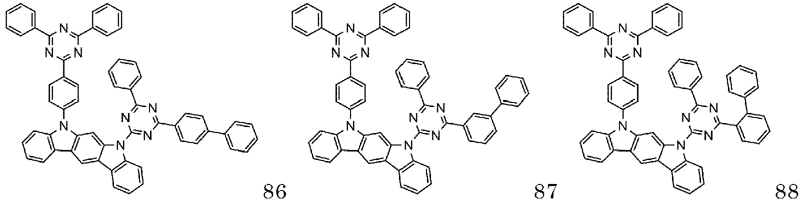

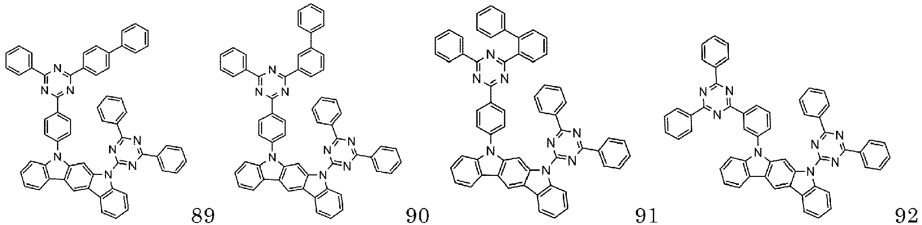

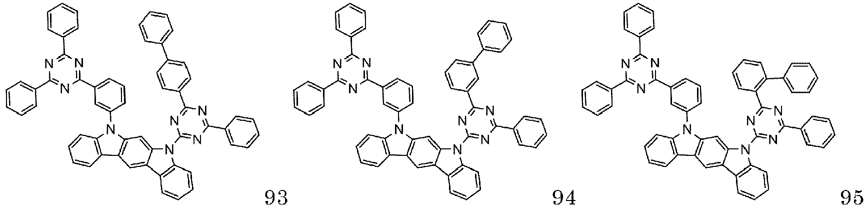

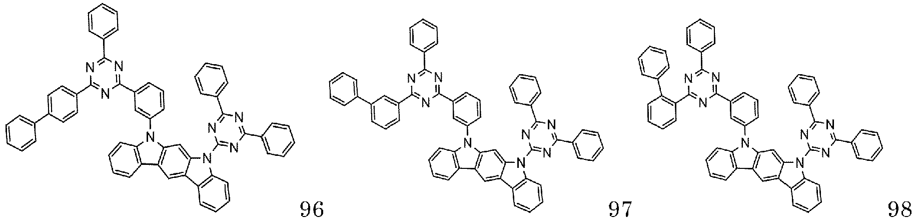

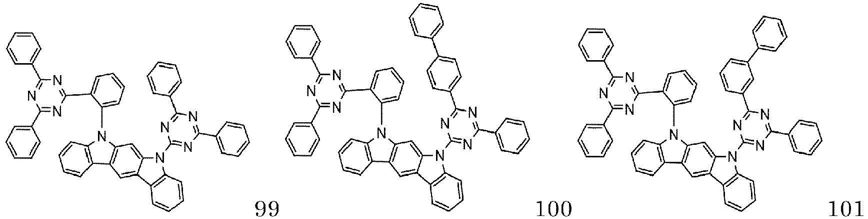

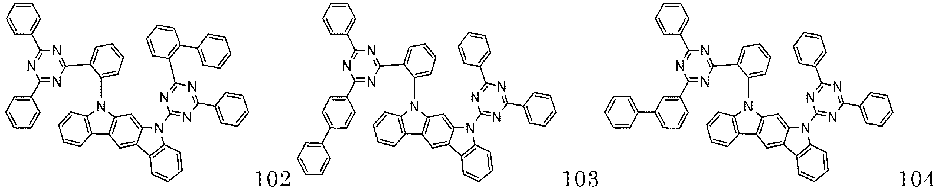

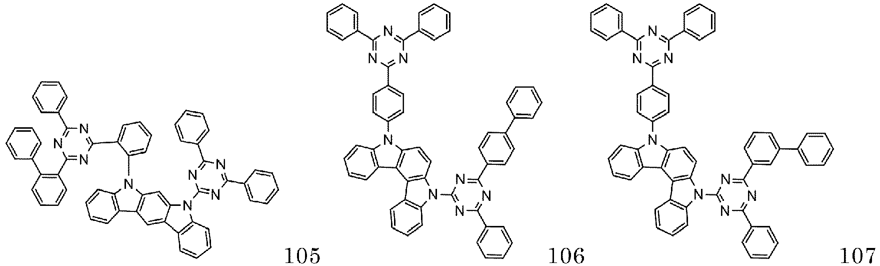

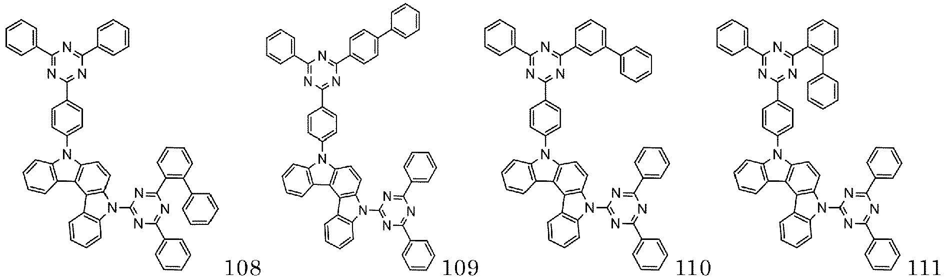

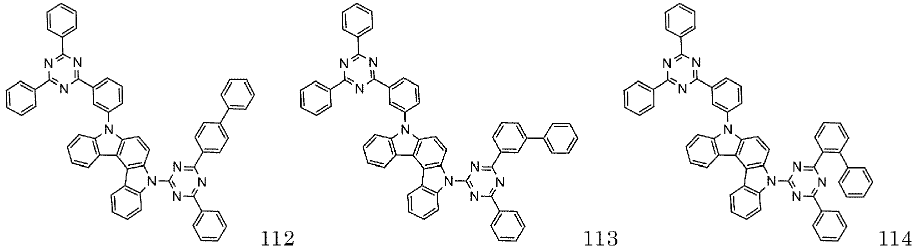

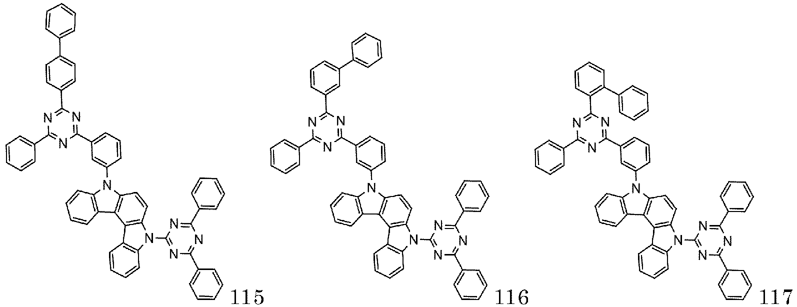

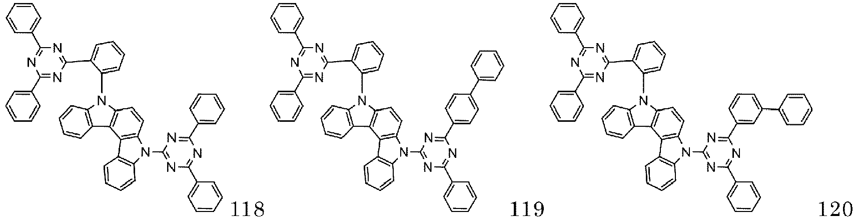

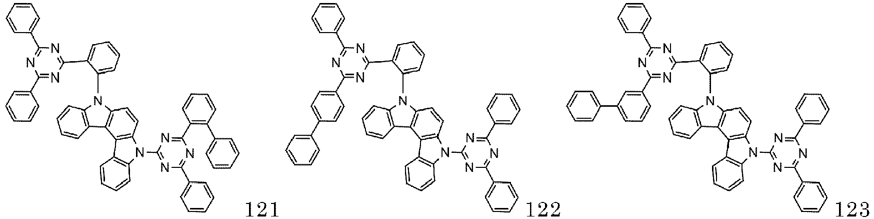

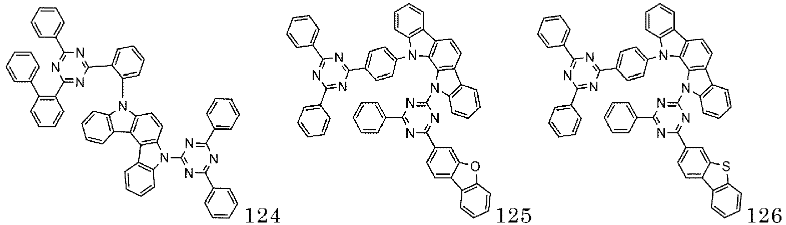

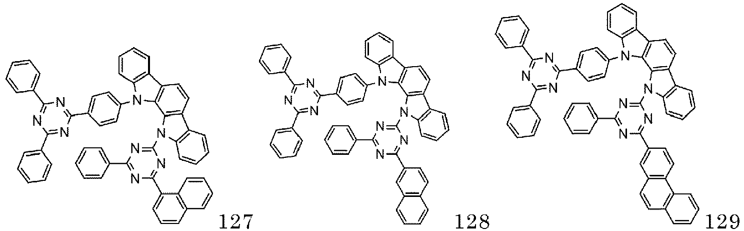

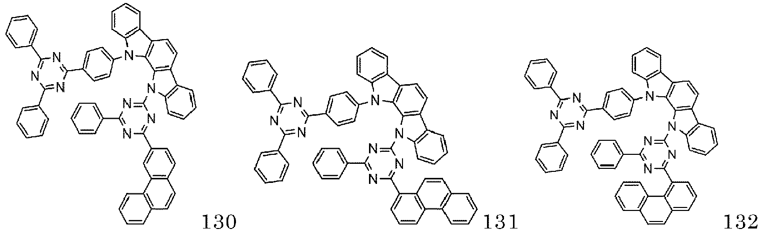

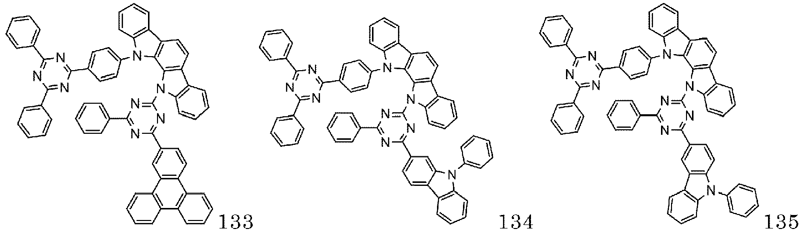

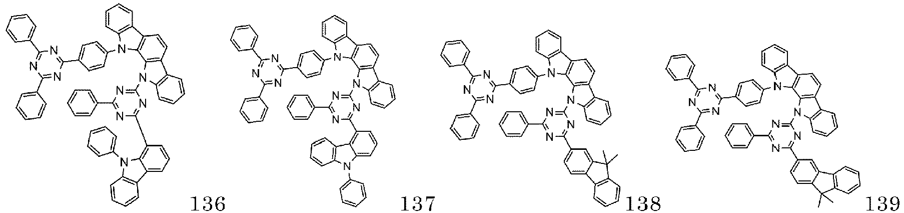

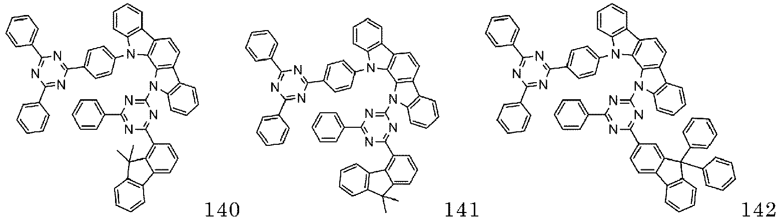

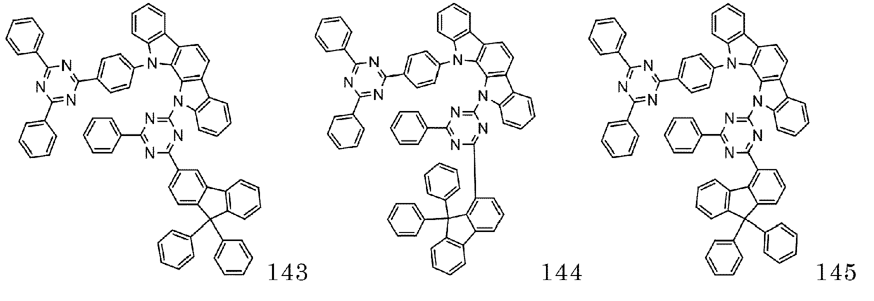

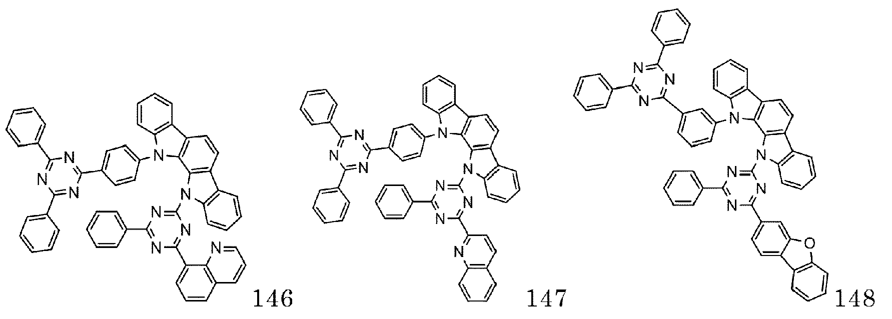

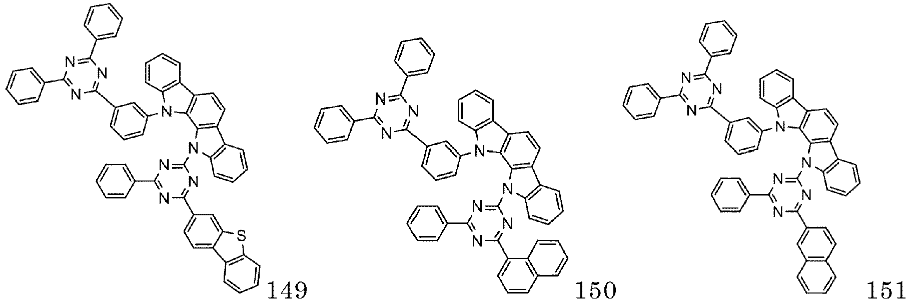

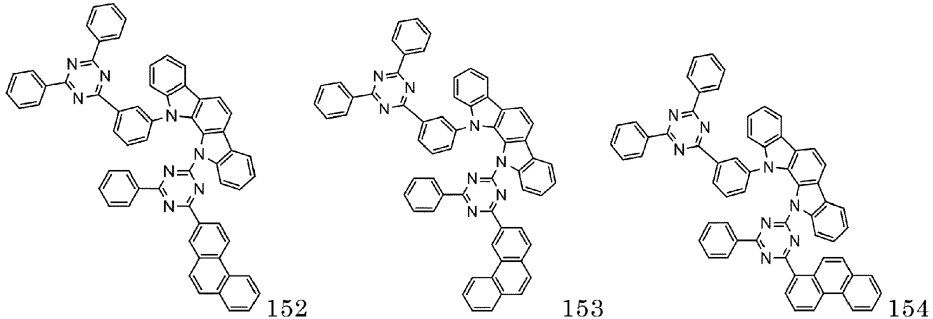

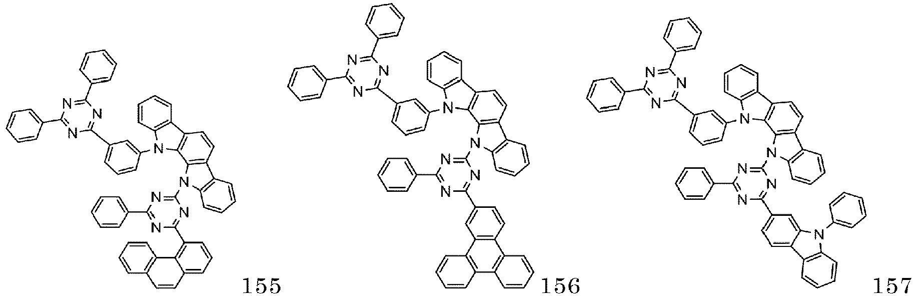

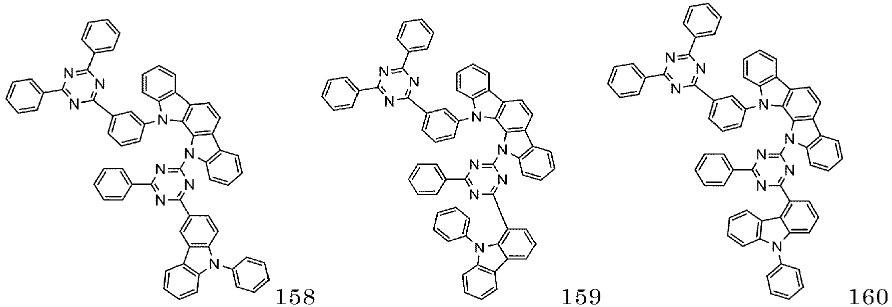

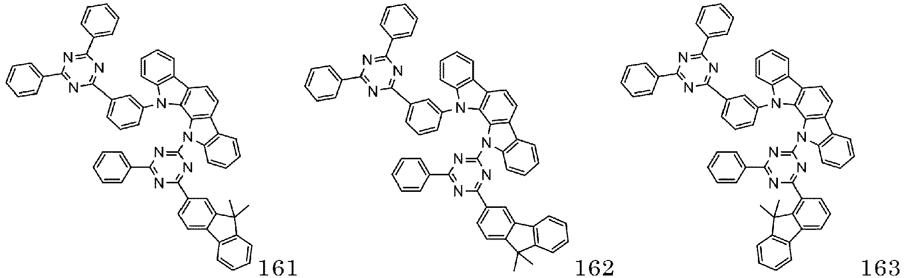

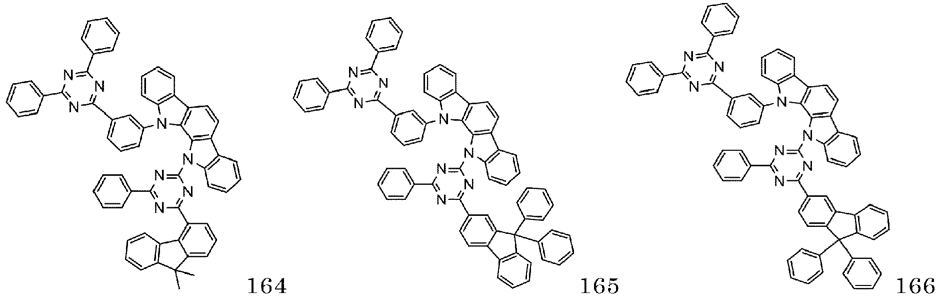

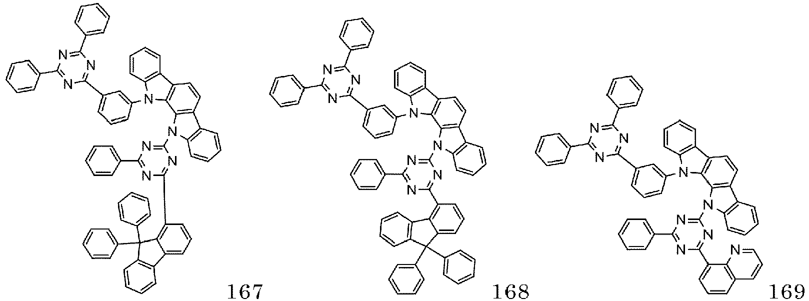

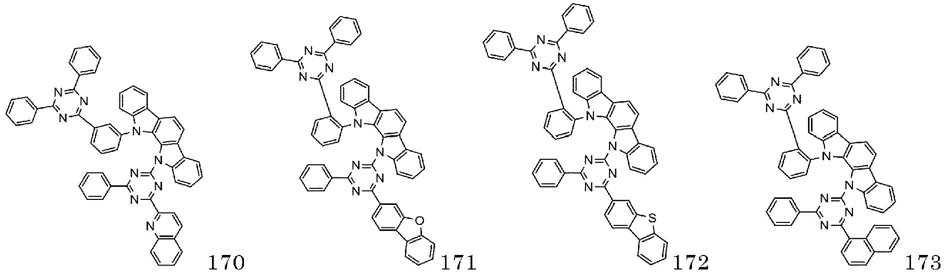

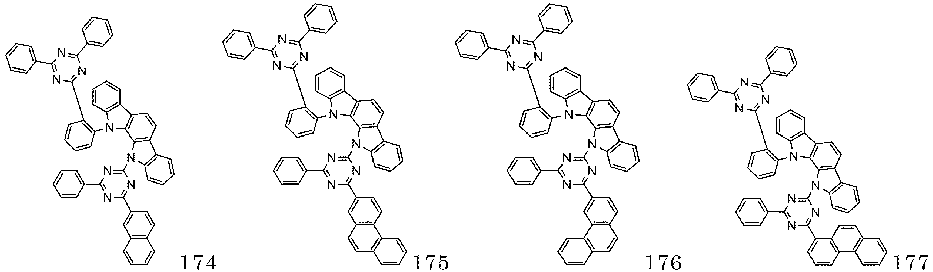

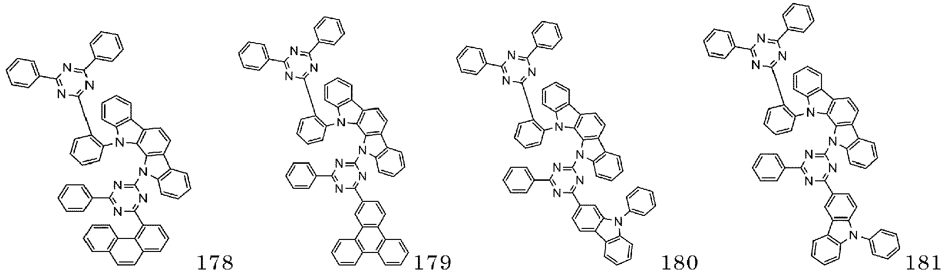

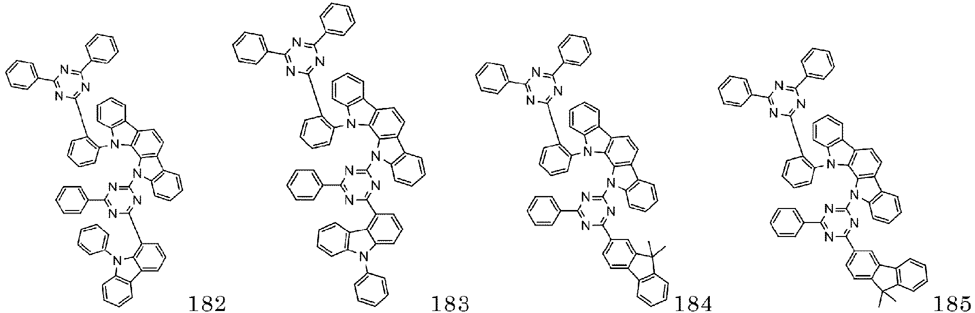

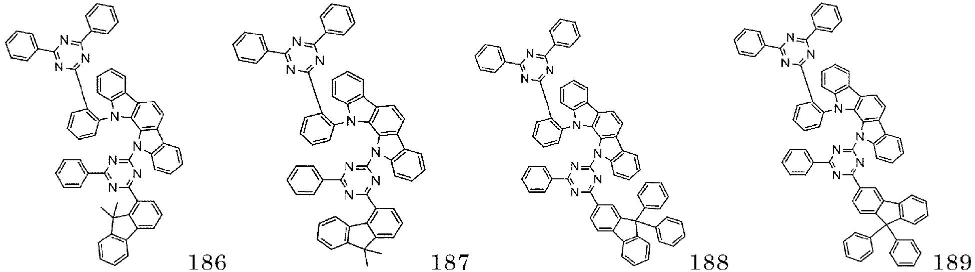

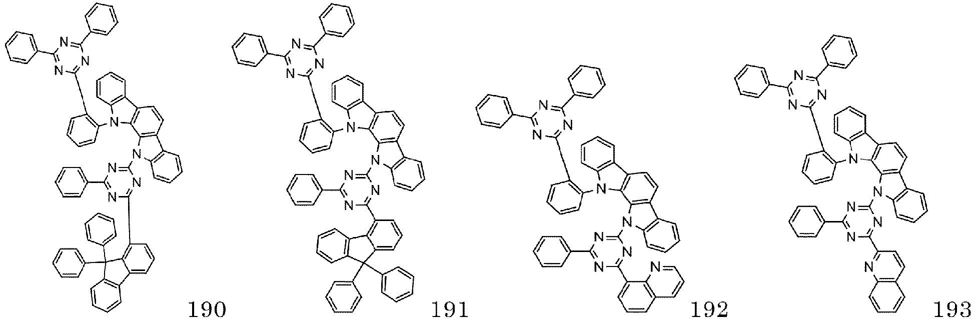

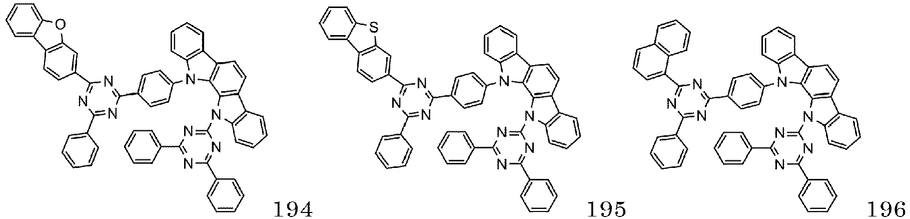

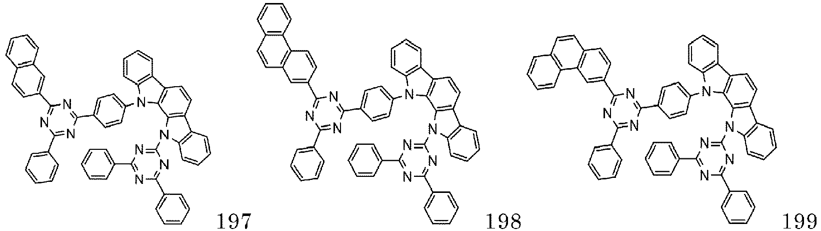

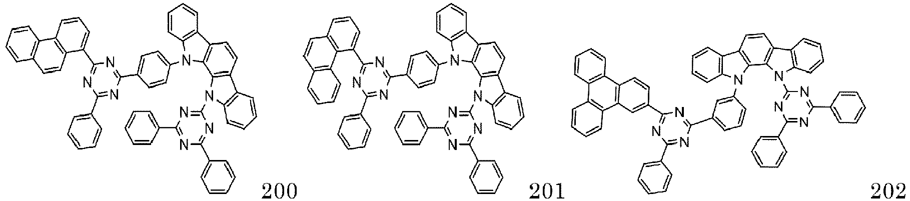

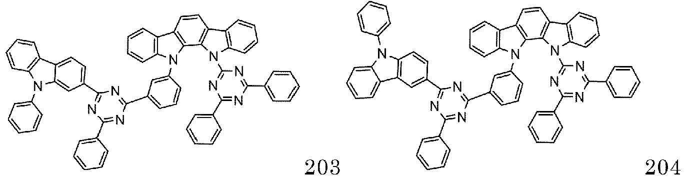

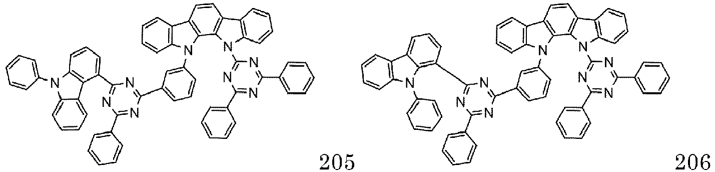

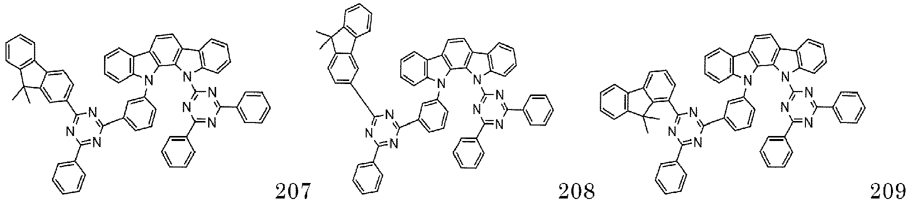

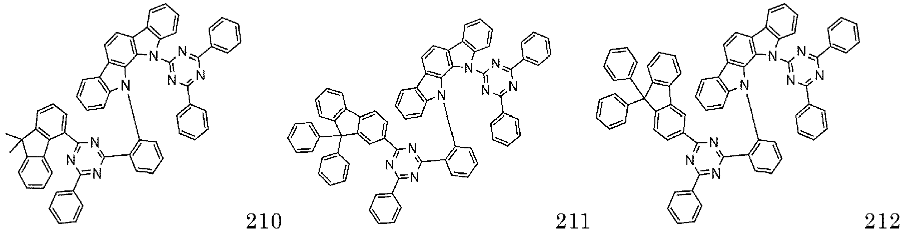

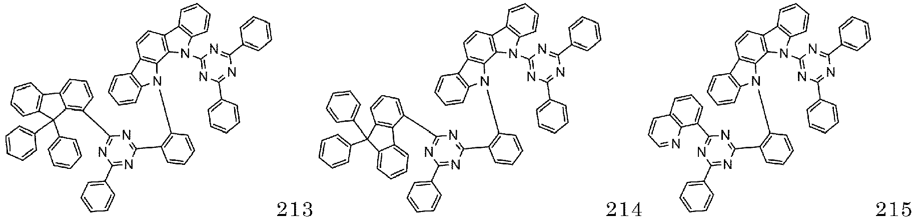

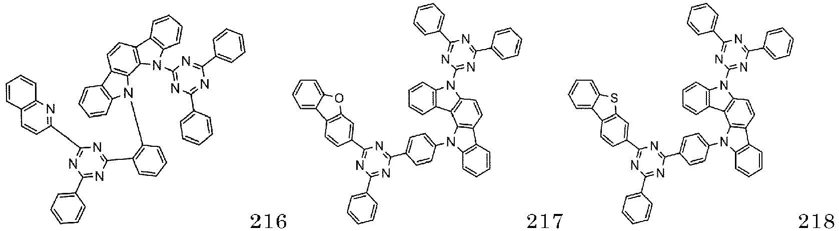

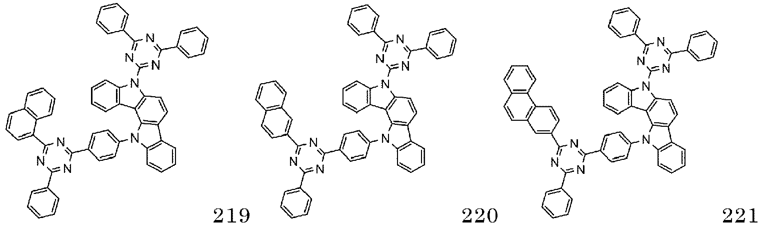

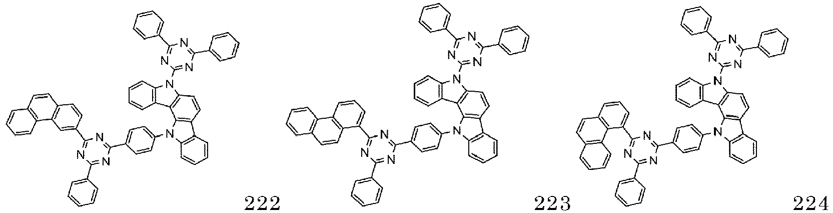

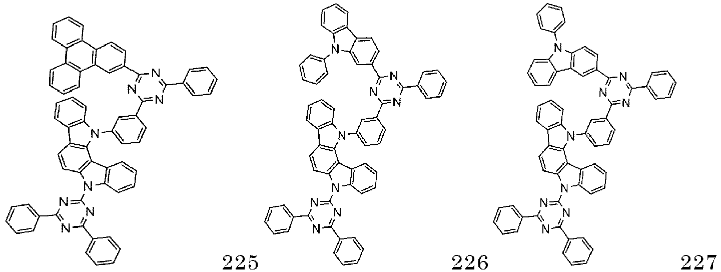

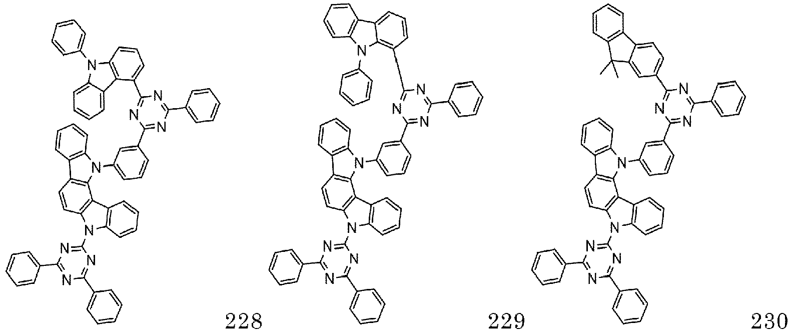

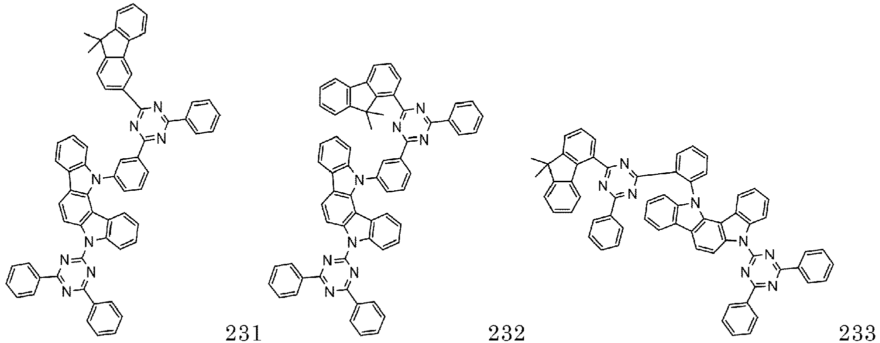

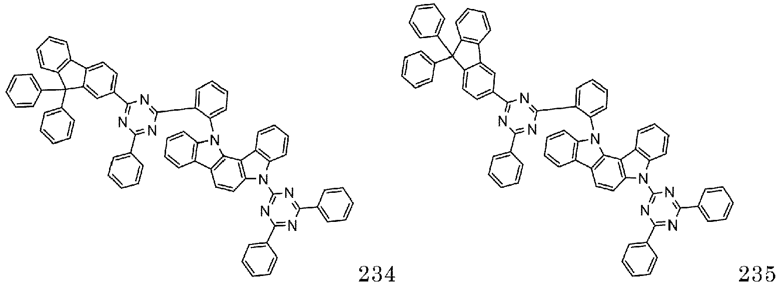

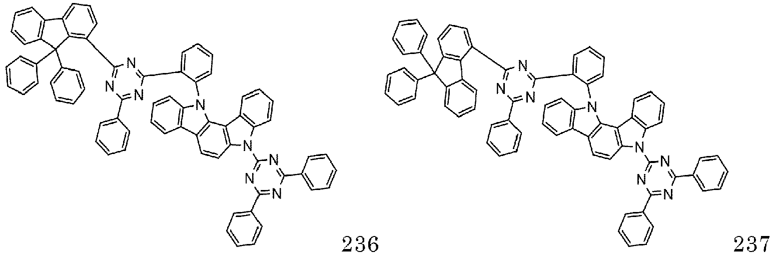

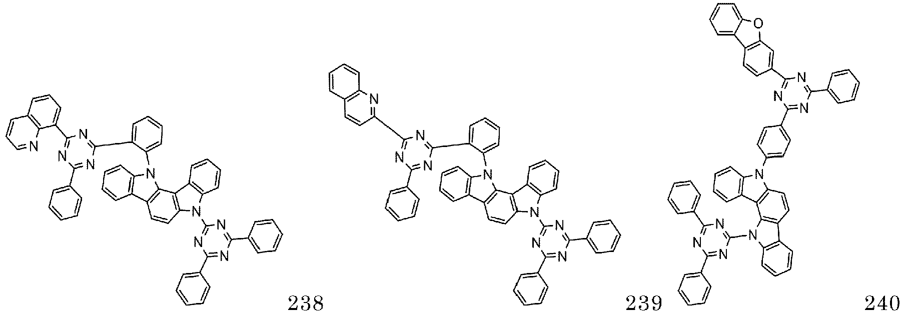

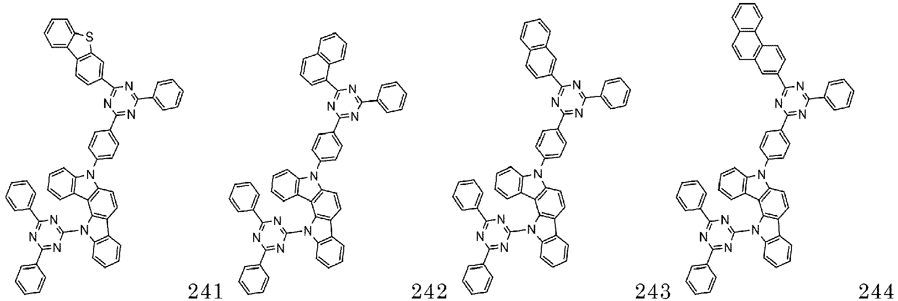

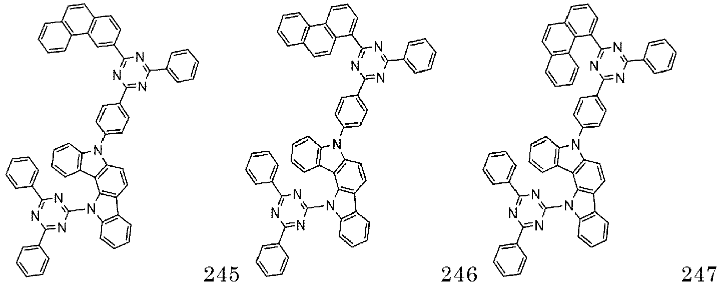

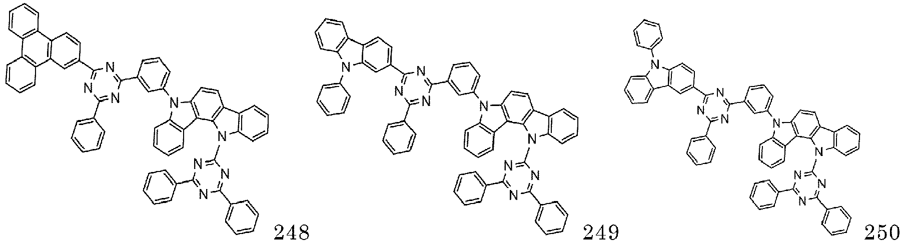

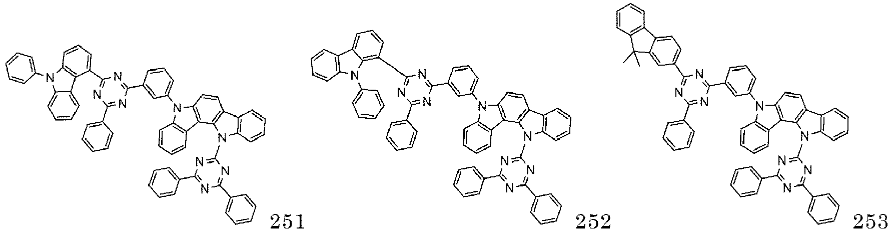

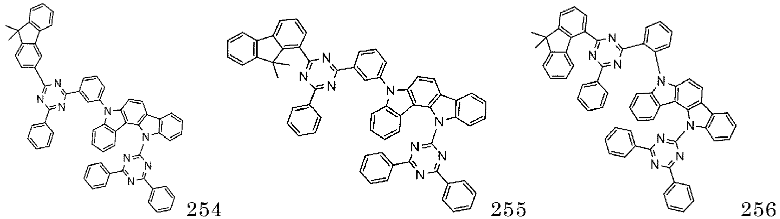

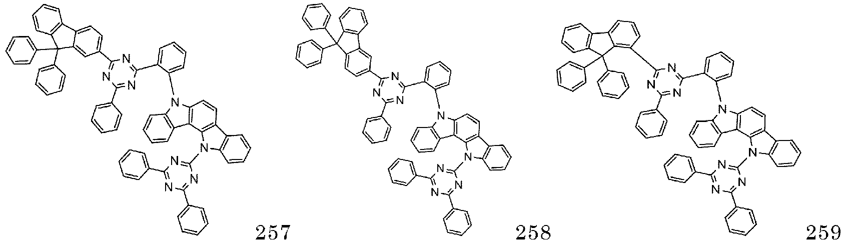

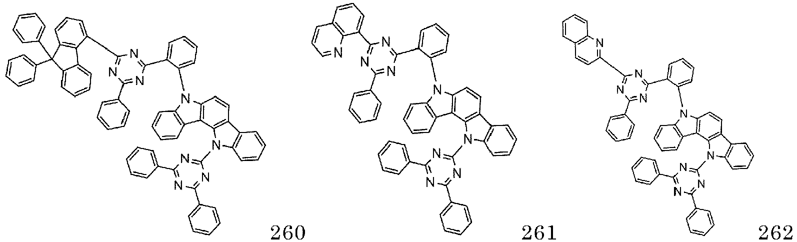

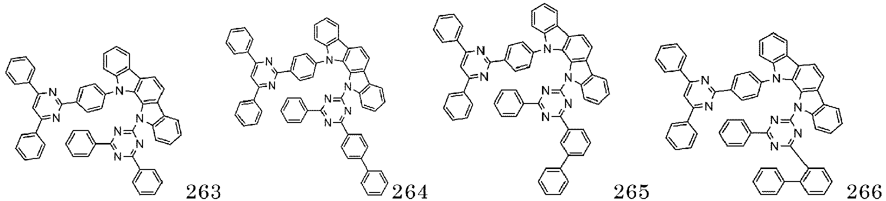

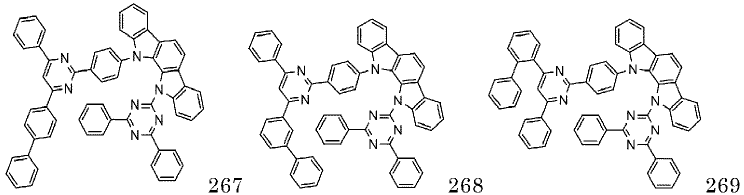

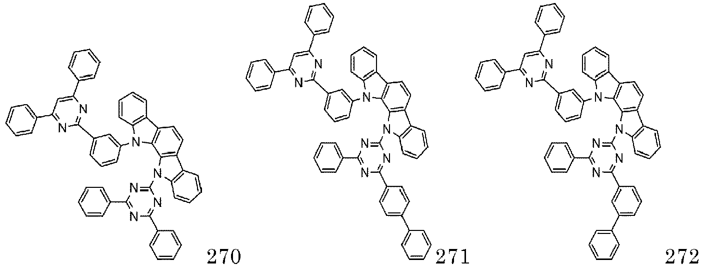

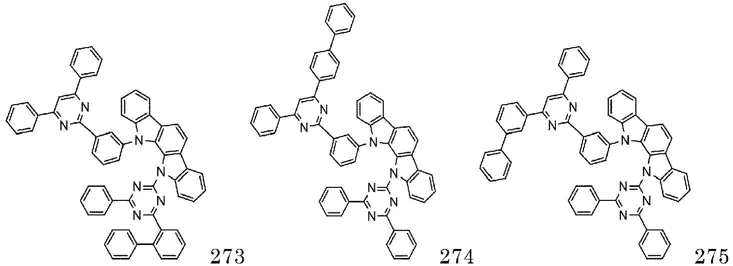

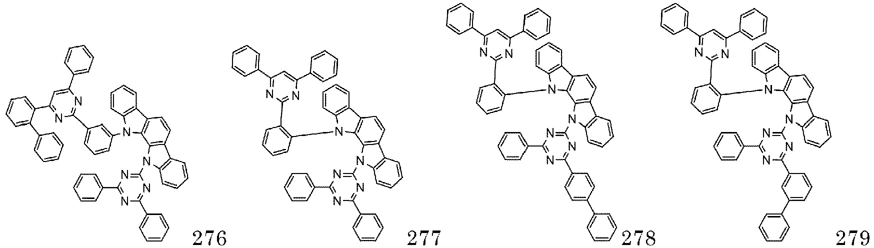

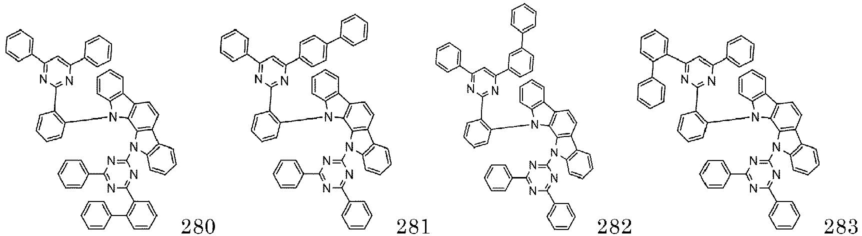

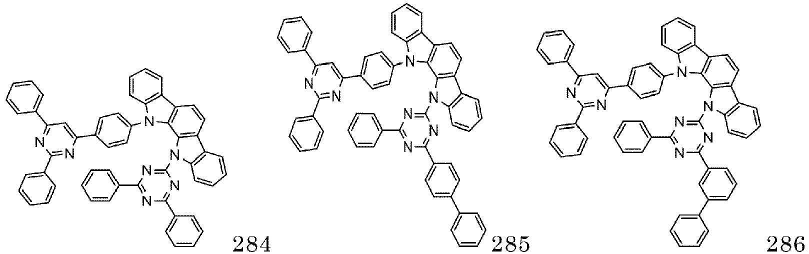

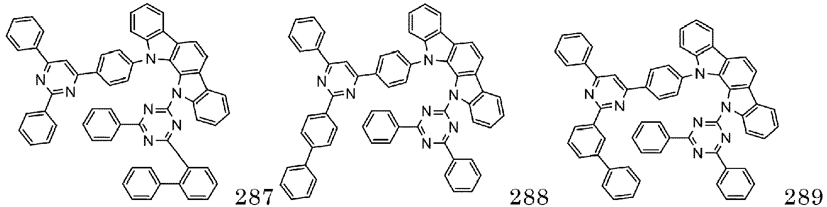

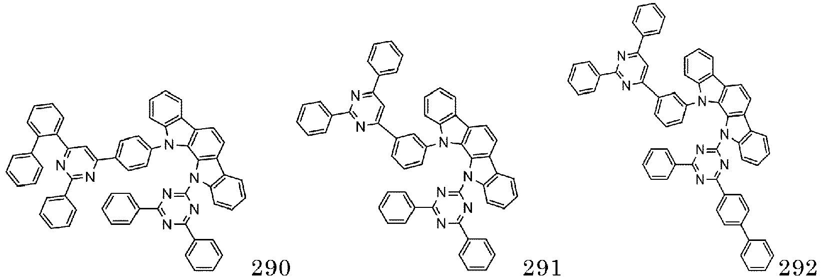

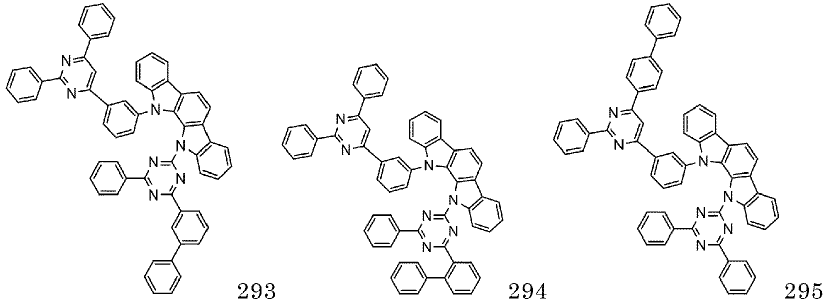

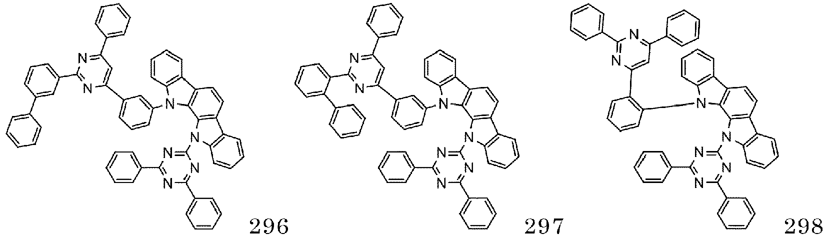

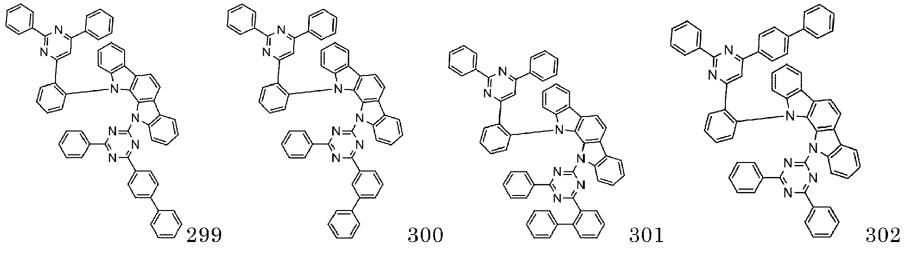

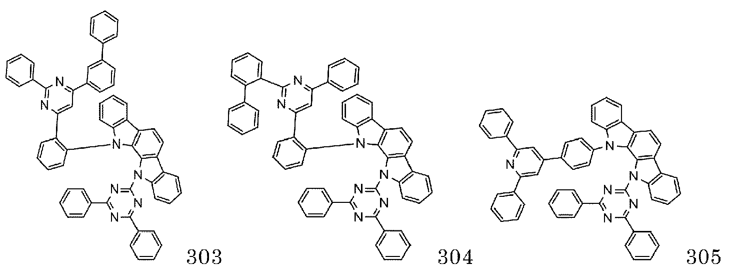

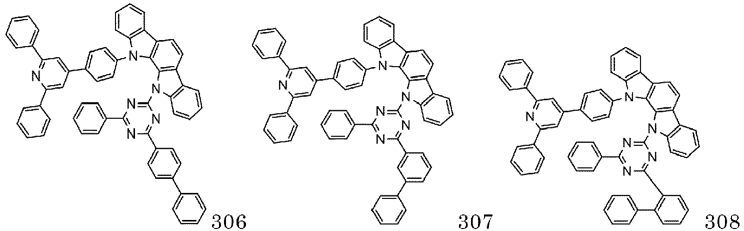

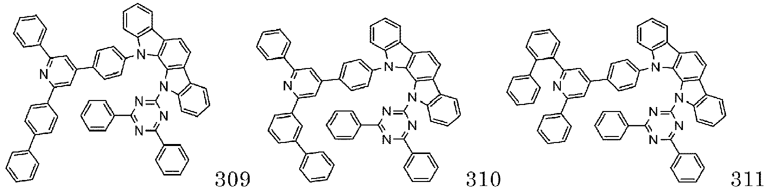

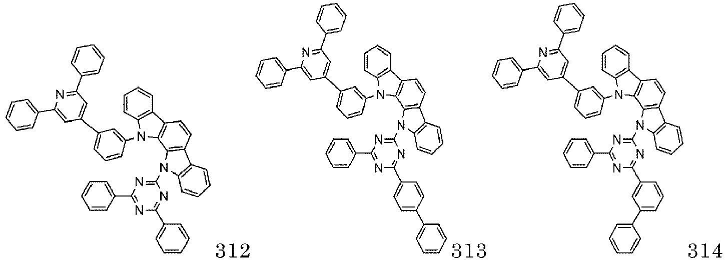

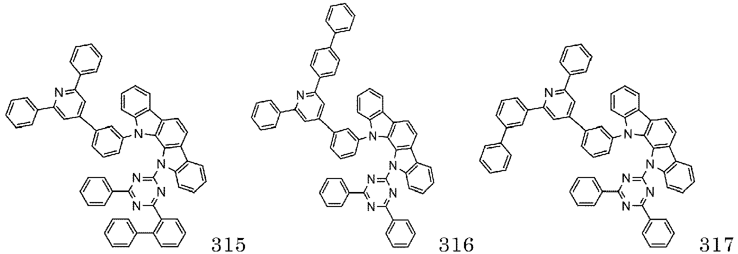

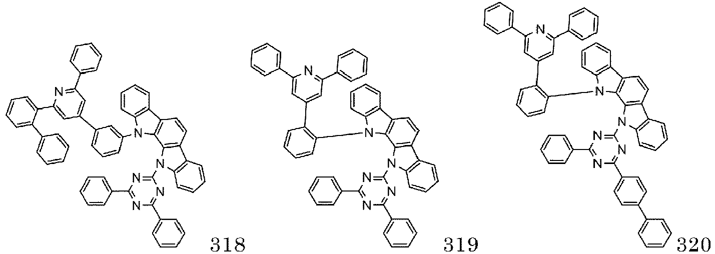

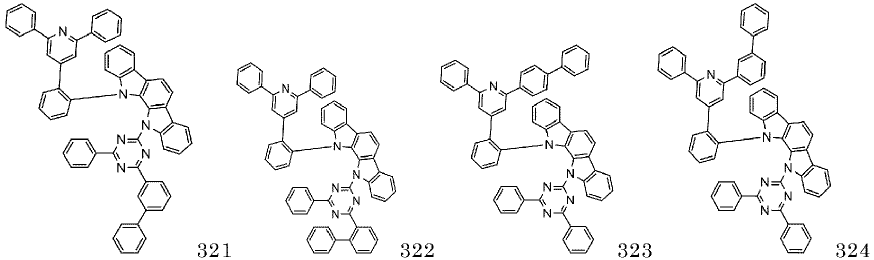

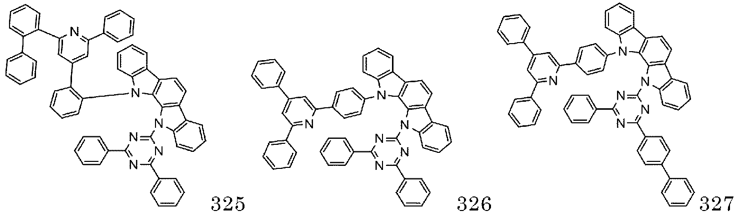

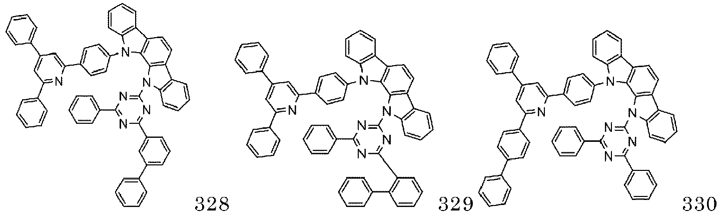

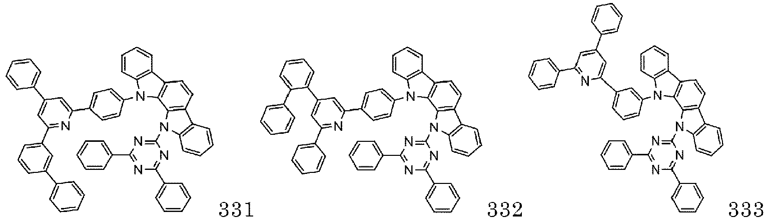

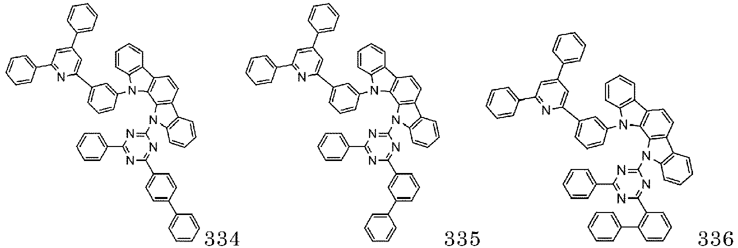

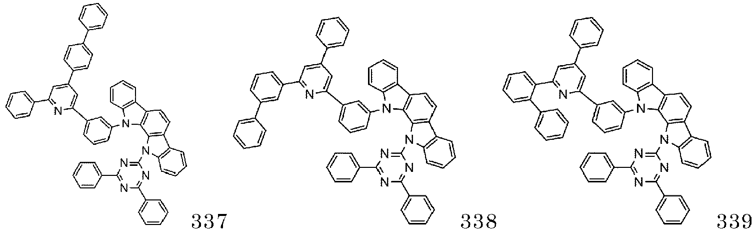

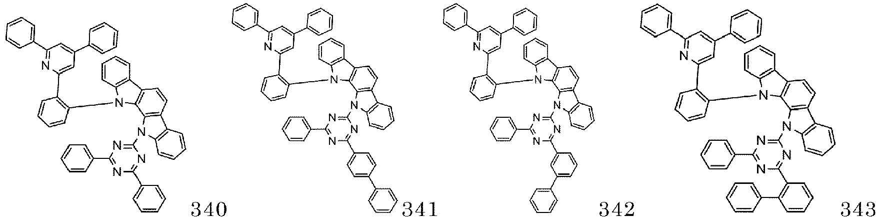

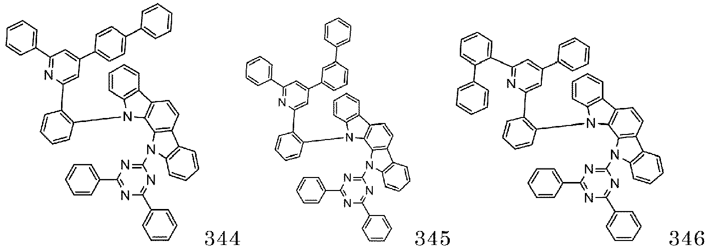

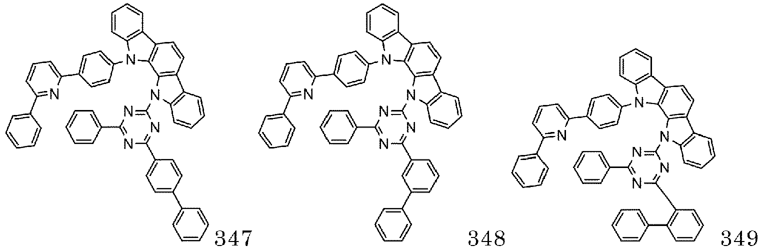

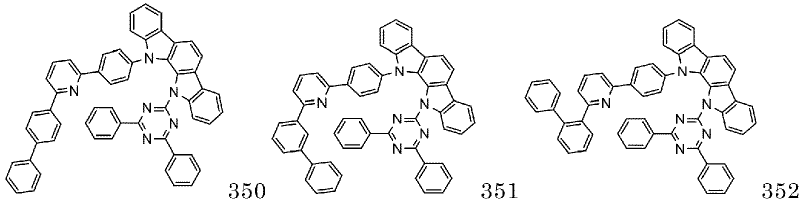

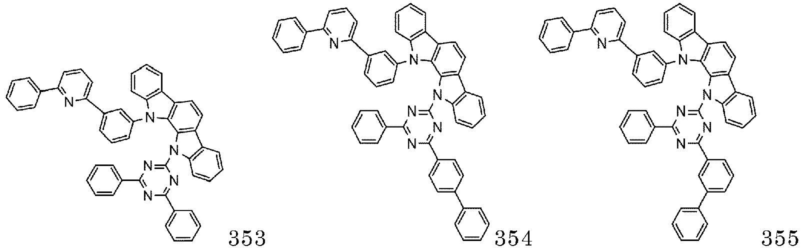

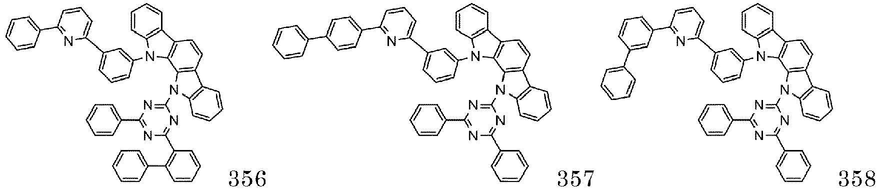

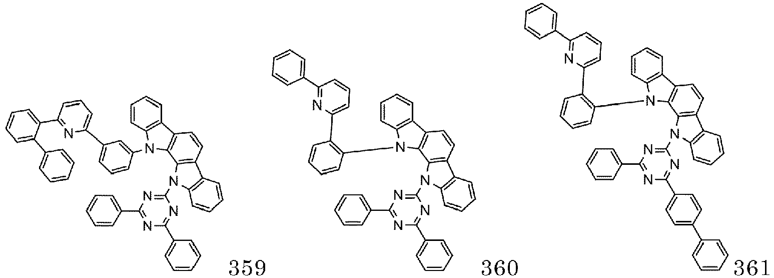

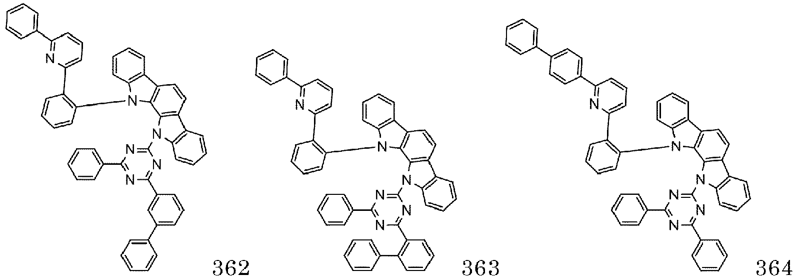

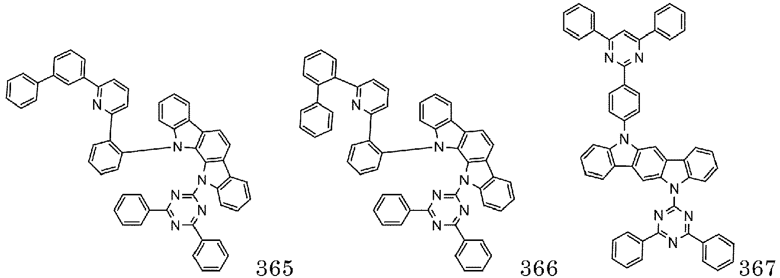

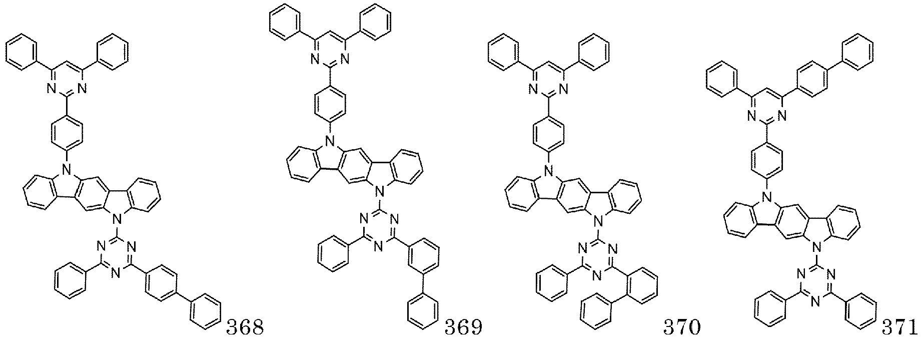

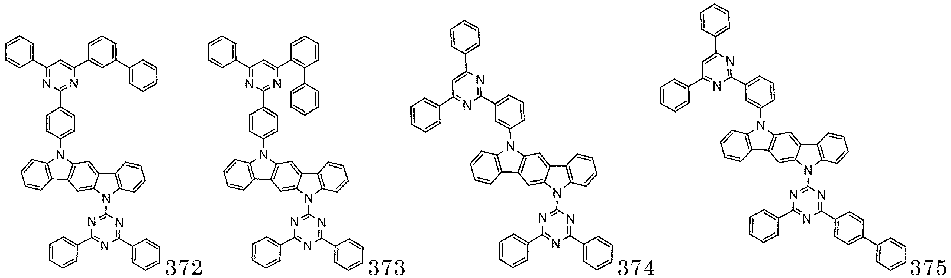

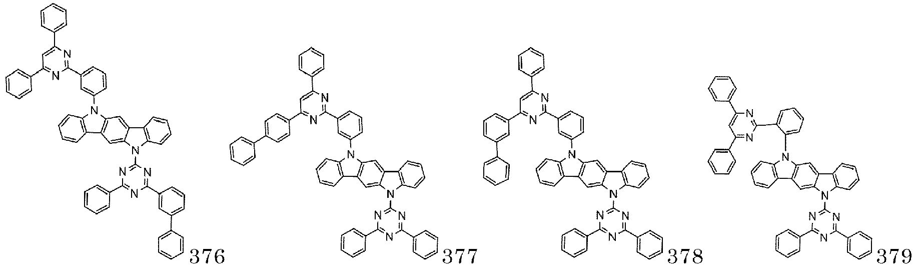

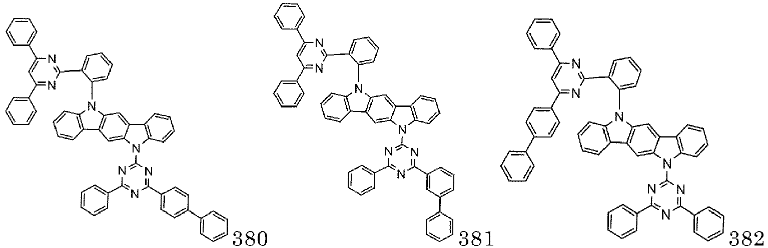

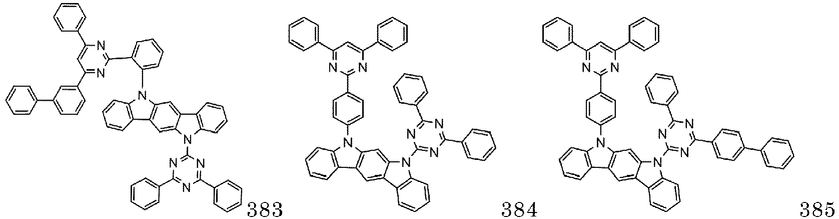

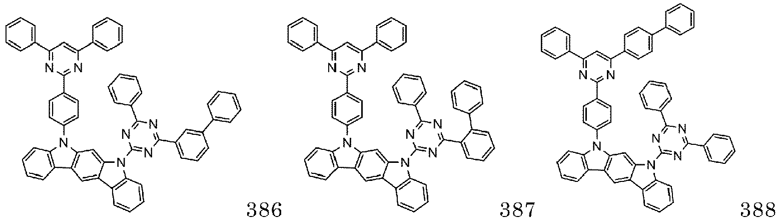

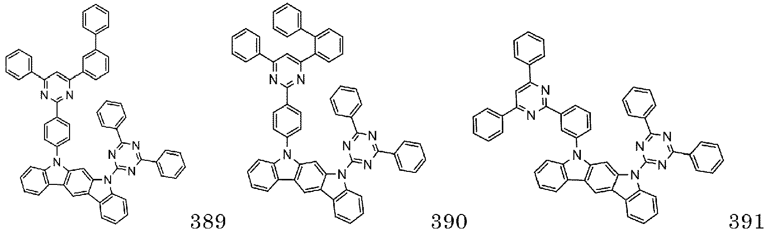

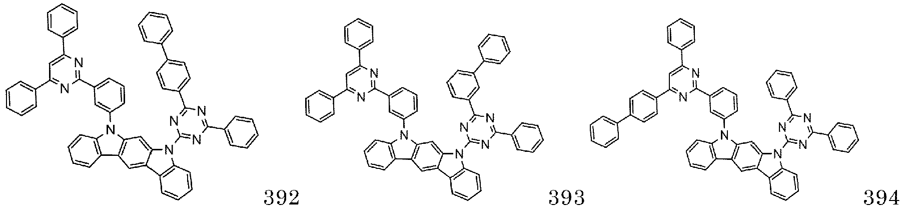

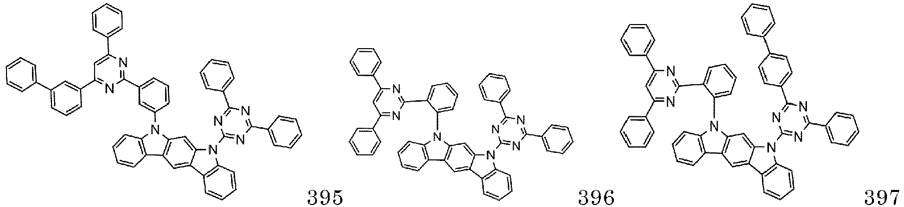

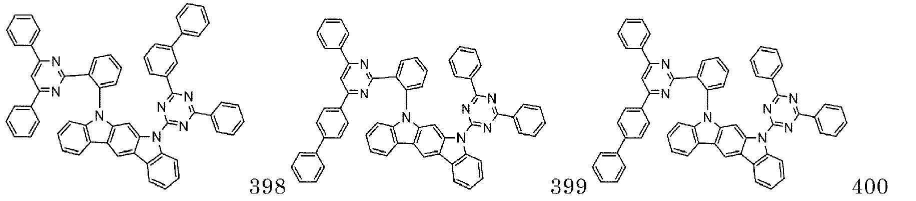

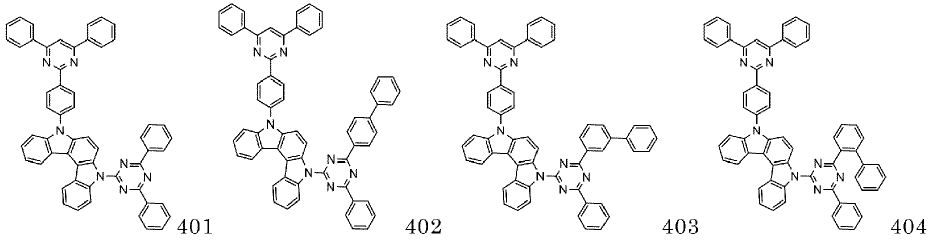

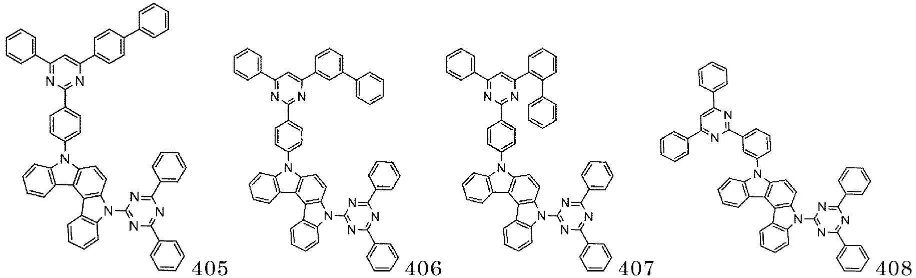

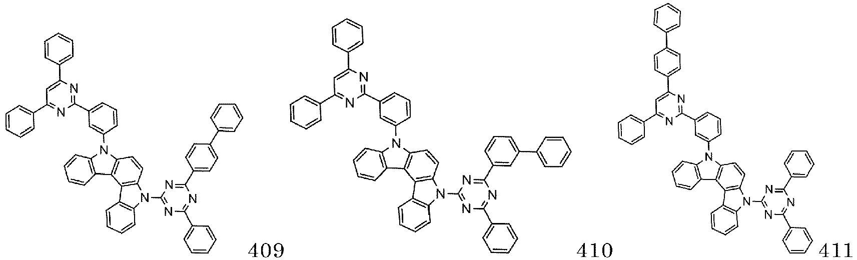

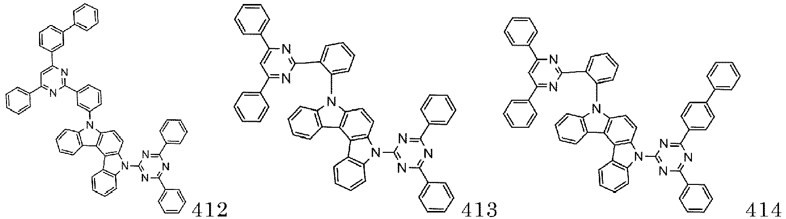

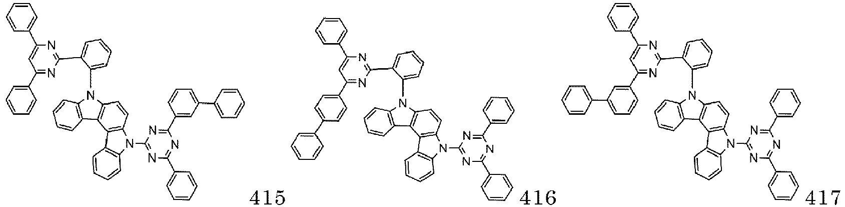

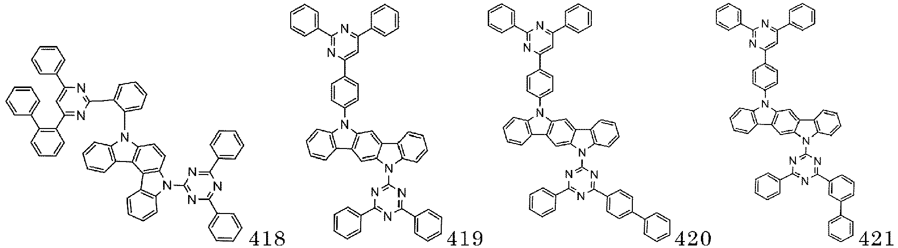

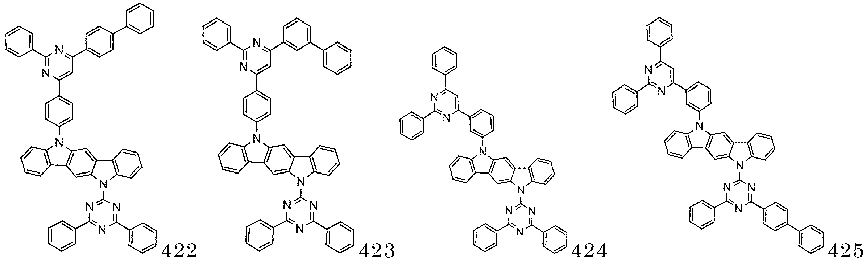

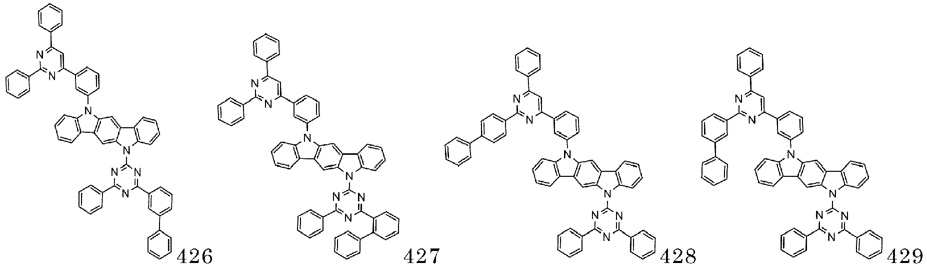

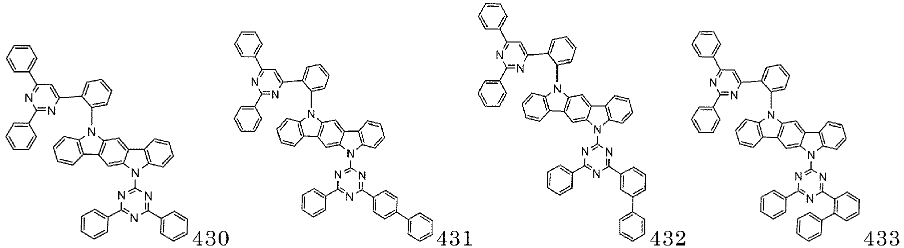

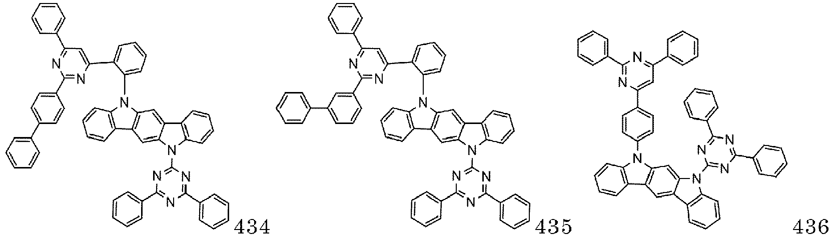

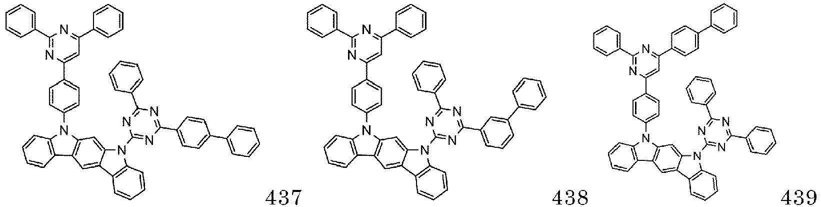

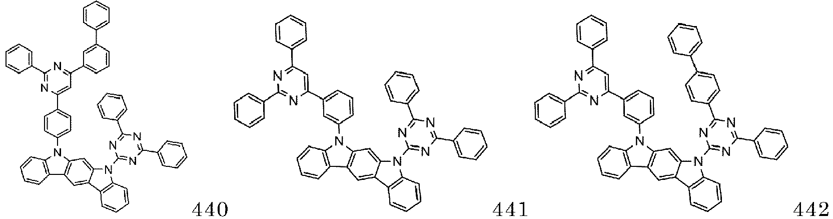

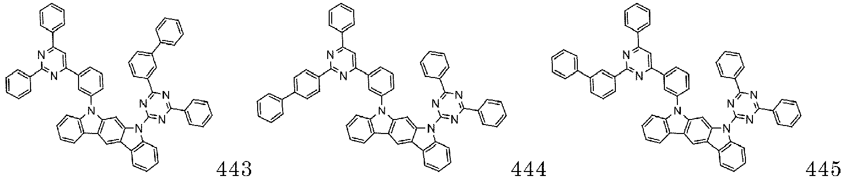

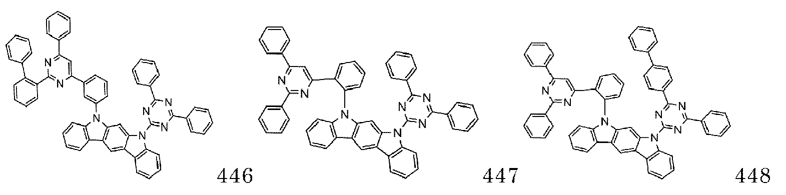

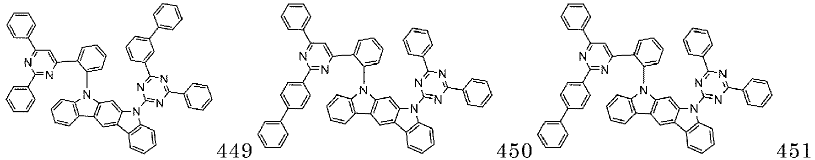

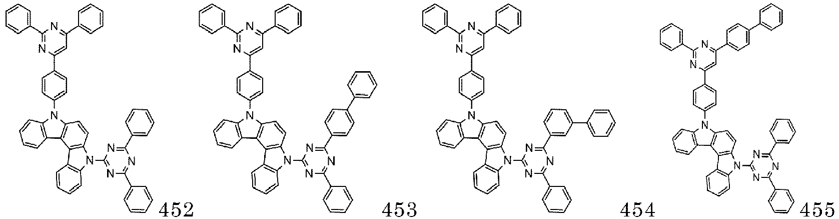

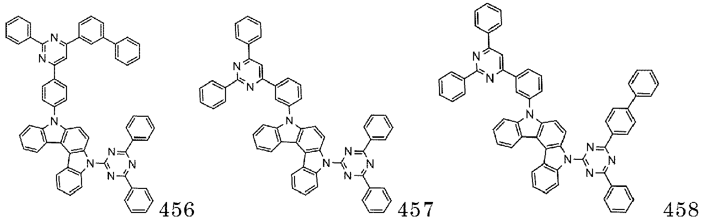

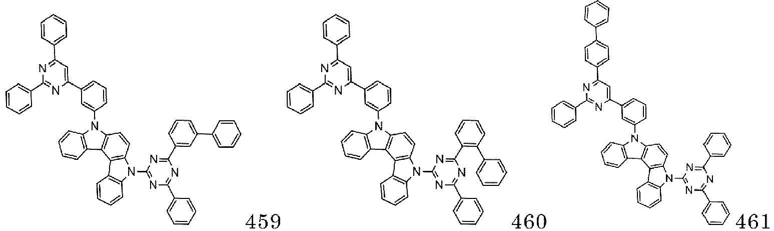

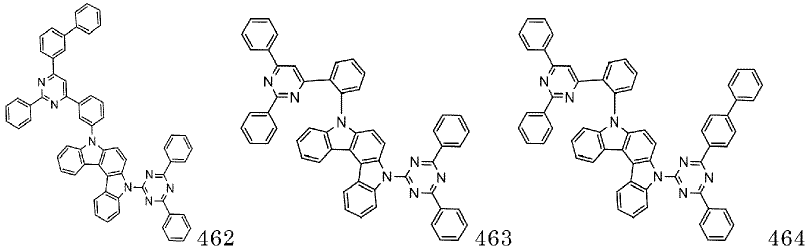

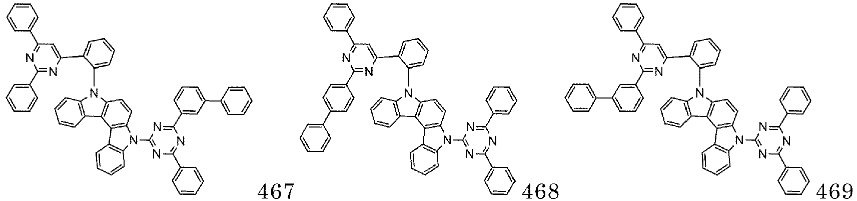

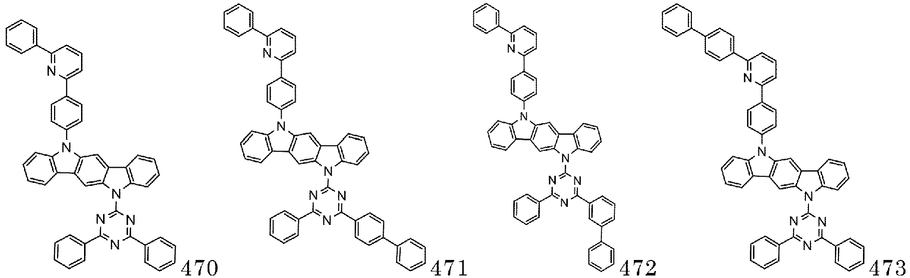

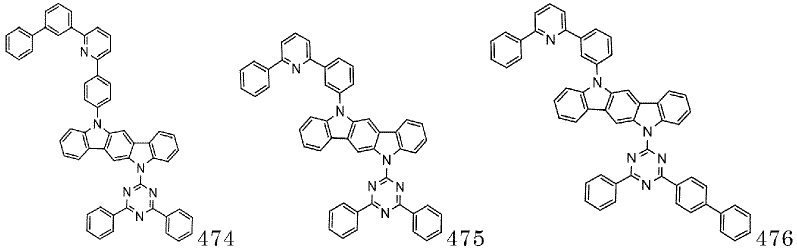

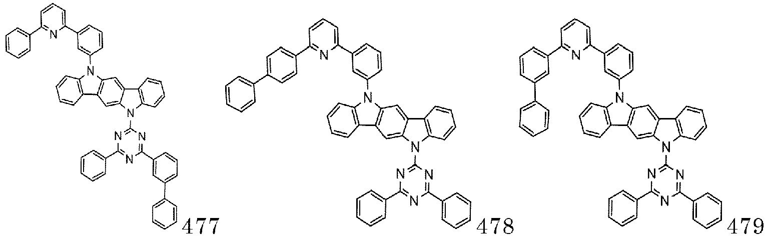

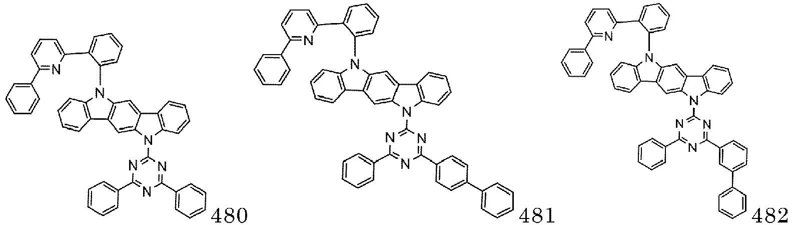

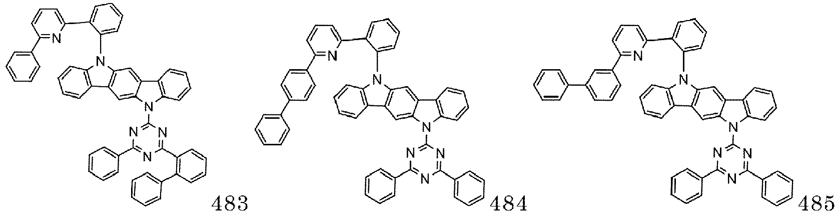

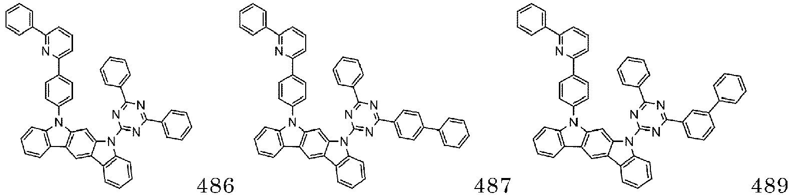

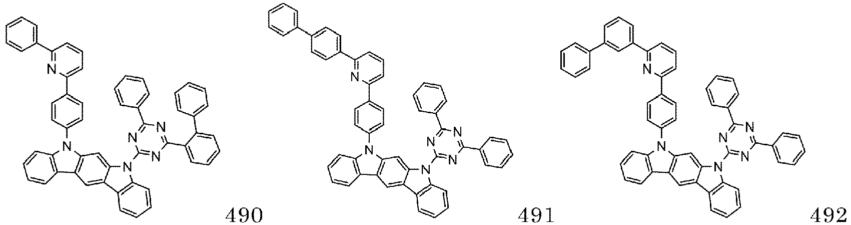

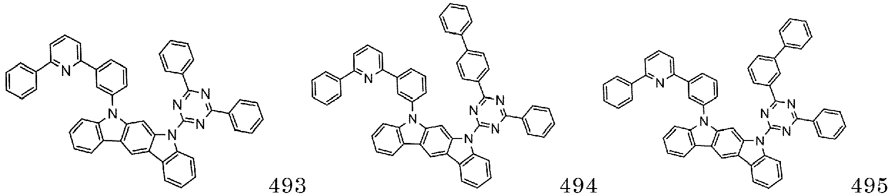

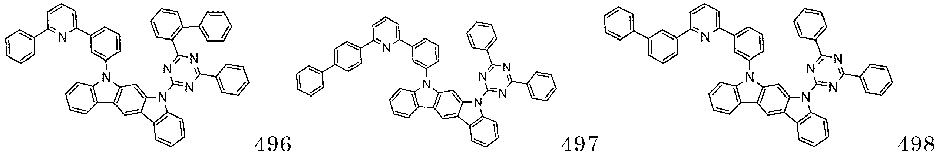

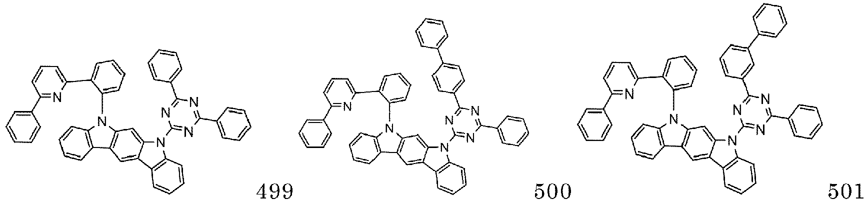

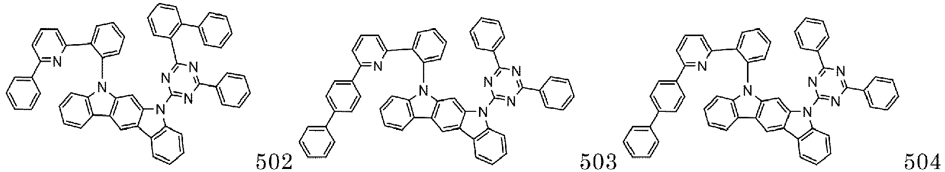

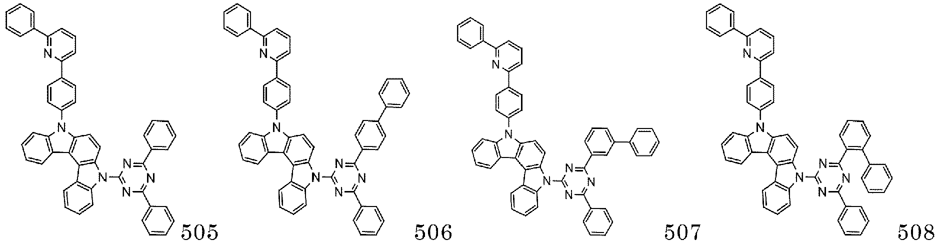

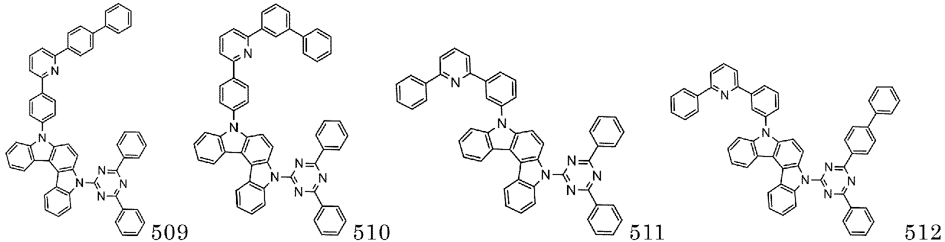

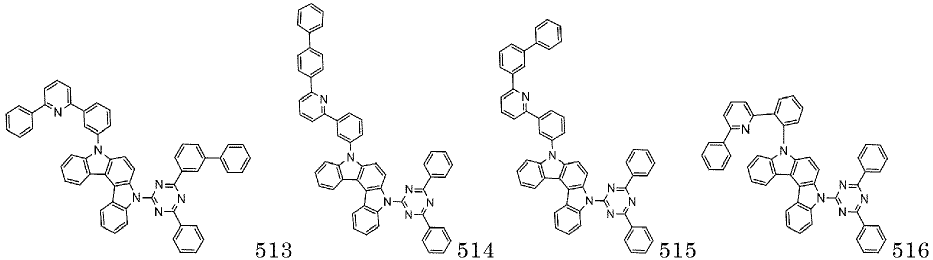

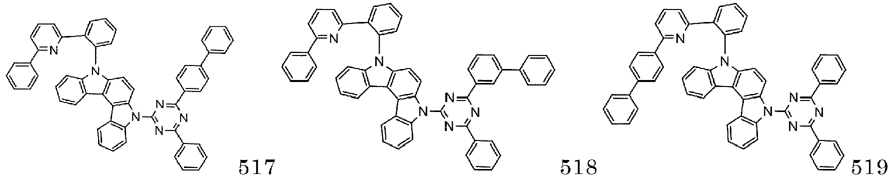

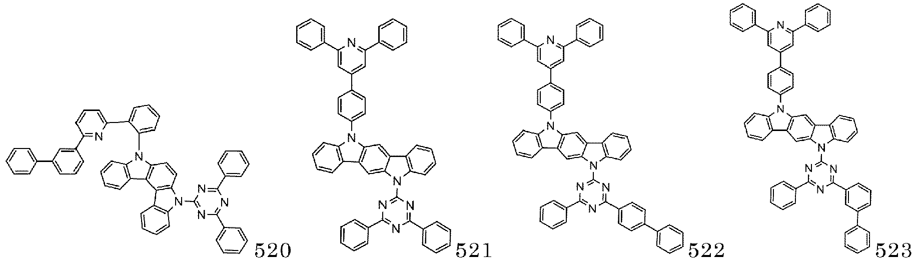

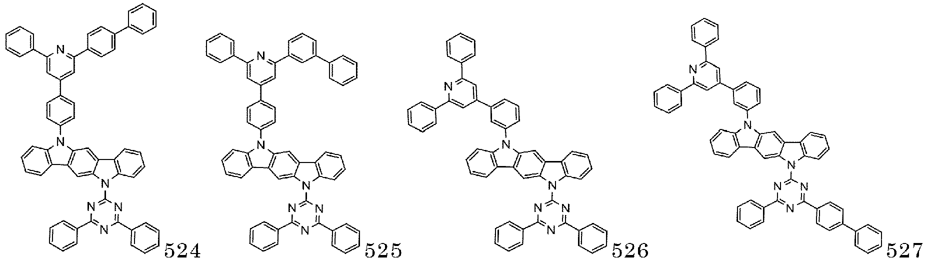

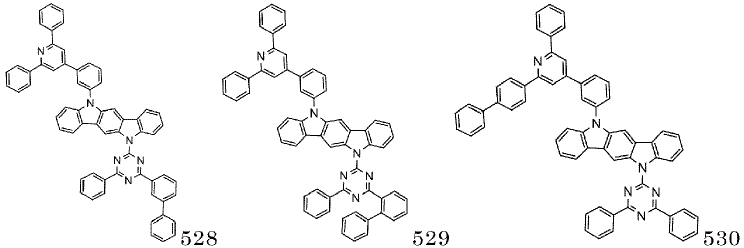

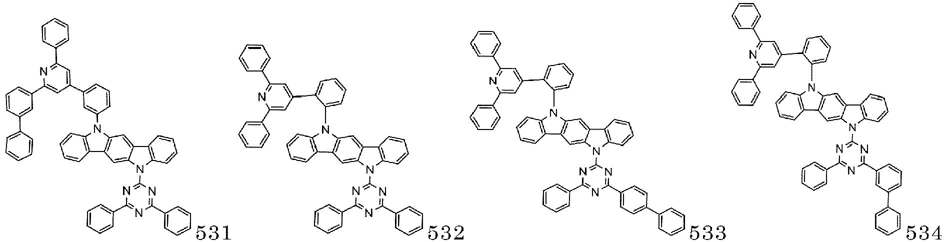

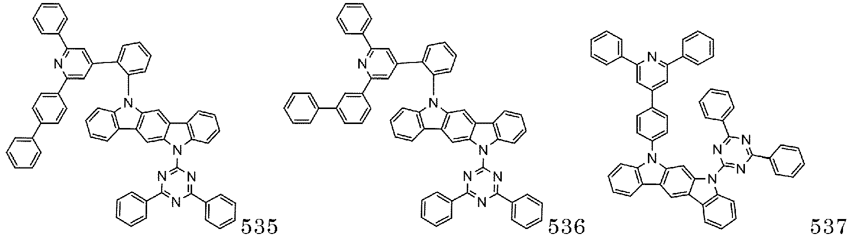

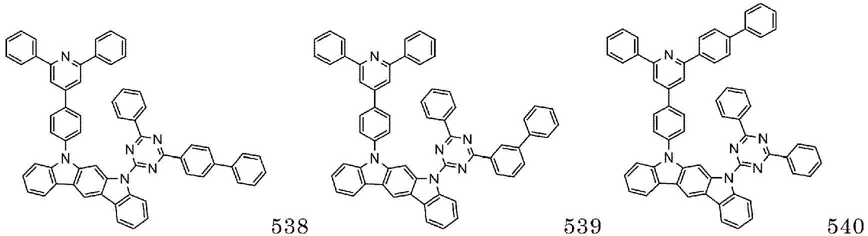

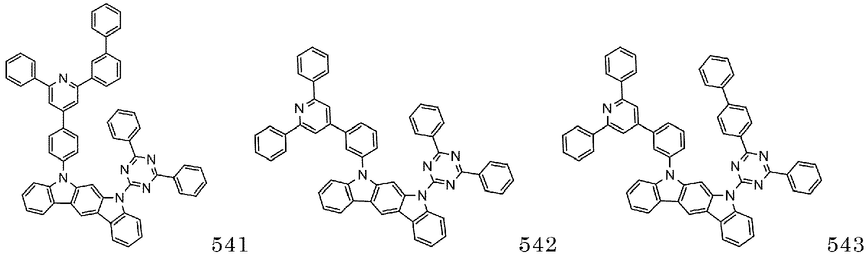

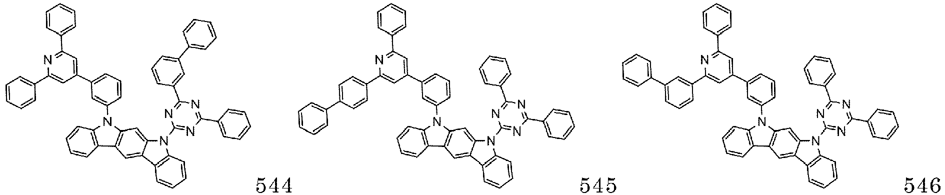

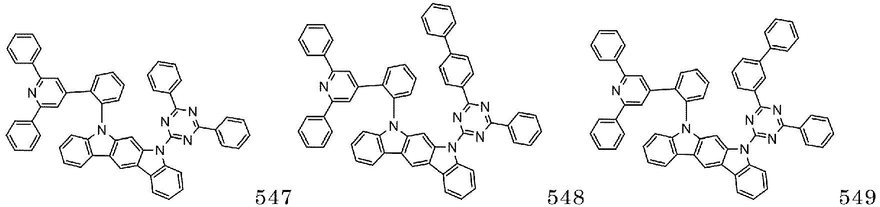

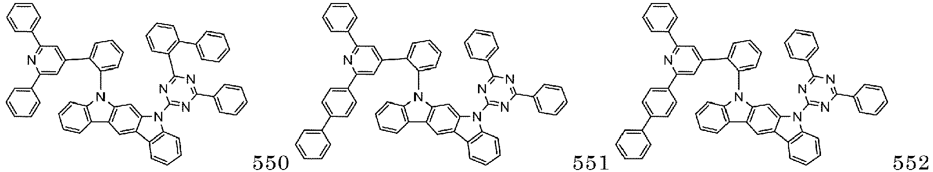

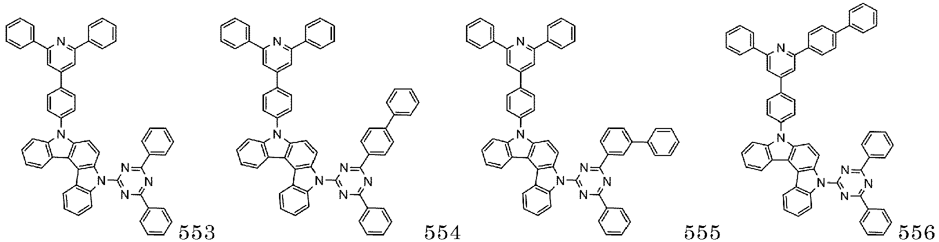

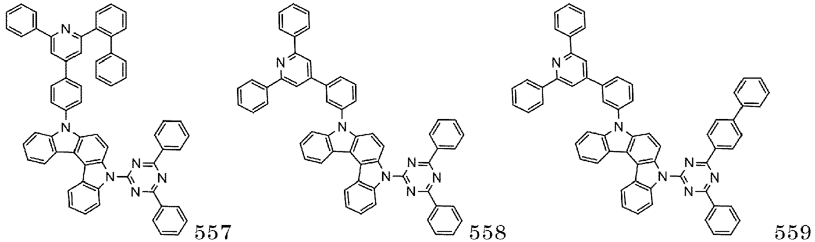

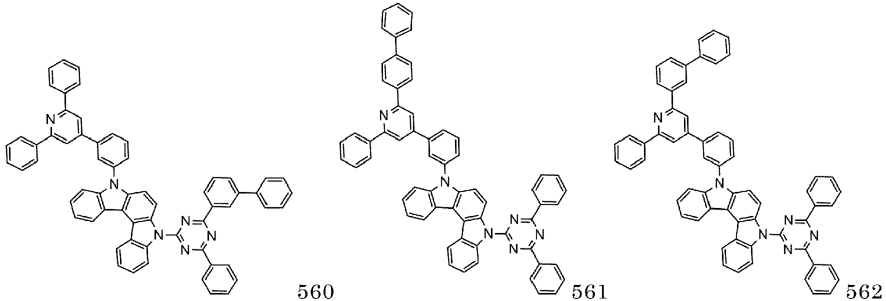

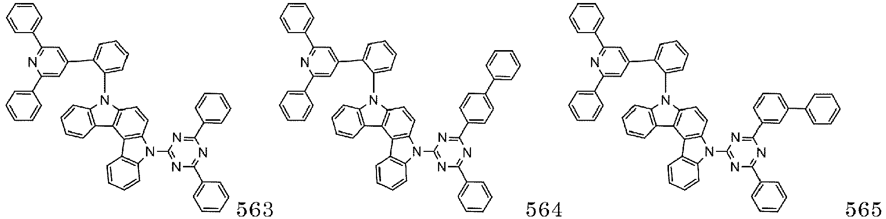

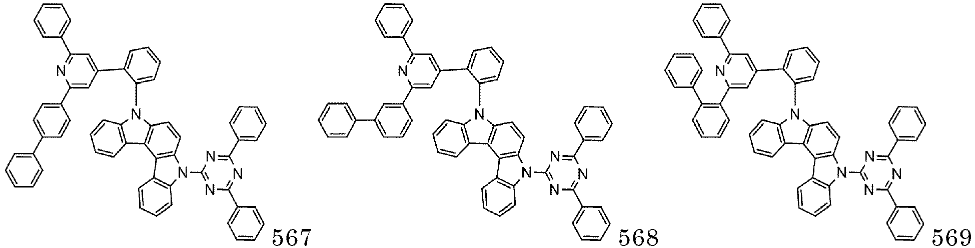

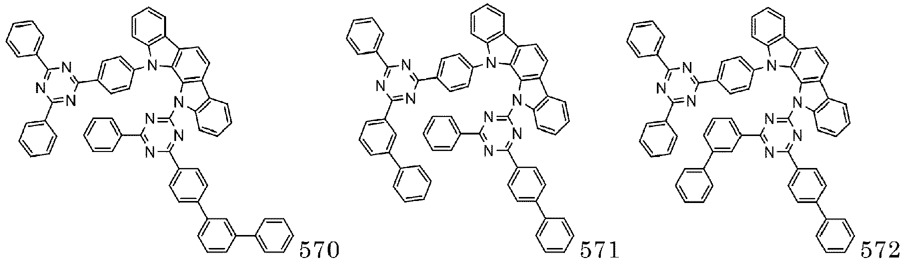

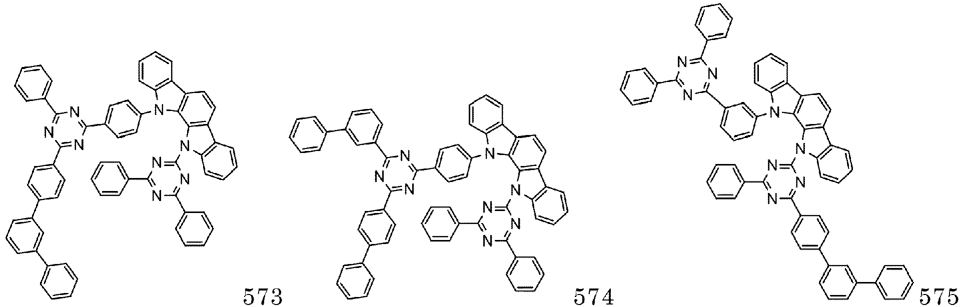

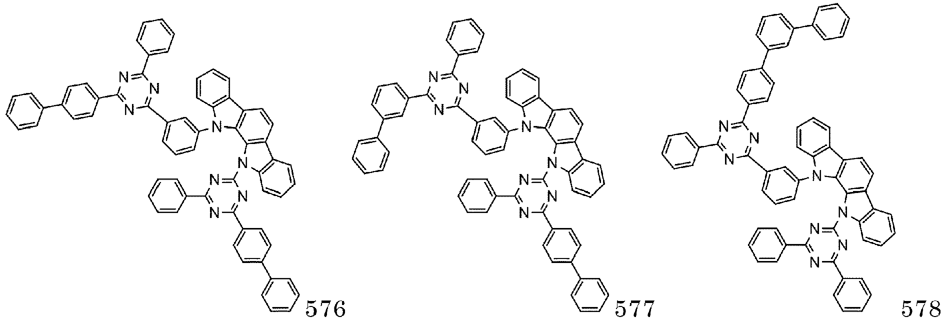

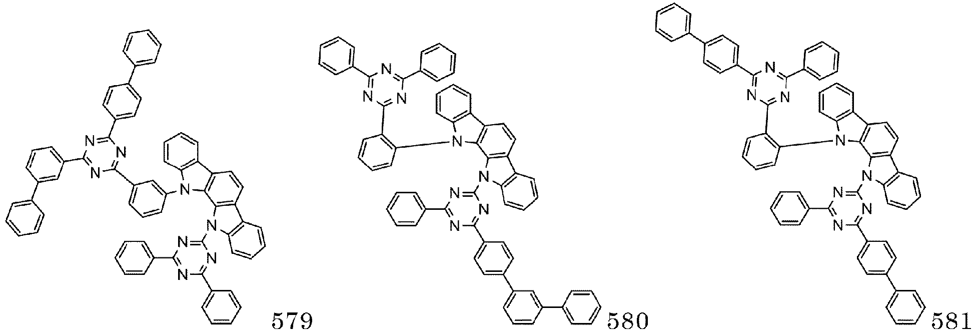

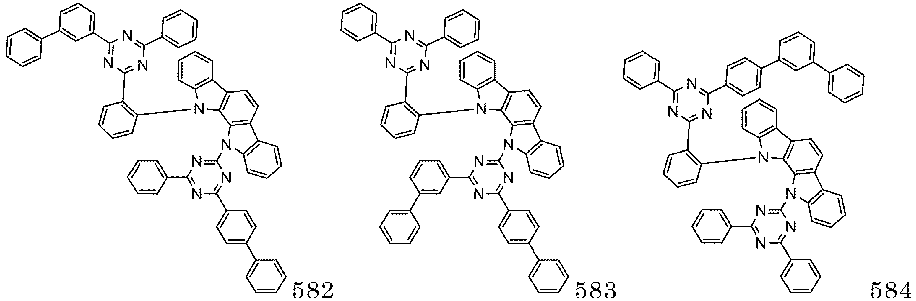

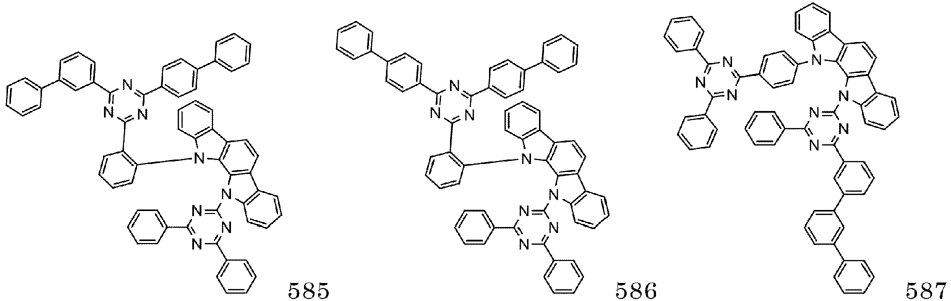

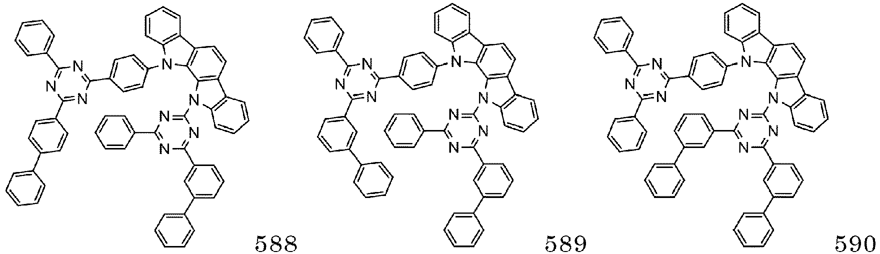

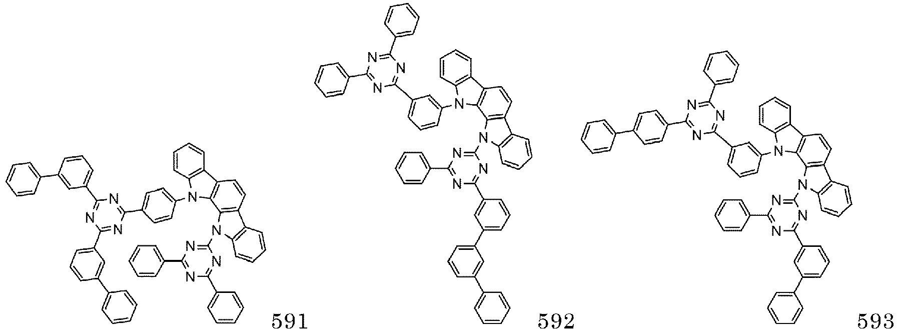

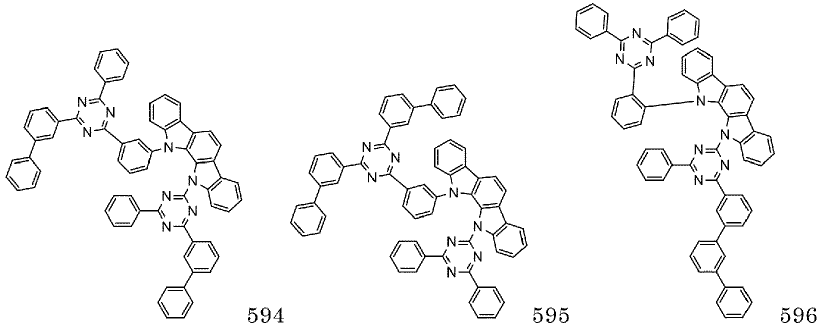

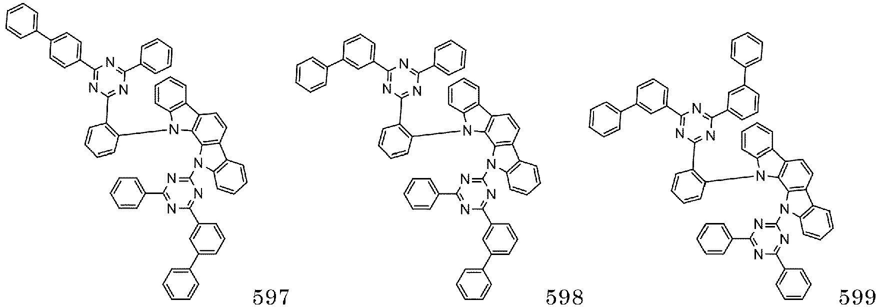

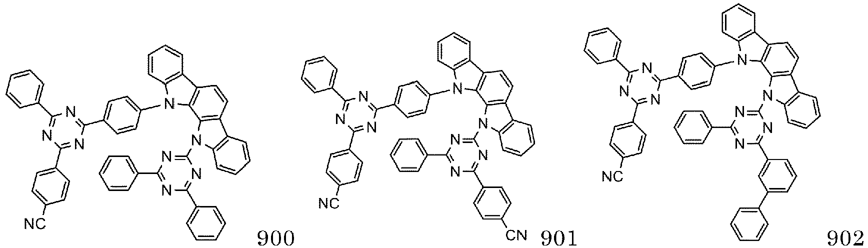

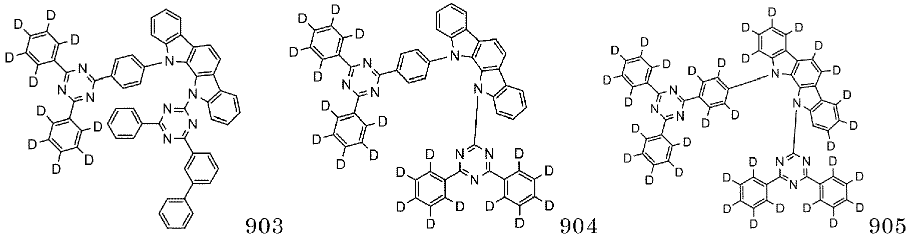

The organic EL device of the present invention is an organic EL device comprising one or more light-emitting layers between an anode and a cathode opposed to each other, wherein at least one of the light-emitting layers contains a first host selected from the compound represented by the general formula (1), a second host represented by the general formula (2), and a light-emitting dopant material.

The compound represented by the general formula (2) is preferably any compound represented by the following formulas (4a) to (4c). More preferred is a compound represented by formula (4a).

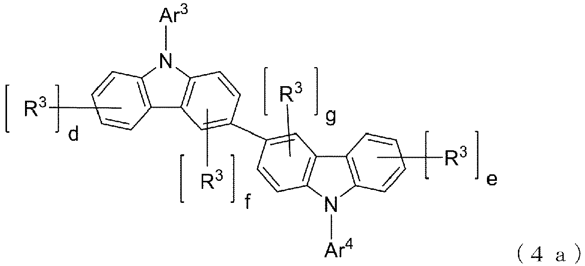

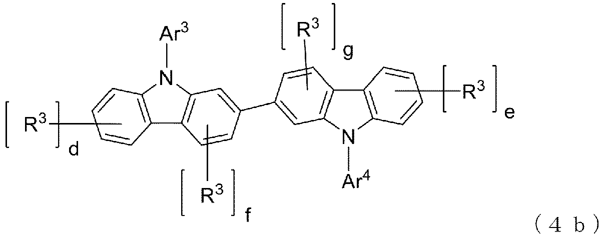

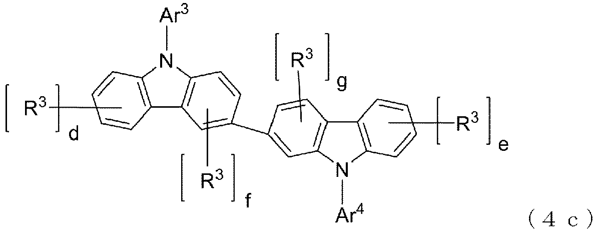

In the general formula (2) and formulas (4a) to (4c), the same symbols have the same meaning.

Ar³ and Ar⁴ independently represent a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group in which two to five of these aromatic groups are linked to each other. Preferred is a substituted or unsubstituted aromatic hydrocarbon group having 6 to 12 carbon atoms, or a substituted or unsubstituted linked aromatic group in which two to four of these aromatic hydrocarbon groups are linked to each other, and more preferred is a substituted or unsubstituted aromatic hydrocarbon group having 6 to 10 carbon atoms, or a substituted or unsubstituted linked aromatic group in which two to three of these aromatic hydrocarbon groups are linked to each other. Further preferred is any of a substituted or unsubstituted phenyl group, m-biphenyl group, or p-biphenyl group. The substituted phenyl group is here a phenyl group substituted with a group other than an aromatic group.

In the case of Ar³ and Ar⁴ being an unsubstituted aromatic hydrocarbon group, aromatic heterocyclic group, or linked aromatic group, specific examples thereof are the same as in the case of Ar¹ and Ar². Preferred examples thereof include a group generated by removing one hydrogen from benzene, naphthalene, or compounds in which two to four of these are linked to each other. More preferred is a substituted or unsubstituted phenyl group, biphenyl group, or terphenyl group. The terphenyl group may be linked linearly or branched.

Each R³ independently represents deuterium, an aliphatic hydrocarbon group having 1 to 10 carbon atoms, a substituted or unsubstituted aromatic hydrocarbon group having 6 to 18 carbon atoms, or a substituted or unsubstituted aromatic heterocyclic group having 3 to 17 carbon atoms.

In the case of R³ being an unsubstituted aliphatic hydrocarbon group, aromatic hydrocarbon group, or aromatic heterocyclic group, specific examples thereof are the same as in the case of R¹ and R². Preferred examples thereof include benzene, naphthalene, acenaphthene, acenaphthylene, azulene, anthracene, chrysene, pyrene, phenanthrene, fluorene, or triphenylene. More preferred is a phenyl group, a biphenyl group, or a terphenyl group. The terphenyl group may be linked linearly or branched.

d and e represent an integer of 0 to 4, and f and g represent an integer of 0 to 3. Preferably, d and e represent an integer of 0 to 2, and f and g represent an integer of 0 to 2. More preferably, all d, e, f, and g represent 0.

Specific examples of the compounds represented by the general formula (2) are shown below, but are not limited to these exemplified compounds.

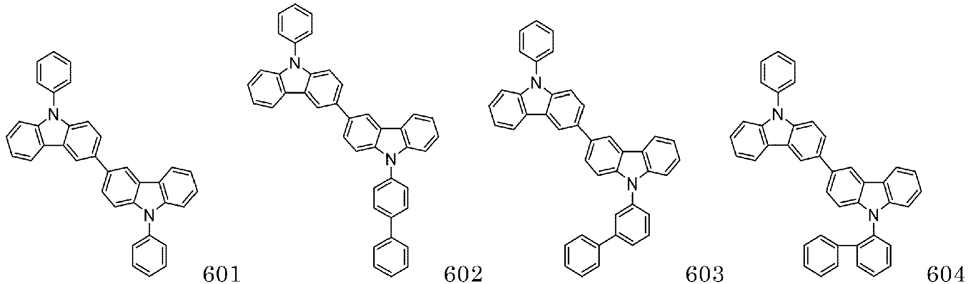

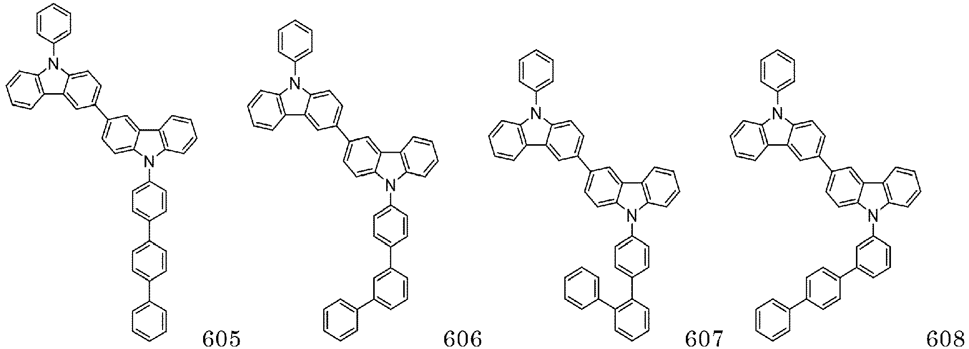

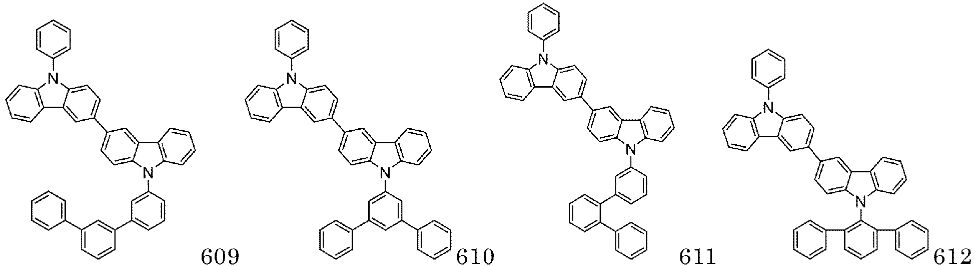

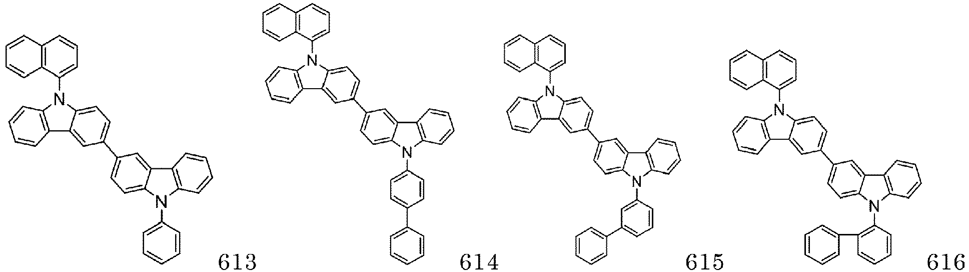

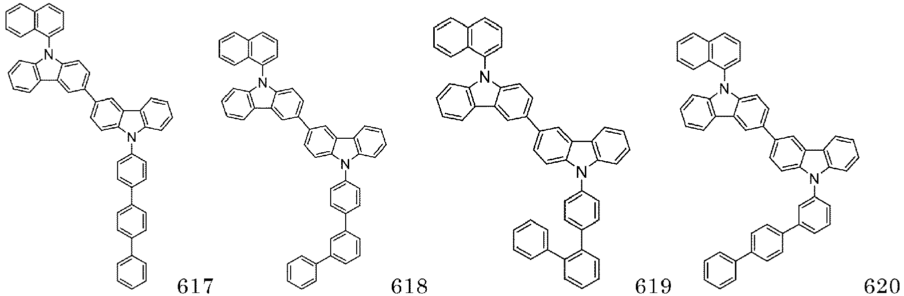

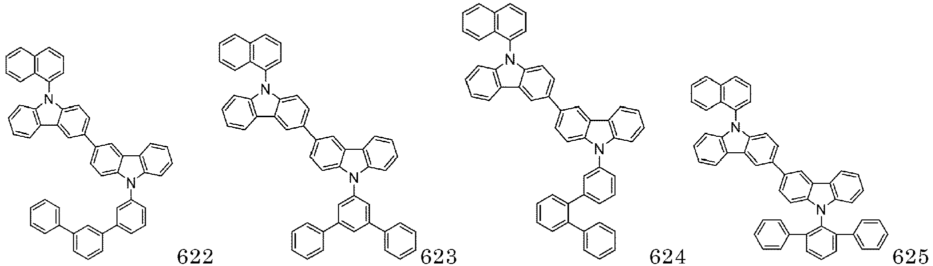

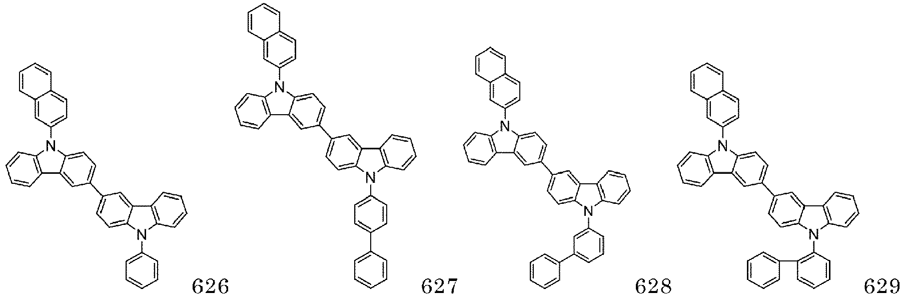

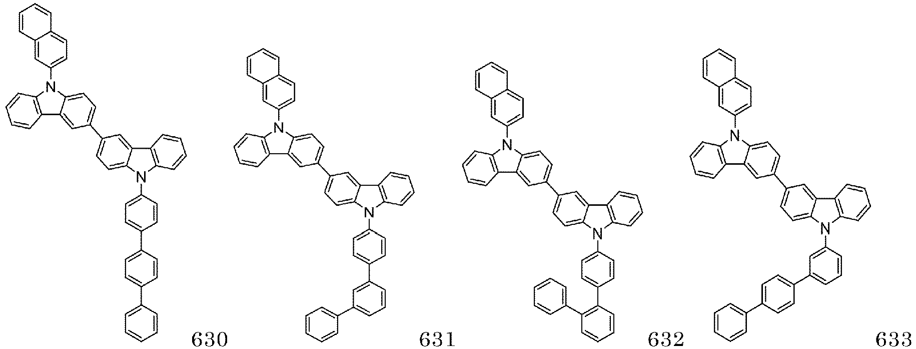

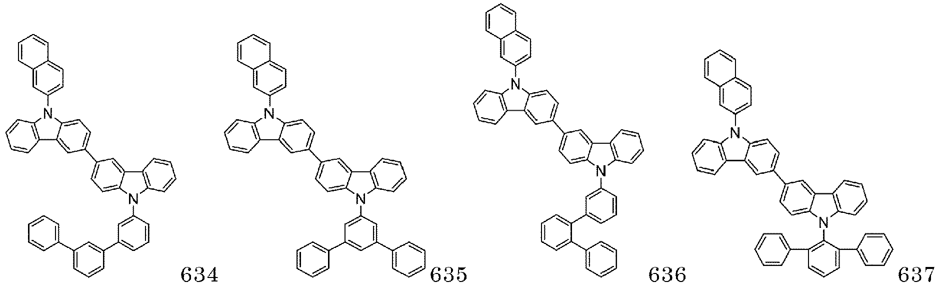

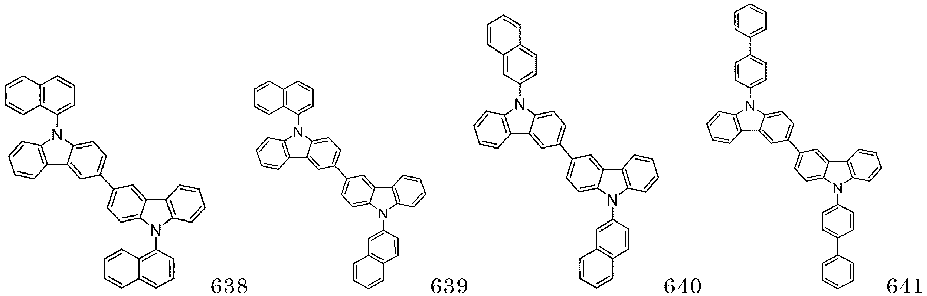

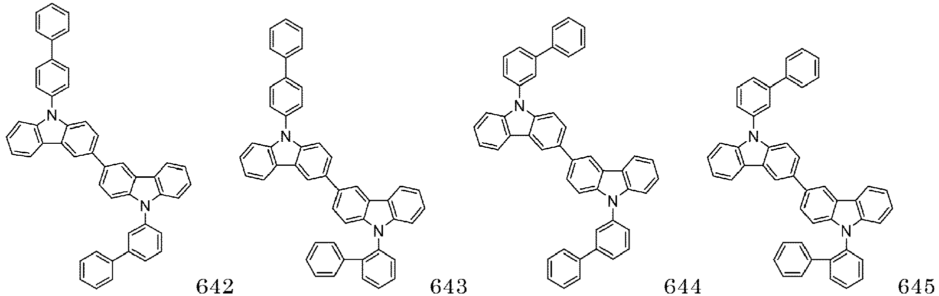

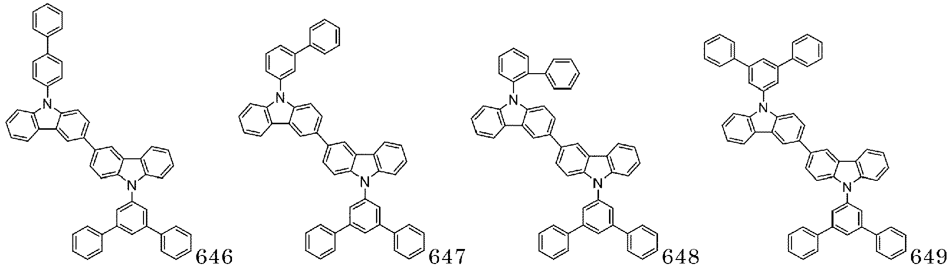

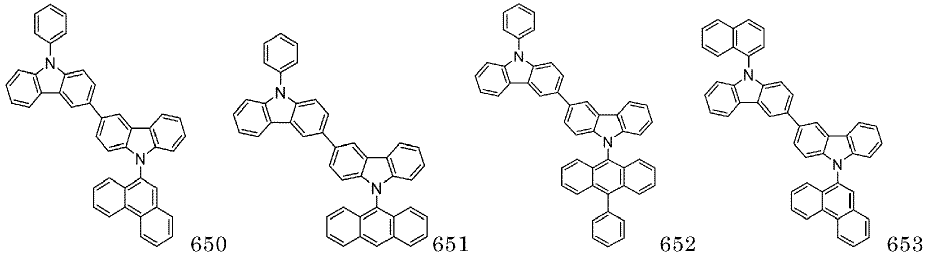

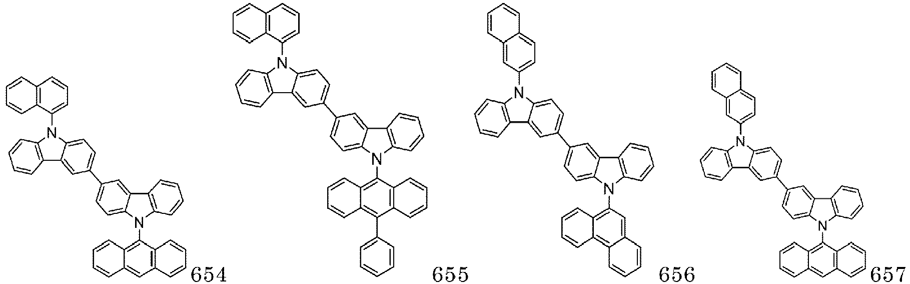

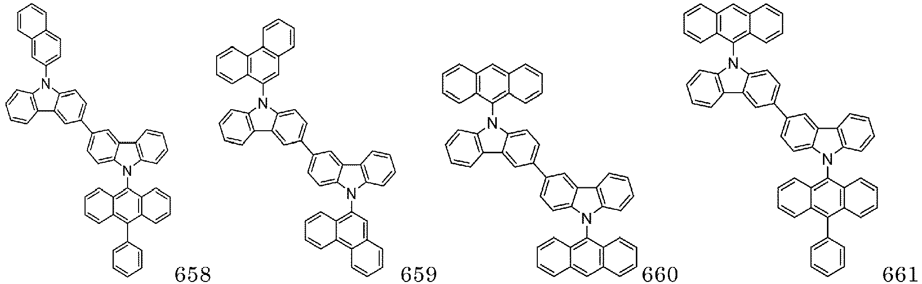

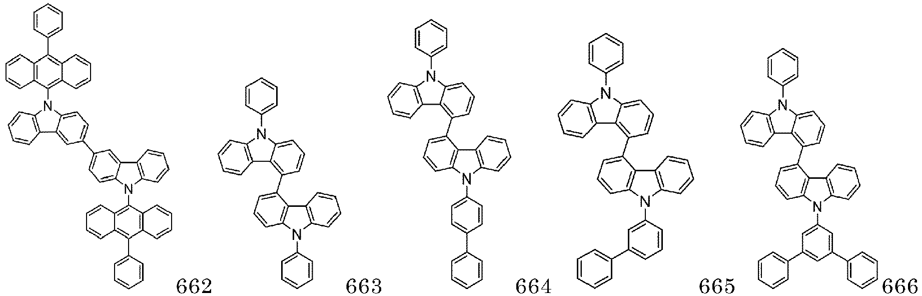

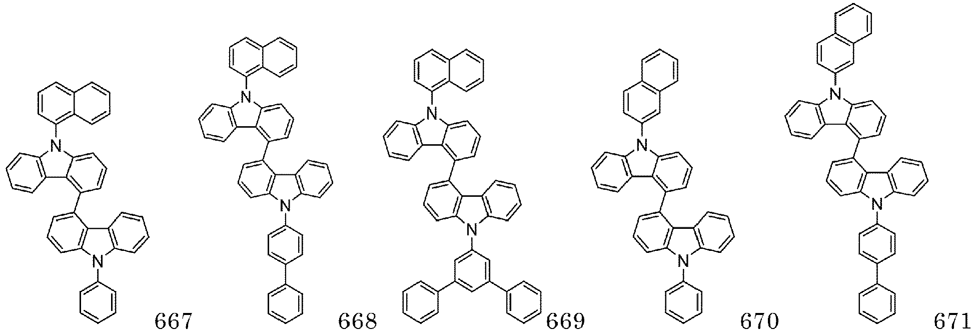

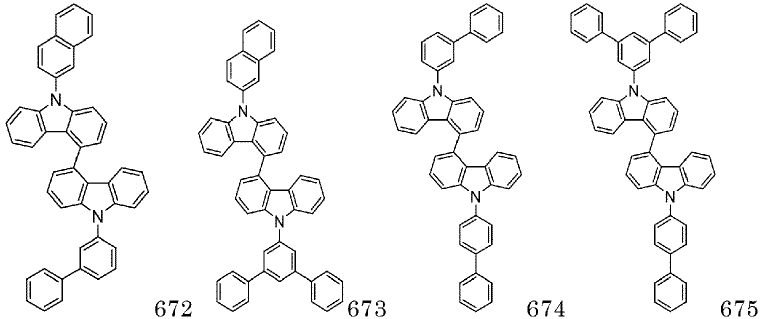

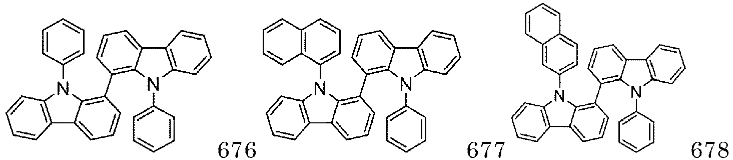

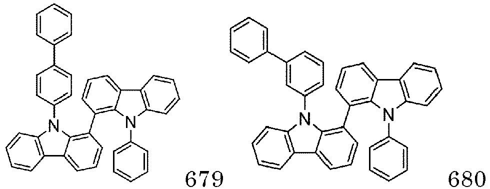

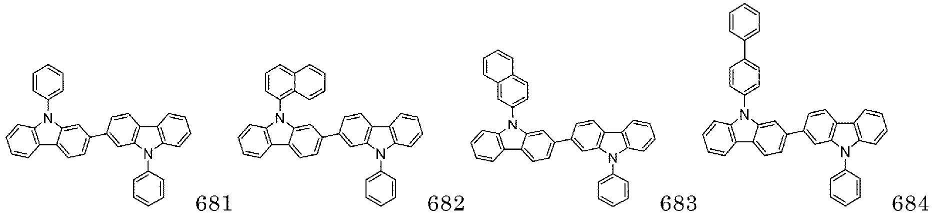

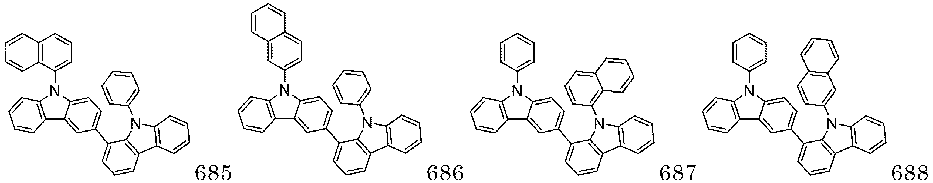

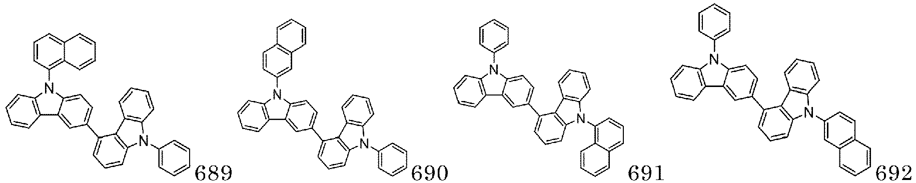

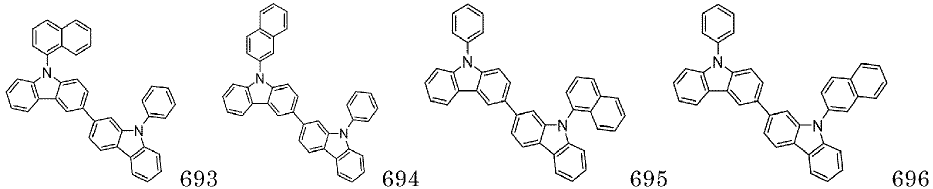

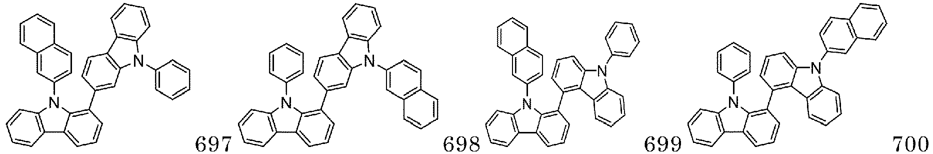

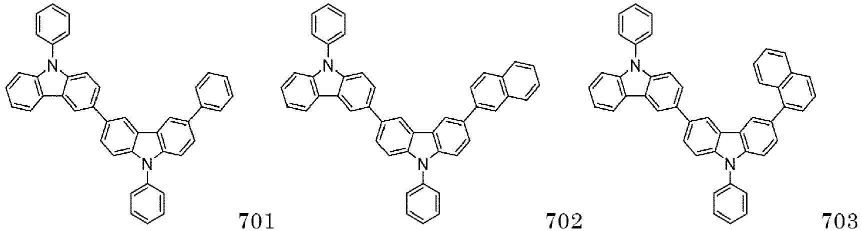

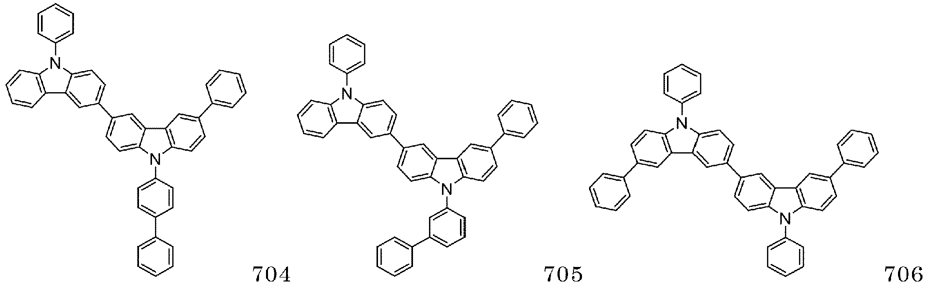

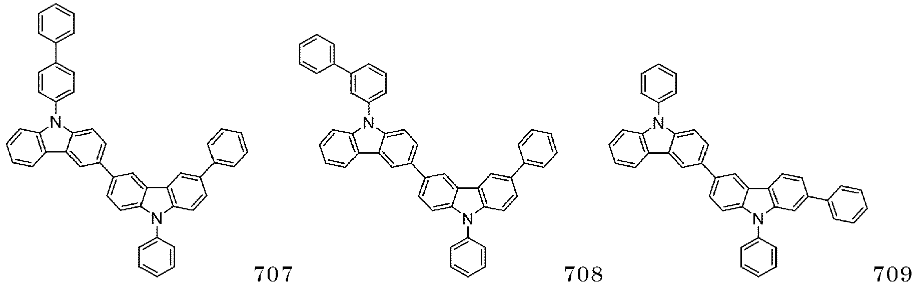

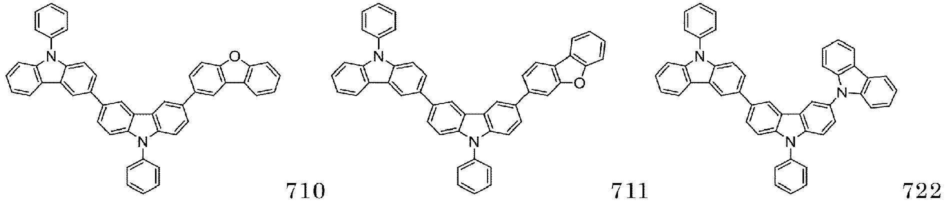

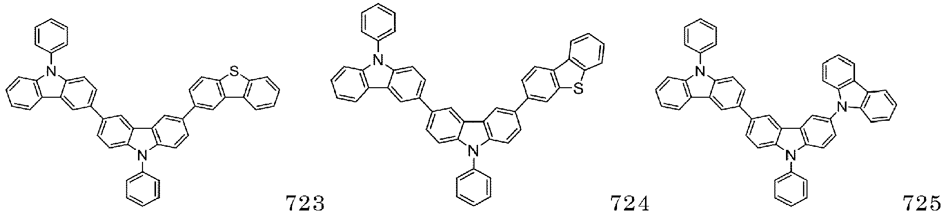

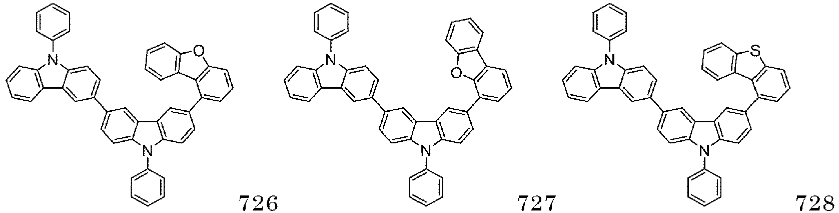

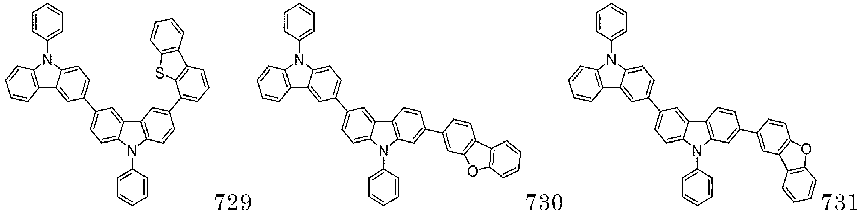

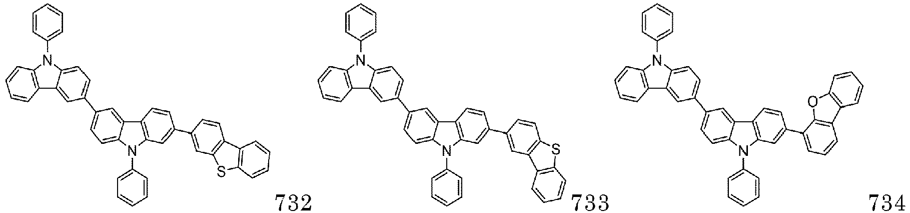

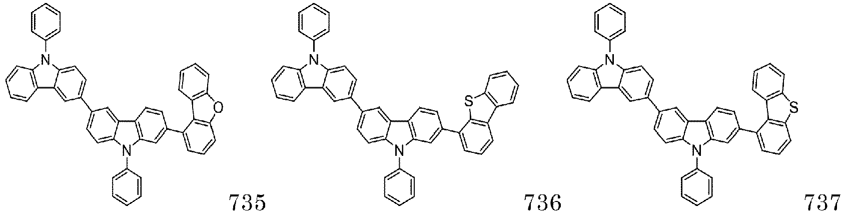

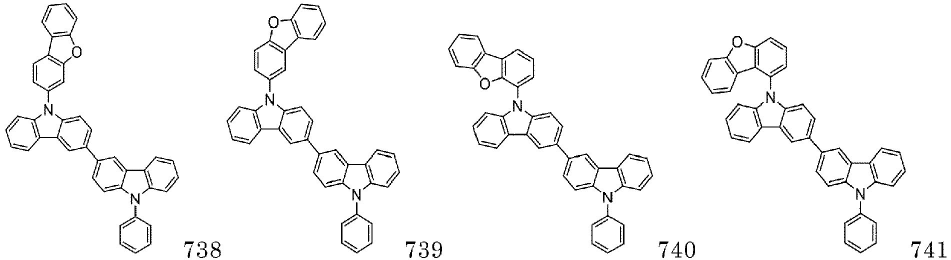

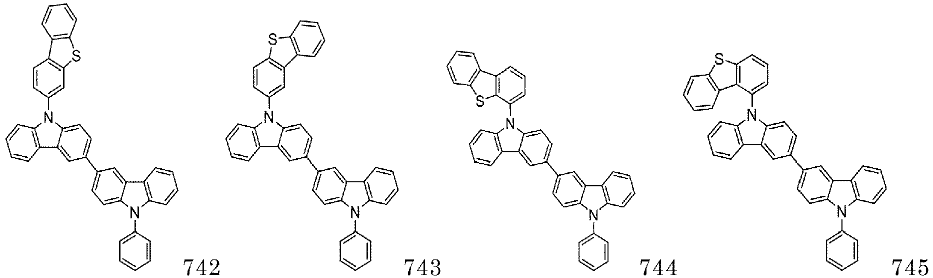

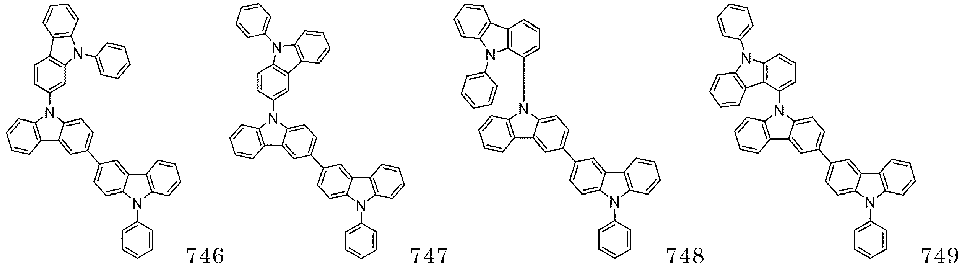

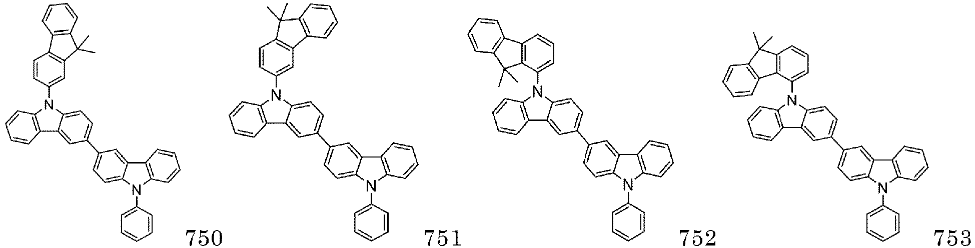

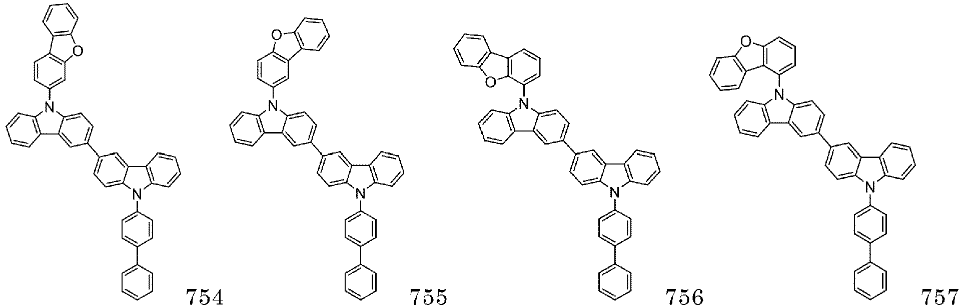

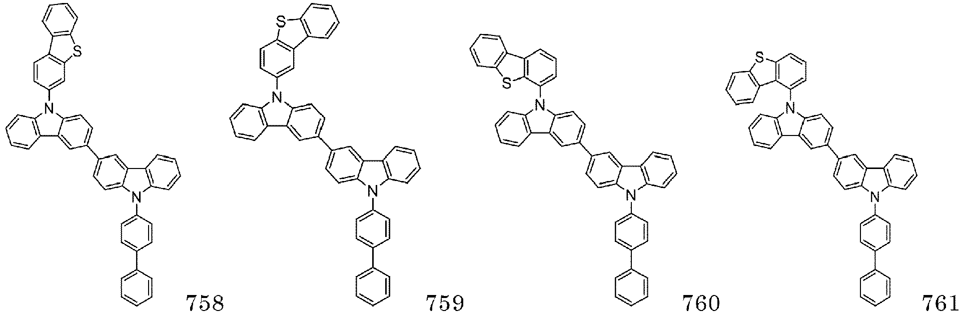

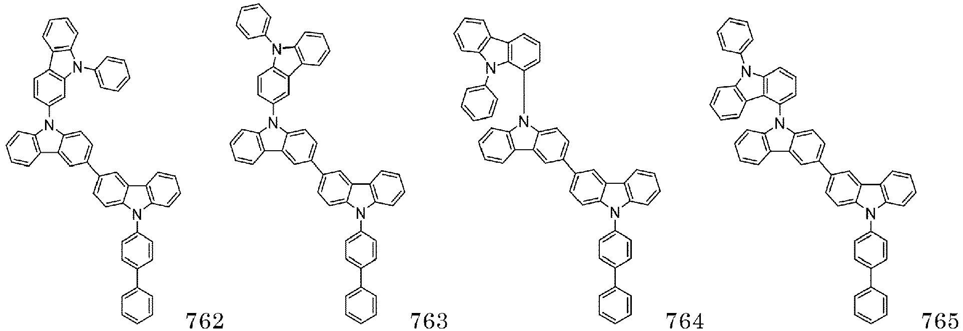

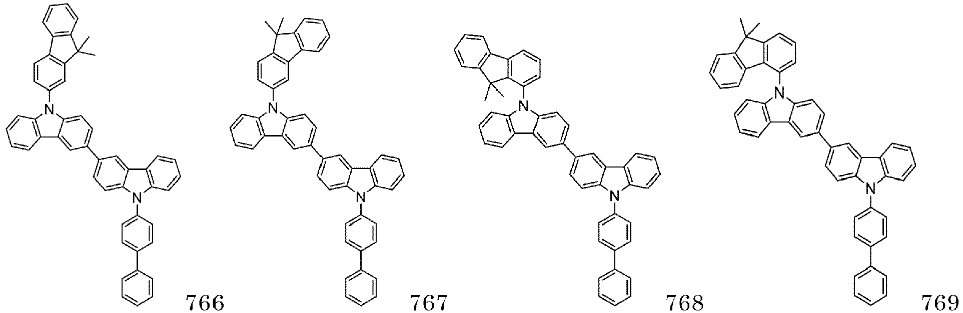

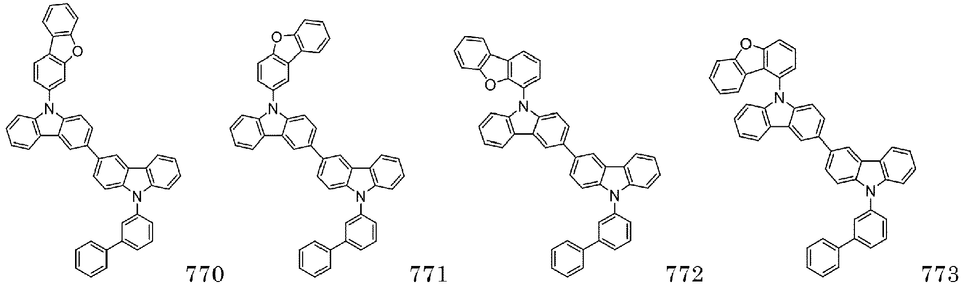

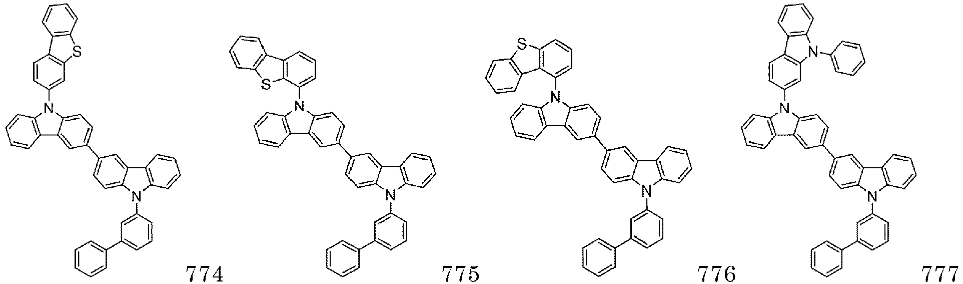

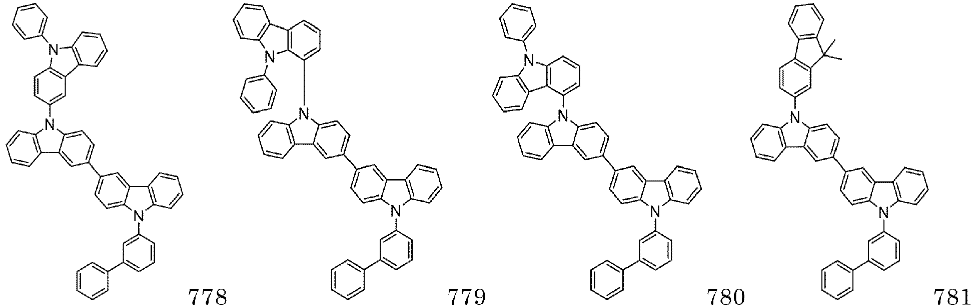

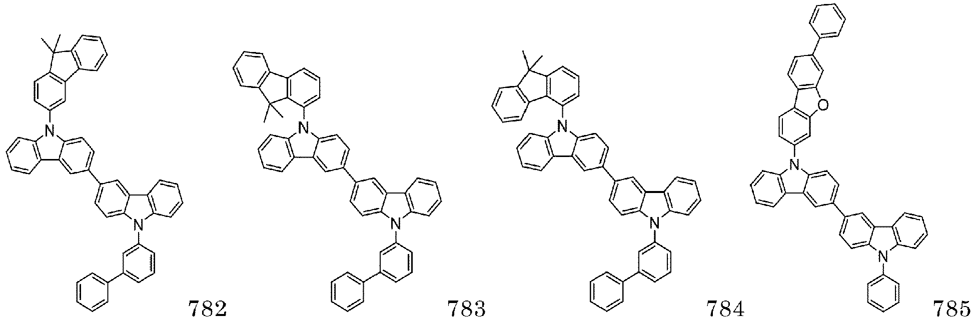

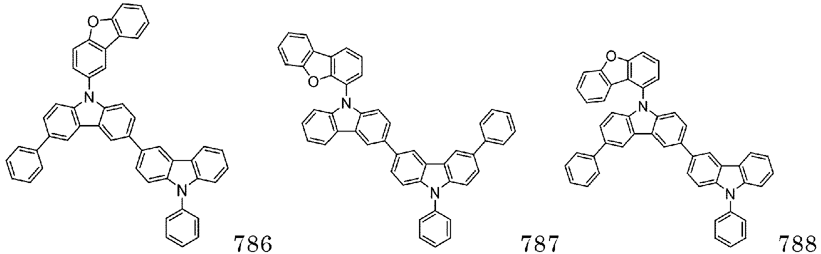

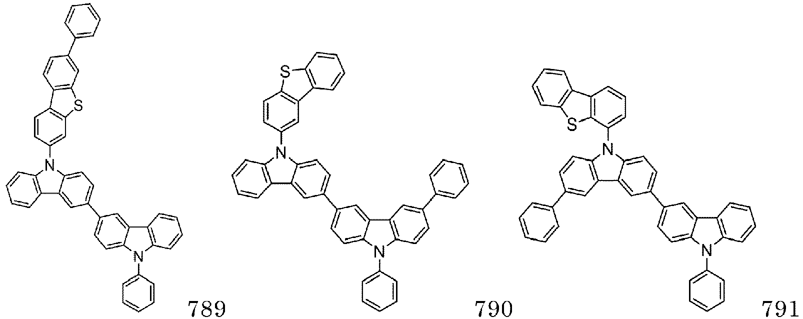

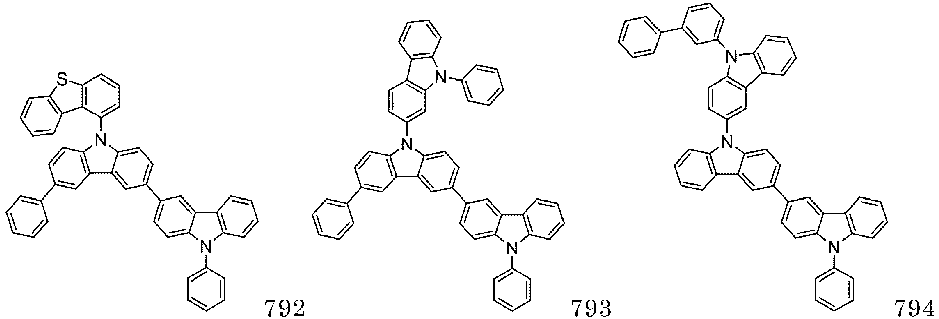

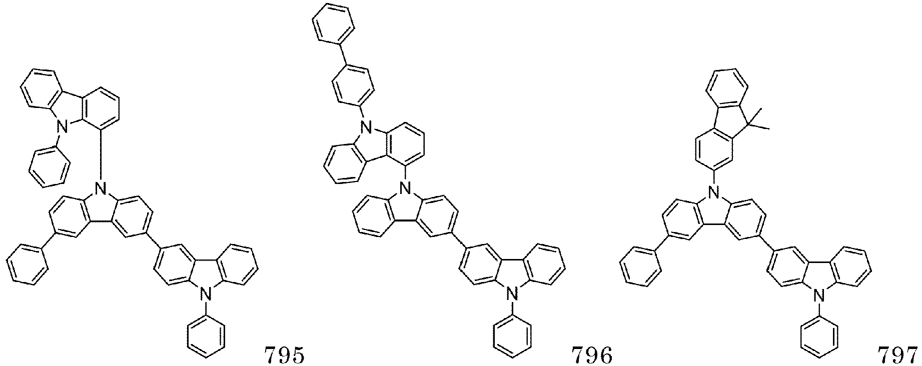

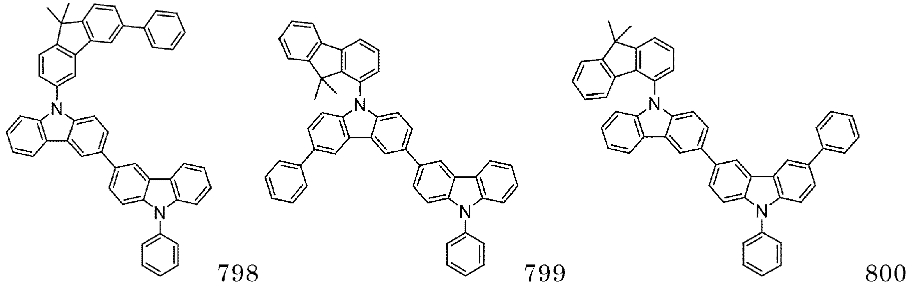

The material for an organic EL device of the present invention is contained in the organic layer, and this organic layer may be selected from the group consisting of a light-emitting layer, a hole injection layer, a hole transport layer, an electron transport layer, an electron injection layer, a hole blocking layer, and an electron blocking layer.

Preferred is a light-emitting layer, and the light-emitting layer may contain at least one light-emitting dopant.

If the material for an organic EL device of the present invention is contained in the light-emitting layer, it is desirable that the material be contained as a host. Advantageously, the material for an organic EL device of the present invention may be contained as the first host, and a compound selected from the compounds represented by the general formula (2) as the second host.

The first host and the second host can be used by vapor deposition from different individual vapor deposition sources, but the light-emitting layer is preferably formed by obtaining a premixture by premixing before vapor deposition to prepare a premixed composition (also referred to as a premixture) and vapor-depositing the premixture simultaneously from one vapor deposition source. In this case, the premixture may be mixed with a light-emitting dopant material necessary for formation of a light-emitting layer, or another host to be used as necessary. However, when there is a large difference in temperatures to provide desired vapor pressure, vapor deposition may be performed from another vapor deposition source.

In addition, regarding the mixing ratio (weight ratio) between the first host and the second host, the proportion of the first host may be 10 to 70%, and is preferably 15% to 65%, and more preferably 20 to 60% based on the first host and the second host in total.

Next, the structure of the organic EL device of the present invention will be described by referring to the drawing, but the structure of the organic EL device of the present invention is not limited thereto.

Figure 1 is a cross-sectional view showing a structure example of an organic EL device generally used for the present invention, in which there are indicated a substrate 1, an anode 2, a hole injection layer 3, a hole transport layer 4, a light-emitting layer 5, an electron transport layer 6, and a cathode 7. The organic EL device of the present invention may have an exciton blocking layer adjacent to the light-emitting layer and may have an electron blocking layer between the light-emitting layer and the hole injection layer. The exciton blocking layer can be inserted into either of the cathode side and the anode side of the light-emitting layer and inserted into both sides at the same time. The organic EL device of the present invention has the anode, the light-emitting layer, and the cathode as essential layers, and preferably has a hole injection transport layer and an electron injection transport layer in addition to the essential layers, and further preferably has a hole blocking layer between the light-emitting layer and the electron injection transport layer. Note that the hole injection transport layer refers to either or both of a hole injection layer and a hole transport layer, and the electron injection transport layer refers to either or both of an electron injection layer and an electron transport layer.

A structure reverse to that of Figure 1 is applicable, in which a cathode 7, an electron transport layer 6, a light-emitting layer 5, a hole transport layer 4, and an anode 2 are laminated on a substrate 1 in this order. In this case, layers may be added or omitted as necessary.

### - Substrate -

The organic EL device of the present invention is preferably supported on a substrate. The substrate is not particularly limited, and those conventionally used in organic EL devices may be used, and substrates made of, for example, glass, a transparent plastic, or quartz may be used.

### - Anode -

Regarding an anode material for an organic EL device, it is preferable to use a material of a metal, an alloy, an electrically conductive compound, and a mixture thereof, each having a large work function (4 eV or more). Specific examples of such an electrode material include a metal such as Au, and a conductive transparent material such as CuI, indium tin oxide (ITO), SnO₂, and ZnO. In addition, an amorphous material such as IDIXO (In₂O₃-ZnO), which is capable of forming a transparent conductive film, may be used. Regarding the anode, such an electrode material is used to form a thin film by, for example, a vapor-deposition or sputtering method, and a desired shape pattern may be formed by a photolithographic method; or if the pattern accuracy is not particularly required (about 100 µm or more), a pattern may be formed via a desired shape mask when the electrode material is vapor-deposited or sputtered. Alternatively, when a coatable substance such as an organic conductive compound is used, a wet film formation method such as a printing method or a coating method may be used. For taking emitted light from the anode, it is desired to have a transmittance of more than 10%, and the sheet resistance for the anode is preferably several hundreds **Ω**/□ or less. The film thickness is selected usually within 10 to 1000 nm, preferably within 10 to 200 nm though depending on the material.

### - Cathode -

Meanwhile, regarding a cathode material, a material of a metal (an electron injection metal), an alloy, an electrically conductive compound, or a mixture thereof, each having a small work function (4 eV or less) is used. Specific examples of such an electrode material include sodium, a sodium-potassium alloy, magnesium, lithium, a magnesium/copper mixture, a magnesium/silver mixture, a magnesium/aluminum mixture, a magnesium/indium mixture, an aluminum/aluminum oxide (Al₂O₃) mixture, indium, a lithium/aluminum mixture, and a rare earth metal. Among these, from the viewpoint of the electron injectability and the durability against oxidation and the like, a mixture of an electron injection metal and a second metal which is a stable metal having a larger work function value is suitable, and examples thereof include a magnesium/silver mixture, a magnesium/aluminum mixture, a magnesium/indium mixture, an aluminum/aluminum oxide mixture, a lithium/aluminum mixture and aluminum. The cathode can be produced by forming a thin film by a method such as vapor-depositing or sputtering of such a cathode material. In addition, the sheet resistance of cathode is preferably several hundreds **Ω**/□ or less. The film thickness is selected usually within 10 nm to 5 µm, preferably within 50 to 200 nm. Note that for transmission of emitted light, if either one of the anode and cathode of the organic EL device is transparent or translucent, emission luminance is improved, which is convenient.

In addition, formation of a film of the above metal with a thickness of 1 to 20 nm, followed by formation of a conductive transparent material described in the description on the anode thereon, enables production of a transparent or translucent cathode, and application of this enables production of a device wherein an anode and a cathode both have transmittance.

### - Light-emitting layer -

The light-emitting layer is a layer that emits light after excitons are generated when holes and electrons injected from the anode and the cathode, respectively, are recombined. As a light-emitting layer, an organic light-emitting dopant material and a host may be contained.

As a host, the first host and the second host may be used.

Regarding the compound represented by the general formula (1) as the first host, one kind thereof may be used, or two or more kinds of different compounds may be used. Similarly, regarding the carbazole compound represented by the general formula (2) as the second host, one kind thereof may be used, or two or more kinds of different compounds may be used.

If necessary, one, or two or more other known host materials may be used in combination; however, it is preferable that an amount thereof to be used be 50 wt% or less, preferably 25 wt% or less based on the host materials in total.

The host and the premixture thereof may be in powder, stick, or granule form.

In the case of use of a plurality of kinds of hosts, such respective hosts can be vapor-deposited from different vapor deposition sources or can be simultaneously vapor-deposited from one vapor deposition source by premixing the hosts before vapor deposition to provide a premixture.

The premixing method is desirably a method that can allow for mixing as uniformly as possible, and examples thereof include pulverization and mixing, a heating and melting method under reduced pressure or under an atmosphere of an inert gas such as nitrogen, and sublimation, but not limited thereto.

When the first host and the second host are premixed and used, it is desirable that a difference in 50% weight reduction temperature (T₅₀) be small in order to produce an organic EL device having favorable characteristics with high reproducibility. The 50% weight reduction temperature is a temperature at which the weight is reduced by 50% when the temperature is raised to 550°C from room temperature at a rate of 10°C/min in TG-DTA measurement under a nitrogen stream reduced pressure (1 Pa). It is considered that vaporization due to evaporation or sublimation the most vigorously occurs around this temperature.

The difference in 50% weight reduction temperatures of the first host and the second host is preferably within 20°C, more preferably within 15°C. Regarding a premixing method, a known method such as pulverization and mixing can be used, and it is desirable to mix them as uniformly as possible.

When a phosphorescent dopant is used as a light-emitting dopant material, preferred is a phosphorescent dopant including an organic metal complex containing at least one metal selected from ruthenium, rhodium, palladium, silver, rhenium, osmium, iridium, platinum and gold. Specifically, iridium complexes described in J. Am. Chem. Soc. 2001, 123, 4304 and Japanese Translation of PCT International Application Publication No. 2013-530515 are preferably used, but the phosphorescent dopant is not limited thereto.

Regarding the phosphorescent dopant material, only one kind thereof may be contained in the light-emitting layer, or two or more kinds thereof may be contained. A content of the phosphorescent dopant material is preferably 0.1 to 30 wt% and more preferably 1 to 20 wt% with respect to the host material.

The phosphorescent dopant material is not particularly limited, and specific examples thereof include the following.

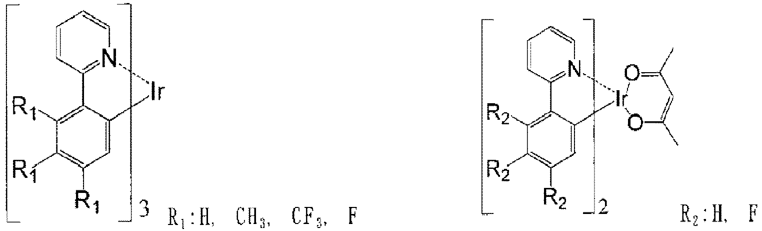

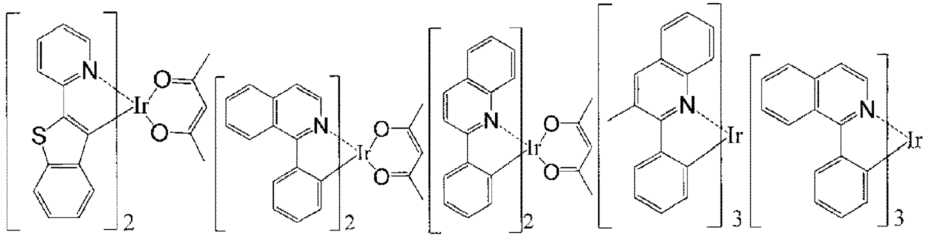

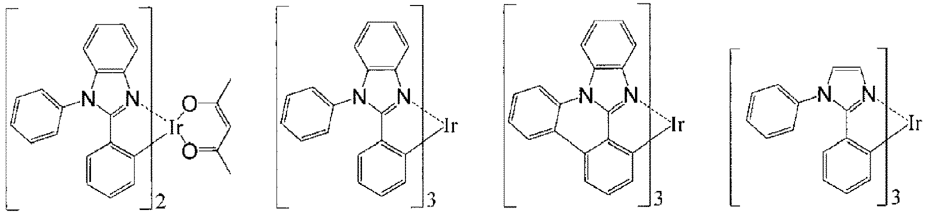

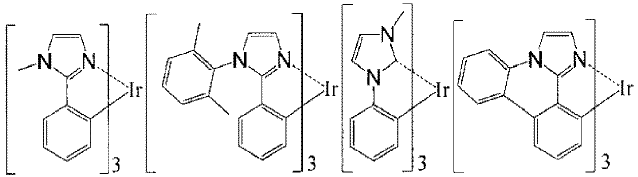

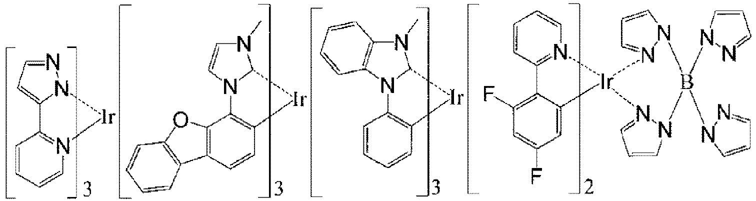

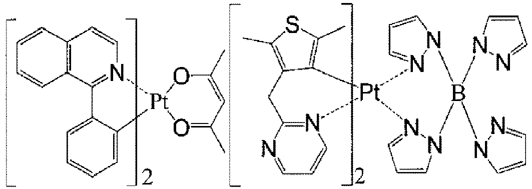

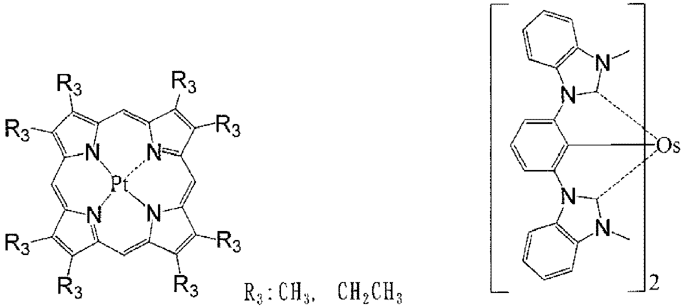

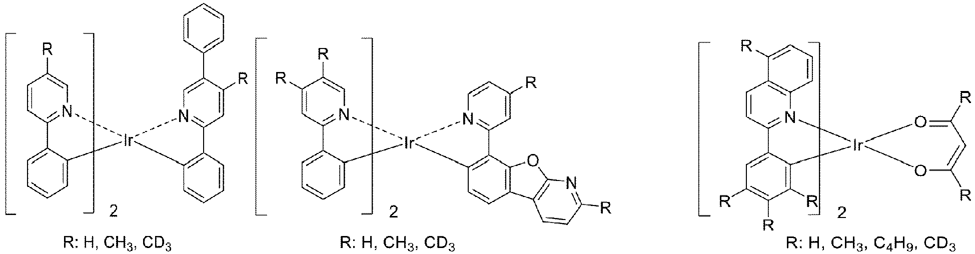

When a fluorescence-emitting dopant is used as the light-emitting dopant material, the fluorescence-emitting dopant is not particularly limited. Examples thereof include benzoxazole derivatives, benzothiazole derivatives, benzimidazole derivatives, styrylbenzene derivatives, polyphenyl derivatives, diphenylbutadiene derivatives, tetraphenyl butadiene derivatives, naphthalimido derivatives, coumarin derivatives, fused aromatic compounds, perinone derivatives, oxadiazole derivatives, oxazine derivatives, aldazine derivatives, pyrrolidine derivatives, cyclopentadiene derivatives, bisstyryl anthracene derivatives, quinacridone derivatives, pyrrolopyridine derivatives, thiadiazolopyridine derivatives, styrylamine derivatives, diketopyrrolopyrrole derivatives, aromatic dimethylidine compounds, metal complexes of 8-quinolinol derivatives or metal complexes of pyromethene derivatives, rare earth complexes, various metal complexes represented by transition metal complexes, polymer compounds such as polythiophene, polyphenylene, and polyphenylene vinylene, and organosilane derivatives. Preferred examples thereof include fused aromatic derivatives, styryl derivatives, diketopyrrolopyrrole derivatives, oxazine derivatives, pyromethene metal complexes, transition metal complexes, and lanthanoid complexes. More preferable examples thereof include naphthalene, pyrene, chrysene, triphenylene, benzo[c]phenanthrene, benzo[a]anthracene, pentacene, perylene, fluoranthene, acenaphthofluoranthene, dibenzo[a,j]anthracene, dibenzo[a,h]anthracene, benzo[a]naphthalene, hexacene, naphtho[2,1-f]isoquinoline, α-naphthaphenanthridine, phenanthrooxazole, quinolino[6,5-f]quinoline, and benzothiophanthrene. These may have an alkyl group, an aryl group, an aromatic heterocyclic group, or a diarylamino group as a substituent.

Regarding the fluorescence-emitting dopant material, only one kind thereof may be contained in the light-emitting layer, or two or more kinds thereof may be contained. A content of the fluorescence-emitting dopant material is preferably 0.1% to 20% and more preferably 1% to 10% with respect to the host material.

When a thermally activated delayed fluorescence-emitting dopant is used as the light-emitting dopant material, the thermally activated delayed fluorescence-emitting dopant is not particularly limited. Examples thereof include: metal complexes such as a tin complex and a copper complex; indolocarbazole derivatives described in WO2011/070963; cyanobenzene derivatives and carbazole derivatives described in Nature 2012, 492, 234; and phenazine derivatives, oxadiazole derivatives, triazole derivatives, sulfone derivatives, phenoxazine derivatives, and acridine derivatives described in Nature Photonics 2014, 8,326.

The thermally activated delayed fluorescence-emitting dopant material is not particularly limited, and specific examples thereof include the following.

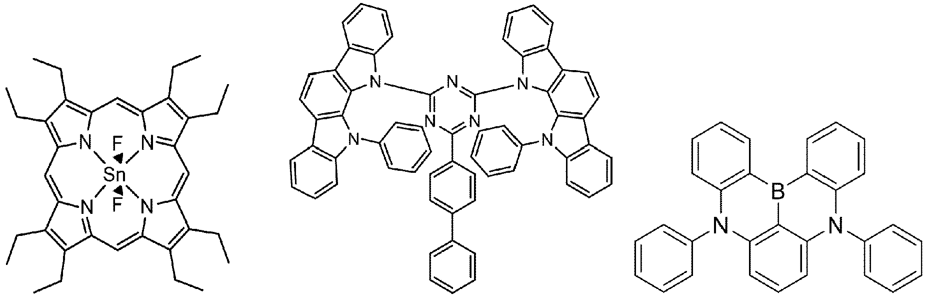

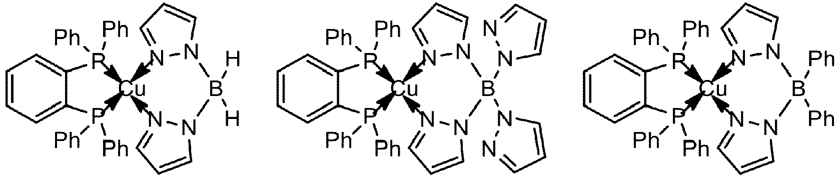

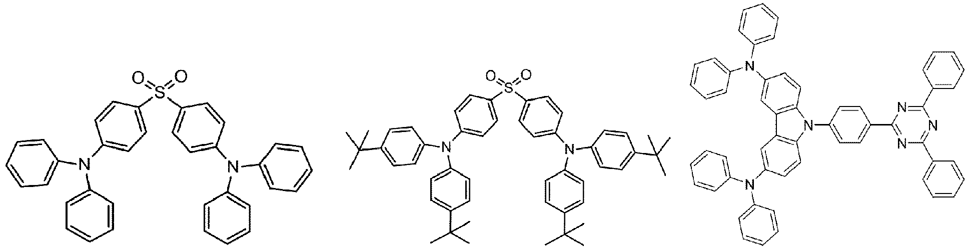

Regarding the thermally activated delayed fluorescence-emitting dopant material, only one kind thereof may be contained in the light-emitting layer, or two or more kinds thereof may be contained. In addition, the thermally activated delayed fluorescence-emitting dopant may be used by mixing with a phosphorescent dopant and a fluorescence-emitting dopant. A content of the thermally activated delayed fluorescence-emitting dopant material is preferably 0.1% to 50% and more preferably 1% to 30% with respect to the host material.

### - Injection Layer -

The injection layer is a layer that is provided between an electrode and an organic layer in order to lower a driving voltage and improve emission luminance, and includes a hole injection layer and an electron injection layer, and may be present between the anode and the light-emitting layer or the hole transport layer, and between the cathode and the light-emitting layer or the electron transport layer. The injection layer can be provided as necessary.

### - Hole Blocking Layer -

The hole blocking layer has a function of the electron transport layer in a broad sense, and is made of a hole blocking material having a function of transporting electrons and a significantly low ability to transport holes, and can block holes while transporting electrons, thereby improving a probability of recombining electrons and holes in the light-emitting layer.

### - Electron Blocking Layer -

The electron blocking layer has a function of a hole transport layer in a broad sense and blocks electrons while transporting holes, thereby enabling a probability of recombining electrons and holes in the light-emitting layer to be improved.

Regarding the material of the electron blocking layer, a known electron blocking layer material can be used and a material of the hole transport layer to be described below can be used as necessary. A film thickness of the electron blocking layer is preferably 3 to 100 nm, and more preferably 5 to 30 nm.

### - Exciton Blocking Layer -

The exciton blocking layer is a layer for preventing excitons generated by recombination of holes and electrons in the light-emitting layer from being diffused in a charge transport layer, and insertion of this layer allows excitons to be efficiently confined in the light-emitting layer, enabling the luminous efficiency of the device to be improved. The exciton blocking layer can be inserted, in a device having two or more light-emitting layers adjacent to each other, between two adjacent light-emitting layers.

Regarding the material of the exciton blocking layer, a known exciton blocking layer material can be used. Examples thereof include 1,3-dicarbazolyl benzene (mCP) and bis(2-methyl-8-quinolinolato)-4-phenylphenolato aluminum (III) (BAlq).

### - Hole Transport Layer -

The hole transport layer is made of a hole transport material having a function of transporting holes, and the hole transport layer can be provided as a single layer or a plurality of layers.

The hole transport material has either hole injection, transport properties or electron barrier properties, and may be an organic material or an inorganic material. For the hole transport layer, any one selected from conventionally known compounds can be used. Examples of such a hole transport material include porphyrin derivatives, arylamine derivatives, triazole derivatives, oxadiazole derivatives, imidazole derivatives, polyarylalkane derivatives, pyrazoline derivatives and pyrazolone derivatives, phenylenediamine derivatives, arylamine derivatives, amino-substituted chalcone derivatives, oxazole derivatives, styryl anthracene derivatives, fluorenone derivatives, hydrazone derivatives, stilbene derivatives, silazane derivatives, an aniline copolymer, and a conductive polymer oligomer, and particularly a thiophene oligomer. Use of porphyrin derivatives, arylamine derivatives, or styrylamine derivatives is preferred. Use of arylamine compounds is more preferred.

### - Electron Transport Layer -

The electron transport layer is made of a material having a function of transporting electrons, and the electron transport layer can be provided as a single layer or a plurality of layers.

The electron transport material (which may also serve as a hole blocking material) may have a function of transferring electrons injected from the cathode to the light-emitting layer. For the electron transport layer, any one selected from conventionally known compounds can be used, and examples thereof include polycyclic aromatic derivatives such as naphthalene, anthracene, and phenanthroline, tris(8-quinolinolato)aluminum(III) derivatives, phosphine oxide derivatives, nitro-substituted fluorene derivatives, diphenylquinone derivatives, thiopyrandioxide derivatives, carbodiimide, fluorenylidene methane derivatives, anthraquinodimethane and anthrone derivatives, bipyridine derivatives, quinoline derivatives, oxadiazole derivatives, benzimidazole derivatives, benzothiazole derivatives, and indolocarbazole derivatives. In addition, a polymer material in which the above material is introduced into a polymer chain or the above material is used for a main chain of a polymer can be used.

### Examples

Hereafter, the present invention will be described in detail by referring to Examples, but the present invention is not limited these Examples and can be implemented in various forms without departing from the gist thereof.

### Synthesis Example 1

Intermediate (1-1) was synthesized in accordance with the next reaction formula.

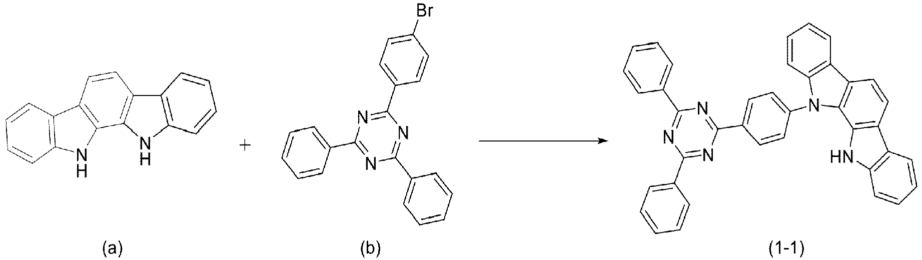

To 25.6 g (100 mmol) of compound (a) were added 42.7 g (110 mmol) of compound (b), 19.2 g (200 mmol) of sodium tert-butoxide, and 900 ml of toluene, 1.12 g (5 mmol) of palladium acetate and 1.01 g (5 mmol) of tert-butylphosphine dissolved in 20 ml of toluene under a nitrogen atmosphere were added, and the mixture was stirred at 120°C for 1 hour. The mixture was cooled to room temperature, and then purified by silica gel column chromatography and crystallization to give 35.0 g (62.1 mmol, 62% yield) of intermediate (1-1) as a yellow solid.

Compound (1) was synthesized in accordance with the next reaction formula.

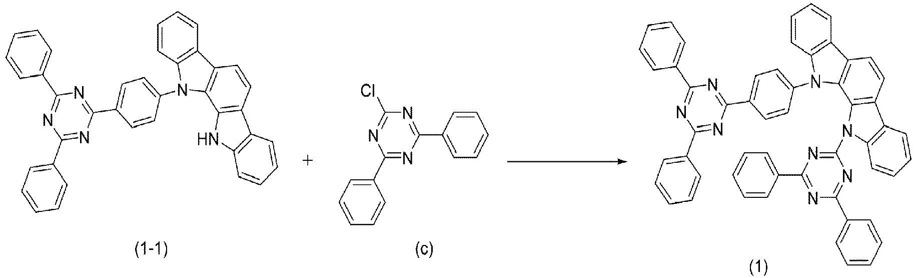

Under a nitrogen atmosphere, 0.7 g (16 mmol) of 60 wt% sodium hydride was added to 15 ml of N,N'-dimethylacetamide to prepare a suspension. To this was added 9.02 g (16 mmol) of intermediate (1-1), and the mixture was stirred for 30 minutes. To this was added 4.71 g (17.6 mmol) of compound (c), then the mixture was stirred for 4 hours. The reaction solution was added to a solution of methanol (300 ml) mixed with distilled water (100 ml) while being stirred, and the resulting precipitated solid was collected by filtration. The resulting solid was purified by silica gel column chromatography and crystallization to give 9.5 g (12.0 mmol, 75% yield) of compound (1) as a yellow solid compound (APCI-TOFMS, m/z 795 [M+H]⁺).

### Synthesis Example 2

Intermediate (2-1) was synthesized in accordance with the next reaction formula.

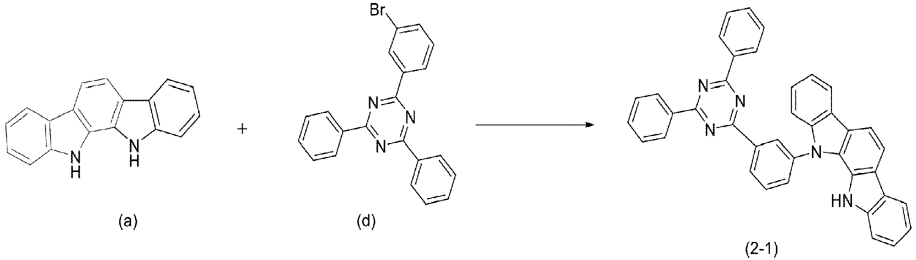

To 5.13 g (20 mmol) of compound (a) were added 8.54 g (22 mmol) of compound (d), 3.84 g (40 mmol) of sodium tert-butoxide, and 200 ml of toluene, 225 mg (1.0 mmol) of palladium acetate and 202 mg (1.0 mmol) of tert-butylphosphine dissolved in 10 ml of toluene under a nitrogen atmosphere were added, and the mixture was stirred at 120°C for 1 hour. The mixture was cooled to room temperature, and then purified by silica gel column chromatography and crystallization to give 7.0 g (12.2 mmol, 61% yield) of intermediate (2-1) as a yellow solid.

Compound (8) was synthesized in accordance with the next reaction formula.

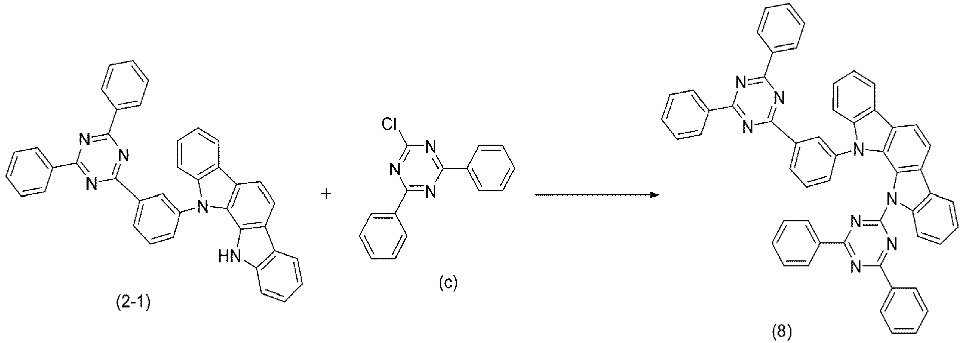

Under a nitrogen atmosphere, 0.212 g (5.3 mmol) of 60 wt% sodium hydride was added to 15 ml of N,N'-dimethylacetamide to prepare a suspension. To this was added 2.99 g (5.3 mmol) of intermediate (2-1), and the mixture was stirred for 30 minutes. To this was added 1.56 g (5.83 mmol) of compound (c), then the mixture was stirred for 4 hours. The reaction solution was added to a solution of methanol (200 ml) mixed with distilled water (100 ml) while being stirred, and the resulting precipitated solid was collected by filtration. The resulting solid was purified by silica gel column chromatography and crystallization to give 2.90 g (3.6 mmol, 69% yield) of compound (8) as a yellow solid compound (APCI-TOFMS, m/z 795 [M+H]⁺).

### Synthesis Example 3

Intermediate (3-1) was synthesized in accordance with the next reaction formula.

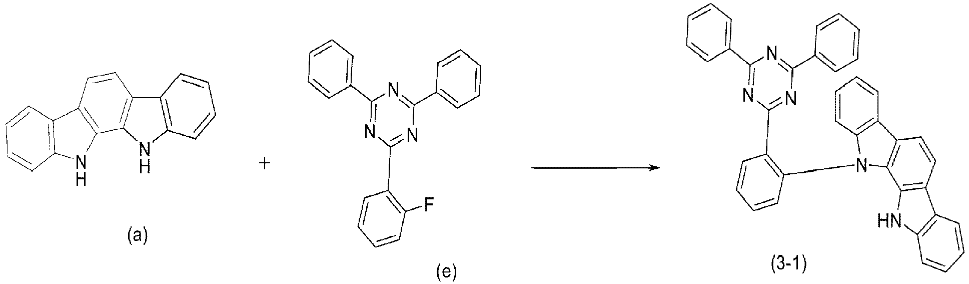

Under a nitrogen atmosphere, 12.8 g (50.0 mmol) of compound (a), 19.6 g (60.0 mmol) of compound (e), 48.8 g (150.0 mmol) of cesium carbonate, and 300 ml of 1,3-dimethyl-2-imidazolidinone were mixed, and stirred at 170°C for 24 hours. After the reaction solution was cooled to room temperature, an inorganic material was separated by filtration. The filtrate was added to 1200 ml of a mixed liquid of methanol and water at 1:1, and then the precipitated product was separated by filtration and dried under reduced pressure. The resulting solid product was purified by silica gel column chromatography to give 20.1 g (35.7 mmol, 71.1% yield) of intermediate (3-1) as a yellow solid.

Compound (16) was synthesized in accordance with the next reaction formula.

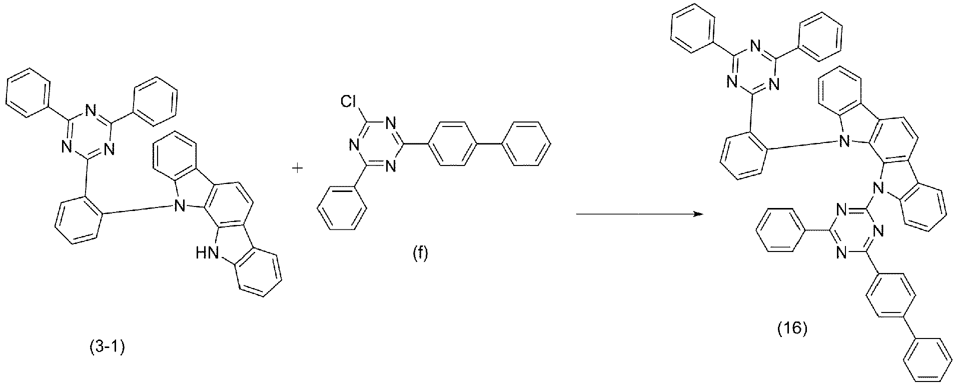

Under a nitrogen atmosphere, 5.7 g (10.0 mmol) of intermediate (3-1), 5.2 g (15.0 mmol) of compound (f), 1.3 g (30.0 mmol) of 60 wt% sodium hydride, and 150 ml of N,N'-dimethylacetamide were mixed, and stirred at room temperature for 4 hours. Thereafter, the reaction solution was added to a 600 ml of a mixture liquid of ethanol and water at 1:1, and then the precipitated product was separated by filtration and dried under reduced pressure. The resulting solid product was purified by silica gel column chromatography to give 6.3 g (7.2 mmol, 72.2% yield) of compound (16) as a yellow solid compound.

Compounds 2 and 148 were synthesized according to the synthesis examples. Compounds A, B, C, D and E were also synthesized for comparison.

### Example 1

On a glass substrate on which an anode made of ITO with a film thickness of 110 nm was formed, respective thin films were laminated by a vacuum evaporation method at a degree of vacuum of 4.0 × 10⁻⁵ Pa. First, HAT-CN was formed with a thickness of 25 nm as a hole injection layer on ITO, and next, Spiro-TPD was formed with a thickness of 30 nm as a hole transport layer. Next, HT-1 was formed with a thickness of 10 nm as an electron blocking layer. Then, compound 1 as a host material and Ir(ppy)₃ as a light-emitting dopant were co-vapor-deposited from different vapor deposition sources, respectively, to form a light-emitting layer with a thickness of 40 nm. In this case, the concentration of Ir(ppy)₃ was 10 wt%. Next, ET-1 was formed with a thickness of 20 nm as an electron transport layer. Further, LiF was formed with a thickness of 1 nm as an electron injection layer on the electron transport layer. Finally, Al was formed with a thickness of 70 nm as a cathode on the electron injection layer to produce an organic EL device.

When an external power supply was connected to the obtained organic EL devices and a DC voltage was applied, an emission spectrum with a maximum wavelength of 517 nm was observed, and it was thus found that light emission from Ir(ppy)₃ was obtained.

### Example 2

Organic EL devices were produced in the same manner as in Example 1 except that compound 8 was used as the host material of the light-emitting layer instead of compound 1 of Example 1. When a DC voltage was applied to the obtained organic EL devices, an emission spectrum with a maximum wavelength of 517 nm was observed.

### Comparative Examples 1 to 2

Organic EL devices were produced in the same manner as in Example 1 except that compounds A or B was used as the host material of the light-emitting layer instead of that of Example 1. When an external power supply was connected to the obtained organic EL devices and a DC voltage was applied, an emission spectrum with a maximum wavelength of 517 nm was observed.

Evaluation results of the produced organic EL devices are shown in Table 1. In the table, the luminance, driving voltage, and power efficiency are values at a driving current of 20 mA/cm², and they exhibit initial characteristics. LT70 is a time period needed for the initial luminance to be reduced to 70% thereof at a driving current of 20 mA/cm², and it represents lifetime characteristics.

**[Table 1]**

| | Host compound | Voltage (V) | Luminance (cd/m²) | Power efficiency (Im/W) | LT70 (h) |
|---|---|---|---|---|---|
| Example 1 | 1 | 3.3 | 10900 | 51.7 | 510 |
| Example 2 | 8 | 3.4 | 11000 | 51.3 | 530 |
| Comparative Example 1 | A | 3.6 | 9000 | 39.7 | 430 |
| Comparative Example 2 | B | 3.5 | 10500 | 46.8 | 400 |

### Example 3

On a glass substrate on which an anode made of ITO with a film thickness of 110 nm was formed, respective thin films were laminated by a vacuum evaporation method at a degree of vacuum of 4.0 × 10⁻⁵ Pa. First, HAT-CN was formed with a thickness of 25 nm as a hole injection layer on ITO, and next, Spiro-TPD was formed with a thickness of 30 nm as a hole transport layer. Next, HT-1 was formed with a thickness of 10 nm as an electron blocking layer. Then, compound 1 as a first host, compound 602 as a second host and Ir(ppy)₃ as a light-emitting dopant were co-vapor-deposited from different vapor deposition sources, respectively, to form a light-emitting layer with a thickness of 40 nm. In this case, co-vapor deposition was performed under vapor deposition conditions such that the concentration of Ir(ppy)₃ was 10 wt%, and the weight ratio between the first host and the second host was 30:70. Next, ET-1 was formed with a thickness of 20 nm as an electron transport layer. Further, LiF was formed with a thickness of 1 nm as an electron injection layer on the electron transport layer. Finally, Al was formed with a thickness of 70 nm as a cathode on the electron injection layer to produce an organic EL device.

### Examples 4 to 9

Organic EL devices were produced in the same manner as in Example 3 except that compounds shown in Table 2 were used as the first host and the second host instead of those of Example 3.

Herein, the weight ratio between the first host and the second host in each of Examples 4 to 8 was 30:70, and it in Example 9 was 50:50.

### Examples 10 and 11

Organic EL devices were produced in the same manner as in Example 3 except that a premixture obtained by weighing a first host (0.30 g) and a second host (0.70 g) shown in Table 2 and mixing them while grinding in a mortar was co-vapor-deposited from one vapor deposition source.

### Example 12

An organic EL device was produced in the same manner as in Example 3 except that a premixture obtained by weighing a first host (0.50 g) and a second host (0.50 g) shown in Table 2 and mixing them while grinding in a mortar was vapor-deposited from one vapor deposition source.

### Comparative Examples 3 to 6

Organic EL devices were produced in the same manner as in Example 3 except that compounds shown in Table 2 were used as the first host and the second host instead of those of Example 3.

### Comparative Example 7

Organic EL devices were produced in the same manner as in Example 10 except that compounds shown in Table 2 were used as the first host and the second host instead of those of Example 10.

### Comparative Example 8

An organic EL device was produced in the same manner as in Example 12 except that compounds shown in Table 2 were used as the first host and the second host instead of those of Example 12.

Evaluation results of the produced organic EL devices are shown in Table 2. In the table, the luminance, driving voltage, power efficiency, and LT70 are the same as those in Table 1.

**[Table 2]**

| | First host compound | Second host compound | Voltage (V) | Luminance (cd/m²) | Power efficiency (Im/W) | LT70 (h) |
|---|---|---|---|---|---|---|
| Example 3 | 1 | 602 | 4.2 | 10400 | 38.7 | 1610 |
| Example 4 | 2 | 602 | 4.1 | 10100 | 38.6 | 1770 |
| Example 5 | 148 | 602 | 4.0 | 10100 | 39.7 | 1690 |
| Example 6 | 8 | 602 | 4.4 | 10500 | 37.7 | 1420 |
| Example 7 | 16 | 602 | 4.6 | 10000 | 34.1 | 2400 |
| Example 8 | 1 | 642 | 4.2 | 10500 | 39.5 | 1660 |
| Example 9 | 1 | 642 | 3.7 | 10700 | 45.0 | 1140 |
| Example 10 | 1 | 642 | 4.1 | 10600 | 40.1 | 1760 |
| Example 11 | 8 | 602 | 4.3 | 10600 | 38.4 | 1510 |
| Example 12 | 1 | 642 | 3.7 | 10800 | 45.4 | 1220 |
| Comparative Example 3 | C | 642 | 4.7 | 10400 | 34.6 | 140 |
| Comparative Example 4 | D | 602 | 4.6 | 9600 | 32.4 | 870 |
| Comparative Example 5 | D | 642 | 4.5 | 9900 | 34.6 | 820 |
| Comparative Example 6 | E | 602 | 4.9 | 10000 | 32.2 | 430 |
| Comparative Example 7 | D | 602 | 4.6 | 9800 | 33.2 | 900 |
| Comparative Example 8 | D | 602 | 3.9 | 10700 | 42.6 | 450 |

From Tables 1 and 2, it is understood that Examples 1 to 12 improved the power efficiency or the lifetime and exhibited good characteristics, as compared with Comparative Examples.

Compounds used in Examples are shown below.

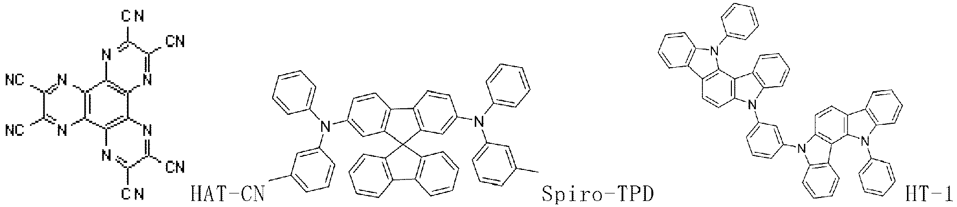

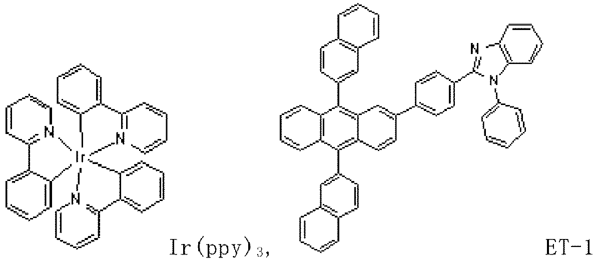

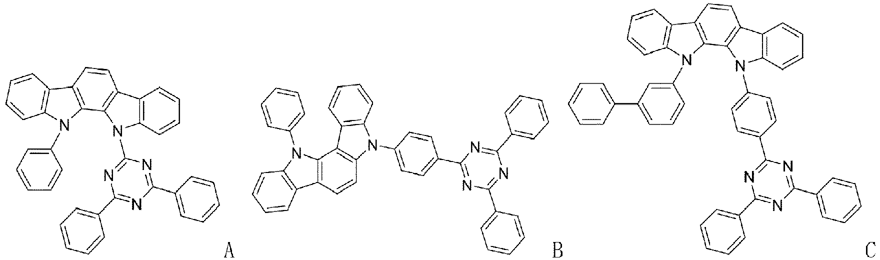

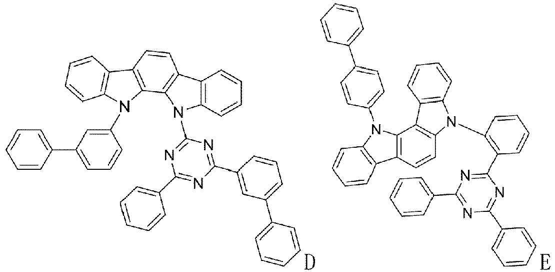

Table 3 shows the 50% weight reduction temperatures (T₅₀) of compounds 1, 8, 602, 642 and D.

**[Table 3]**

| Compound | T₅₀[°C] |
|---|---|
| 1 | 295 |
| 8 | 276 |
| 602 | 279 |
| 642 | 310 |
| D | 271 |

## Claims

1. A compound for an organic electroluminescent device, represented by the following general formula (1): 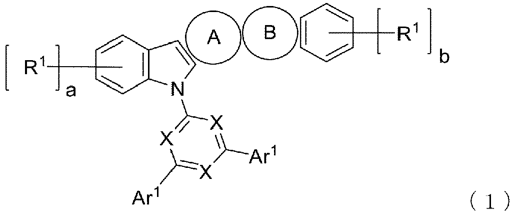 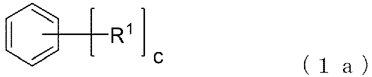 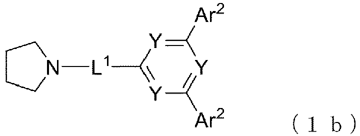
wherein a ring A represents an aromatic hydrocarbon ring fused to two adjacent rings at any position and represented by formula (1a),
a ring B represents a heterocyclic ring fused to two adjacent rings at any position and represented by formula (1b),
X and Y each independently represent CR² or N, wherein at least one thereof represents N,
each R¹ independently represents deuterium, an aliphatic hydrocarbon group having 1 to 10 carbon atoms, a substituted or unsubstituted aromatic hydrocarbon group having 6 to 18 carbon atoms, or a substituted or unsubstituted aromatic heterocyclic group having 3 to 17 carbon atoms,
each R² independently represents hydrogen, deuterium, an aliphatic hydrocarbon group having 1 to 10 carbon atoms,
a substituted or unsubstituted aromatic hydrocarbon group having 6 to 18 carbon atoms, or a substituted or
unsubstituted aromatic heterocyclic group having 3 to 17 carbon atoms,
a and b each independently represent an integer of 0 to 4, and c represents an integer of 0 to 2,
Ar¹ and Ar² each independently represent hydrogen, a substituted or unsubstituted aromatic hydrocarbon group having 6 to 18 carbon atoms, a substituted or
unsubstituted aromatic heterocyclic group having 3 to 17 carbon atoms, or a substituted or unsubstituted linked aromatic group in which two to five of these aromatic rings are linked to each other, and
L¹ represents a substituted or unsubstituted aromatic hydrocarbon group having 6 to 18 carbon atoms, or a substituted or unsubstituted linked aromatic group in which two to five of these aromatic hydrocarbon groups are linked to each other.

2. The compound for an organic electroluminescent device according to claim 1, wherein all of X in the general formula (1) represent N.

3. The compound for an organic electroluminescent device according to claim 1 or 2, wherein L¹ in the general formula (1) represents a substituted or unsubstituted phenylene group.

4. The compound for an organic electroluminescent device according to any one of claims 1 to 3, wherein Ar¹ and Ar² in the general formula (1) independently represent hydrogen, a substituted or unsubstituted aromatic hydrocarbon group having 6 to 18 carbon atoms, or a substituted or unsubstituted linked aromatic group in which two to five of these aromatic hydrocarbon groups are linked to each other.

5. The compound for an organic electroluminescent device according to any one of claims 1 to 4, wherein all of Y in the general formula (1) represent N.

6. An organic electroluminescent device comprising one or more light-emitting layers between an anode and a cathode opposed to each other, wherein at least one of the light-emitting layers contains a first host, a second host, and a light-emitting dopant material, the first host is selected from the compound for an organic electroluminescent device according to any one of claims 1 to 5, and the second host is selected from a compound represented by the following general formula (2): 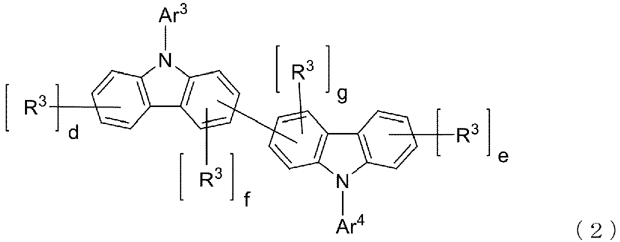
wherein Ar³ and Ar⁴ independently represent a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group in which two to five of these aromatic groups are linked to each other,
each R³ independently represents deuterium, an aliphatic hydrocarbon group having 1 to 10 carbon atoms, a substituted or unsubstituted aromatic hydrocarbon group having 6 to 18 carbon atoms, or a substituted or
unsubstituted aromatic heterocyclic group having 3 to 17 carbon atoms, and
d and e represent an integer of 0 to 4, and f and g represent an integer of 0 to 3.

7. The organic electroluminescent device according to claim 6, wherein Ar³ and Ar⁴ in the general formula (2) independently represent a substituted or unsubstituted aromatic hydrocarbon group having 6 to 18 carbon atoms, or a substituted or unsubstituted linked aromatic group in which two to five of these aromatic hydrocarbon groups are linked to each other.

8. The organic electroluminescent device according to claim 6 or 7, wherein all d, e, f, and g in the general formula (2) represent 0.

9. The organic electroluminescent device according to any one of claims 6 to 8, wherein Ar³ and Ar⁴ in the general formula (2) independently represent a substituted or unsubstituted phenyl group, m-biphenyl group, or p-biphenyl group.

10. A method for producing the organic electroluminescent device according to any one of claims 6 to 9, comprising providing a premixed composition comprising the first host and the second host, and producing a light-emitting layer by use of the premixed composition.

11. A premixed composition for use in the method for producing the organic electroluminescent device according to claim 10, comprising the first host and the second host.

12. The premixed composition according to claim 11, wherein a difference in 50% weight reduction temperatures of the first host and the second host is within 20°C.
